(19) Europäisches Patentamt / European Patent Office / Office européen des brevets

(11) **EP 4 055 190 B1**

(12) **EUROPEAN PATENT SPECIFICATION**

(45) Date of publication and mention
of the grant of the patent:
**03.07.2024  Bulletin 2024/27**

(21) Application number: **20800687.4**

(22) Date of filing: **09.11.2020**

(51) International Patent Classification (IPC):
*C12Q 1/6883* (2018.01)      *G16B 30/00* (2019.01)
*G16B 20/00* (2019.01)      *G16B 40/20* (2019.01)

(52) Cooperative Patent Classification (CPC):
**C12Q 1/6883; G16B 20/00; G16B 40/20;**
C12Q 2600/156

(86) International application number:
**PCT/EP2020/081458**

(87) International publication number:
**WO 2021/089865 (14.05.2021 Gazette 2021/19)**

(54) **DIAGNOSING OF MOOD DISORDERS USING BLOOD RNA EDITING BIOMARKERS**

DIAGNOSE VON STIMMUNGSSTÖRUNGEN UNTER VERWENDUNG VON
BLUT-RNA-EDITIERUNGSBIOMARKERN

DIAGNOSTIC DES TROUBLES DE L'HUMEUR À L'AIDE DE BIOMARQUEURS D'ÉDITION D'ARN
SANGUIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **08.11.2019  EP 19208124**

(43) Date of publication of application:
**14.09.2022  Bulletin 2022/37**

(73) Proprietor: **ALCEDIAG**
**13790 Peynier (FR)**

(72) Inventors:
• **WEISSMANN, Dinah**
**34270 Mathieu de Treviers (FR)**
• **SALVETAT, Nicolas**
**34070 Montpellier (FR)**
• **CHIMIENTI, Fabrice**
**34830 Jacou (FR)**

(74) Representative: **Yes My Patent**
**32 Boulevard Richard Lenoir**
**75011 Paris (FR)**

(56) References cited:
**EP-A1- 3 272 881      WO-A1-2021/089866
US-A1- 2019 065 666      US-A1- 2019 185 937
US-A1- 2019 249 251**

Remarks:
The complete document including Reference
Table(s) and the Sequence Listing(s) can be
downloaded from the EPO website

**Description**

The present invention is drawn to a method for in vitro diagnosing depression disorders, more preferably major depressive episode (DEP) in a human patient from a biological sample of said patient, using at least two particular A to I editing RNA biomarkers associated to a specific depression score and algorithm. The present invention is also directed to a method for monitoring treatment for depression in a human subject, said method implementing the method for diagnosing mood disorders of the present invention. Kit for determining whether a patient presents depression disorder, are also comprised in the present invention.

Major depressive episode (DEP) is a very common, heterogeneous stress-related mental disorder and the most prevalent, affecting ~10% of men and ~20% of women worldwide. DEP is associated with a markedly increased mortality, mostly due to suicidal behavior but also increased risk of developing other medical conditions, e.g. heart disease, diabetes, and stroke. The chronic and recurrent course of DEP, associated with the treatment resistance frequently observed - the remission rate following the first treatment course is 37%- further augment the use of healthcare system. Currently, DEP is diagnosed through a clinical assessment against criteria set out in the 5th edition of Diagnostic and Statistics Manual for Mental Disorders[1]. DEP is characterized by episodes of at least 2 weeks during which an individual experiences a combination of five or more different symptoms, e.g. depressed mood, loss of interest in daily activities, sleep dysregulation, fatigue or indecisiveness. Hence, the principal problem is that the diagnosis of DEP is based on descriptive criteria and thus subjective by nature. Accurate diagnosis is further complicated by the heterogeneous nature of DEP, for which the most common depression scales might not overlap completely, and by its symptomatic similarity with other psychiatric and somatic disorders.

A wealth of studies have investigated putative biomarkers for DEP[2], which could help to diagnose disease or provide reliable substitutes for clinical response to treatment. The definition for a biomarker is "a characteristic that is objectively measured and evaluated as an indicator of normal biological processes, pathogenic processes, or pharmacologic responses to a therapeutic intervention". As such, a biomarker can be used as a diagnostic tool, as a tool for staging the disease and/or for prediction and monitoring of clinical response to an intervention. The development of biomarker(s) which could be suitable for a diagnostic test in the field of mood disorders is a complex and difficult challenge. Several biomarkers have been suggested for DEP, including circulating proinflammatory cytokines (e.g. IL6, TNF$\alpha$, IL-1$\beta$) or the inflammation marker CRP, neurotrophic factors (e.g. BDNF), and hormones e.g. ACTH or cortisol (for review see [3]). However, when used separately none of these biomarkers fulfills the sensitivity and specificity criteria for a clinical application. Over the past decade, numerous studies have focused on brain biomarkers, including functional neuroimaging studies, or explored all the -omics field, including genomics, transcriptomics, proteomics or metabolomics.

Most promising methods seem to reside in a multi-parameter biomarker panel, for which there is a biological explanation. Those biomarkers should be quantified using a routine blood sample and standard laboratory methodology; a specific diagnostic algorithm is often developed in parallel[4]. A major issue in the identification of biomarkers suitable for a diagnostic assay in DEP is that it might require the use of a combination of several biomarkers reflecting changes in different biological mechanisms.

Accumulating evidence have indicated that RNA editing, which is mostly performed by the ADAR (adenosine deaminase acting on RNA) enzymes may be critically involved in neurological or immune diseases, among other pathologies. Inflammation is known as a pathogenic factor, possibly explaining a part of physiopathology of depression. Recent evidence supports the notion that RNA editing plays a crucial role in immune-inflammation processes It has been shown that ADAR1 regulates crucial immune responses through RNA editing. A recent study reported an overall increased RNA editing in systemic lupus erythematosus (SLE), a chronic autoimmune disorder, which could well increase the autoantigen load and promote SLE progression. By editing specific primary miRNAs that target the IL-6 receptor subunit gp130, ADAR2 controls gp130-dependent IL-6 signaling. We previously demonstrated altered RNA editing in psychiatric patients with chronic hepatitis C virus (HCV) undergoing antiviral combination therapy with IFN-$\alpha$ and ribavirin, in whom treatment-emergent depression is a common complication. In the CNS, RNA editing in the coding sequence of proteins key for mood regulation, including AMPA and Kainate (KA) subtypes of glutamate receptor and the serotonin receptor, can modulate the functional properties of the encoded protein products. Decreased editing of the R/G sites of AMPA receptors due to down-regulation of ADAR2 has a possible role in the pathophysiology of mental disorders. RNA editing at the Q/R site of AMPAR, an ionotropic transmembrane receptor for glutamate might be involved in the pathogenesis of mood disorders and in the action of antidepressants. We and others have identified altered serotonin receptor in the brain of patients with major depression or depressed suicide attempters. In a recent work, we have identified in the brain of suicide decedents modifications in PDE8A RNA editing as an immune response-related brain marker for suicide[5].

US2019/185937A1 relates to RNA editing as biomarkers for mood disorders test.

In the current study, we focused on transcriptome-wide RNA editing modification by RNA-Seq on DEP patients to identify editing variants that could be used as biomarkers for a clinically useful diagnostic assay of DEP. Using measurement of transcriptome-wide RNA editing modifications we identified significant differential editing rate in a number of genes related to immune system and CNS function. Subsequently, we selected the best 15 best biomarkers for further validation on a prioritization cohort. These RNA editing variants were involved in different pathways relevant for mood disorders and DEP, e.g. immune system or neurotransmitters receptors and postsynaptic transmission. A machine-learning approach was applied to combine RNA editing variants and optimize prediction, before validating the biomarkers panel in a large cohort of 411 depressed patients and age-, race- and sex-matched control subjects. The RNA editing variants panel discriminated DEP patients from healthy controls with high specificity and sensitivity. This study is the first to evaluate multiple RNA editing variants in blood samples of DEP patients. Our findings will contribute to a better understanding of the molecular pathophysiology of DEP, and pave the way for the development of a diagnostic assay for DEP with clinical application.

The present invention is defined in the appended claims.

Disclosed, but not part of the claimed invention, is a method for selected at least one biomarker, preferably a combination of at least two biomarkers, which can be used to diagnose mood disorders, particularly depression disorders, more preferably major depressive episode, in a human patient from blood sample of said patient, said method comprising the step of:

a) analyzing the RNA-Seq dataset using Editome analysis pipeline and identifying A-to-I differentially edited positions with a minimum coverage of 30x, which ensures a high degree of confidence at particular base positions;

b) performing a differential analysis to identify sites whose editing could be specifically different between mood disorders, particularly depression disorders, more preferably DEP patients and healthy controls;

c) applying the following pre-specified quality criteria of the group consisting of: coverage >30; AUC>0.6; 0.95>Fold-Change>1.05, p<0.05 and exclusion of intergenic sites;

d) optionally, checking that no difference in global RNA editing was observed between patients and controls, preferably by calculating the global Alu editing index (AEI), which is a measure of the overall rate of RNA editing in Alu repeats;

e) by GSEA (enrichment analysis on gene sets) on the biomarkers selecting in step c) or d), preferably by using gene ontology tools, identifying and selecting biomarkers reflecting changes in different biological process including immune and CNS (central nervous system) functions; and

f) applying the following specified quality criteria of the group consisting of: coverage >30; AUC>0.8; 0.8>Fold-Change>1.20 and p<0.05) to a combination of several biomarkers selected in step e) representing different biological mechanisms, and selected those which clearly discriminated healthy controls and patients having mood disorders, particularly depression disorders, more preferably major depressive episode, in two separate groups.

In a preferred embodiment, in step f), the combinations of at least two biomarkers are selected whether said combinations are statistically significant between control and cases with a p value <$10^{-4}$.

The present invention is directed to an in vitro method for diagnosing depression disorders, more preferably DEP, in a human patient from a biological sample of said patient, said method comprising:

a) determining for at least two A to I editing RNA biomarker, the relative proportion of RNA editing at a given editing site for at least one or a combination of sites which can be edited on the RNA transcript of said t biomarker, and/or

- the relative percentage of an isoform (with edited site(s)) or the non-edited isoform) or of a combination of isoforms of the RNA transcript of said biomarker;

wherein said at least two A to I editing RNA biomarker is selected from the group of consisting of PRKCB, MDM2, PIAS1, IFNAR1, IFNAR2, LYN, AHR, RASSF1, GAB2, CAMK1D, KCNJ15, IL17RA and PTPRC biomarkers

b) determining a value resulting from the percentage(s) obtained in step a);

c) determining whether said result value or depression score obtained in step b) is greater or not greater than a control value obtained for control healthy subject, wherein the control value was determined in a manner comparable to that of the result value, obtained in step b);

wherein:

if said depression score obtained for the patient is greater than a threshold (cut-off), classifying said patient as being positive or having depression disorders, more preferably DEP, or if said depression score is not greater than said threshold, classifying said patient as being negative or not having depression disorders, more preferably DEP.

The criterion symptoms for mood disorders, depression disorders and DEP are well-known from the skilled person and are for example described in the DSM-5™ book from the American Psychiatric Association (2013, pages 155-188).

In the present invention, depression disorders include disruptive mood dysregulation disorder, major depressive disorder (including major depressive episode), persistent depressive disorder (dysthymia), premenstrual dysphoric disorder, substance/medication-induced depressive disorder, depressive disorder due to another medical condition, other specified depressive disorder, and unspecified depressive disorder depress.

In a preferred embodiment, the present invention is related to an in vitro method for diagnosing depression disorders, more preferably DEP in a human patient from a biological sample of said patient, according to present invention wherein said method comprises

a) determining for a combination of at least two A to I editing RNA biomarker:

- for each selected biomarker, the relative proportion of RNA editing at a given editing site for at least one or a combination of sites which can be edited on the RNA transcript of said at least two biomarkers, and/or
- the relative percentage of an isoform or of a combination of isoforms of the RNA transcript of each of said two biomarkers;

wherein said at least two A to I editing RNA biomarkers are selected from the group consisting of PRKCB, MDM2, PIAS1, IFNAR1, IFNAR2, LYN, AHR, RASSF1, GAB2, CAMK1D, KCNJ15, IL17RA, PTPRC and PDE8A biomarkers, b) determining a result value obtained for each of said at least two biomarkers and a final result value based on an algorithm or equation that includes the result value obtained for each selected biomarker; c) determining the final result value or depression score obtained for the patient and a control result value obtained for a healthy control subject, wherein the control result value was determined in a manner comparable to that of the final result value; and

d) if said final result or depression score value is greater than a threshold/cut-off, classifying said patient as being positive or having depression disorders, more preferably DEP or, if said final result value is not greater than said threshold, classifying said patient as being negative or not depression disorders, more preferably MDD.

For example, but non-limited to, in the method of diagnosing according to the present invention, said threshold, cut-off or depression score can be:

- the Z value calculated for healthy control patient when using mROC method, or
- the probability for a patient to be considered as having mood disorders, depression disorders or DEP.

In the present description, the term biomarker or target have the same meaning and can be used interchangeably.

In a preferred embodiment, in the method for diagnosing depression according to present invention, the combinations of at least two biomarkers are selected whether said combinations are statistically significant between control healthy control subject and cases (patients having mood disorders, depression disorders, or DEP) with a p value $<10^{-4}$.

In a more preferred embodiment, in step a), said A to I editing RNA biomarker is selected from the group consisting of PRKCB, MDM2, IFNAR1, KCNJ15, LYN, CAMK1D and GAB2.

In a more preferred embodiment, when at least in step a), a combination of at least two A to I editing RNA biomarkers are used,

said at least two A to I editing RNA biomarkers are selected from the group consisting of PRKCB, MDM2, IFNAR1, KCNJ15, LYN, CAMK1D, GAB2 and PDE8A biomarkers.

In an also more preferred embodiment, the present invention is directed to an in vitro method for diagnosing depression disorders, more preferably major depressive episode

according to the invention, wherein in step b), the result value or depression score is calculated by an algorithm implementing a multivariate method including:

- mROC algorithm, particularly to identify the linear combination, which maximizes the AUC (Area Under the Curve) ROC and wherein the equation for the respective combination is provided and can be used as a new virtual marker Z, as follows:

$$Z = a.(Biomarker\ 1) + b.(Biomarker\ 2) + \ldots i.(Biomarker\ i) + \ldots n\ .(Biomarker\ n)$$

where a, b, i, n are calculated coefficients and (Biomarker 1, 2, i, n) are the level of the considered biomarker (i.e. level of RNA editing site or of isoforms for a given target/biomarker); and/or
- a Random Forest (RF) algorithm applied to assess the RNA editing site(s) and/or isoforms combinations, particularly to rank the importance of the RNA editing site(s) and/or isoform(s), and to combine the best RNA editing site(s) and/or isoform(s), and/or optionally

- a multivariate analysis applied to assess the RNA editing site(s) and/or isoforms combinations for the diagnostic, said multivariate analysis being selecting for example from the group consisting of:
- Logistic regression model applied for univariate and multivariate analysis to estimate the relative risk of patient at different level of RNA editing site or isoforms values;
- CART (Classification And Regression Trees) approach applied to assess RNA editing site(s) and/or isoforms combinations;
- Support Vector Machine (SVM) approach;
- Artificial Neural Network (ANN) approach;
- Bayesian network approach;
- WKNN (weighted k-nearest neighbours) approach;
- Partial Least Square - Discriminant Analysis (PLS-DA);
- Linear and Quadratic Discriminant Analysis (LDA / QDA), and
- Any other mathematical method that combines biomarkers

In a particular preferred embodiment, said depression disorder which is desired to diagnose is the major depressive episode (DEP).

In another preferred embodiment, the present invention is directed to a method for diagnosing depression disorders according to the present invention, wherein said depression disorders include mild, moderate and severe.

In another preferred embodiment, the present invention is directed to a method for diagnosing depression disorders according to the present invention, wherein said depression can be classified as mild, moderate or severe depression depending of the level of the difference between the depression score value obtained in step b) and the control result value obtained for the control subject.

In a preferred embodiment, in the method for diagnosing depression disorders according to the present invention, said biological sample is whole blood, serum, plasma, urine, or cerebrospinal fluid, whole blood being the most preferred biological sample

Are preferred selected biomarkers wherein the result value or depression score obtained in step c) is statistically/specifically different from said control result value at $p<0.05$.

Are also preferred the methods of the present invention wherein the following criteria has to be satisfied for the biomarker or for the combination of biomarkers selected tin step a):

- the coverage >30;
- AUC>0.6, preferably AUC>0.8;
- 0.95>FoldChange>1.05, and
- $p<0.05$.

Are also preferred the methods of the present invention wherein said selected A to I editing RNA biomarker(s) comprised in step a) is selected from the group consisting of:

a) a combination comprising at least 3, 4, 5, 6, 7 or 8 of the following biomarkers: PRKCB, MDM2, IFNAR1, KCNJ15, LYN, CAMK1D, GAB2 and PDE8A, preferably the combination of the cited 8 biomarkers.

Are also preferred the methods of the present invention wherein said selected A to I editing RNA biomarker(s) comprised in step a) the combination of the following 8 biomarkers GAB2, IFNAR1, KCNJ15, LYN, MDM2, PRKCB, CAMK1D and PDE8A when the model is adjusted by age, sex, psychiatric treatment and addiction, or the when the model is adjusted by age and sex, preferably when using a Random Forest (RF) algorithm applied to assess the RNA editing site(s) and/or isoforms combinations, particularly to rank the importance of the RNA editing site(s) and/or isoform(s), and to combine the best RNA editing site(s) and/or isoform(s) .

Are also preferred the methods of the present invention wherein said selected A to I editing RNA biomarker(s) comprised in step a) the combination of the following 8 biomarkers GAB2, IFNAR1, KCNJ15, LYN, MDM2, PRKCB, CAMK1D and PDE8A, when the model is adjusted by age, sex, psychiatric treatment and addiction, or when the model is adjusted by age and sex, using a Random Forest (RF) algorithm applied to assess the RNA editing site(s) and/or isoforms combinations, particularly to rank the importance of the RNA editing site(s) and/or isoform(s), and to combine the best RNA editing site(s) and/or isoform(s), and preferably wherein the calculation of the relative percentage of at least one of the RNA edition site or isoform is based on the RNA edition site or isoform listed in Tables 5.1 and 5.2.

Are also preferred the methods of the present invention wherein said selected A to I editing RNA biomarker comprised in step a) one or combination of at least 2, 3, 4, 5, 6, 7 or 8 selected biomarkers and wherein the calculation of the relative percentage of at least one of the RNA edition site or isoform is based on the RNA edition site or isoform listed in Table 2A (editing sites), 2B (isoforms) or 3 (editing sites) for each of the selected biomarker.

Are also preferred the methods of the present invention wherein said combination of at least 2, 3, 4, 5, 6, 7 or 8 selected biomarkers is a combination selected from the biomarkers combinations given in Table 4 and in Table 5, preferably the combinations comprising the following 8 biomarkers PRKCB, MDM2, IFNAR1, KCNJ15, LYN, CAMK1D, GAB2 and PDE8A.

Are also preferred the combinations comprising the 8 biomarkers PRKCB, MDM2, IFNAR1, KCNJ15, LYN, CAMK1D, GAB2 and PDE8A and selected from the combinations listed in Table 6.

Are also preferred the methods of the present invention wherein the algorithm or equation allowing the calculation of the result value or depression score Z are selected from the Z equations listed in the examples, preferably the equation implemented a combination of the following 8 biomarkers PRKCB, MDM2, IFNAR1, KCNJ15, LYN, CAMK1D, GAB2 and PDE8A.

In another particular aspect, the present invention concerns an in vitro method for diagnosing depression disorders, more preferably MDD in a human patient according to the invention, wherein if the patient to be tested is a female, in step a) said at least one or combination of at least two A to I editing RNA biomarker(s) is selected from the group of biomarkers consisting of PRKCB, MDM2, PIAS1, IFNAR1, IFNAR2, LYN, AHR, RASSF1, GAB2, CAMK1D, KCNJ15, IL17RA and PTPRC, if one biomarker is selected, or said group further comprising the A to I editing RNA PDE8A if a combination of at least two biomarkers (or targets) are selected.

Are also preferred the methods of the present invention wherein said combination of at least 2, 3, 4, 5, 6, 7 or 8 selected biomarkers is a combination selected from the group consisting of PRKCB, MDM2, IFNAR1, KCNJ15, LYN, CAMK1D, GAB2 and PDE8A biomarkers (or targets), particularly the biomarkers combinations or Z equation given in the Examples or figures with patients of the female population, particularly the combinations comprising the 8 biomarkers PRKCB, MDM2, IFNAR1, KCNJ15, LYN, CAMK1D, GAB2 and PDE8A, more preferably the combinations listed in Table 7.

In another particular aspect, the present invention concerns an in vitro method for diagnosing depression in a human patient according to the invention, wherein if the patient to be tested is a male, in step a) said at least one or combination of at least two A to I editing RNA biomarker is selected from the group of biomarkers consisting of PRKCB, MDM2, PIAS1, IFNAR1, IFNAR2, LYN, AHR, RASSF1, GAB2, CAMK1D, KCNJ15, IL17RA and PTPRC, if one biomarker is selected, or said group further comprising the A to I editing RNA PDE8A if a combination of at least two biomarkers (or targets) are selected.

Are also preferred the methods of the present invention wherein said combination of at least 2, 3, 4, 5, 6, 7 or 8 selected biomarkers is a combination selected from the group consisting of PRKCB, MDM2, IFNAR1, KCNJ15, LYN, CAMK1D, GAB2 and PDE8A biomarkers (or targets), particularly the biomarkers combinations or Z equation given in the Examples or figures with patients of the male population, particularly the combinations comprising the 8 biomarkers PRKCB, MDM2, IFNAR1, KCNJ15, LYN, CAMK1D, GAB2 and PDE8A, more preferably the combinations listed in Table 9.

In a more preferred embodiment, the present invention is directed to a method in vitro for diagnosing depression in a human patient according to the present invention, wherein the combination of biomarkers and/or the Z equation or algorithm associated with said combination used for the mood disorders, particularly depression disorders, diagnostic is different whether the patient to be tested is a female or a male.

Preferably, the combination of biomarkers and/or the Z equation whether the patient to be tested is a female or a male is selected from the combination and/or the Z equation depicted in the examples or in the figures for this particular case.

Are also preferred the methods of the present invention, wherein in step a) the relative proportion of RNA editing at a given editing and/or the percentage of an isoform are measuring by NGS in said biological sample.

Are also preferred the methods of the present invention, wherein in step a), the amplicon/nucleic sequence used for the detection of the RNA editing sites and/or the isoforms of the RNA transcript of said biomarker is obtained or obtainable with the set of primers listed in Table 1 for each of the selected biomarkers (SEQ ID NO. 1 to 36). Are also preferred the methods of the present invention, wherein in step a), the amplicon/nucleic sequence used for the detection of the RNA editing sites and/or the isoforms of the RNA transcript of said biomarker is obtained or obtainable with a set or a combination of sets of primers allowing to obtain amplicon(s) or nucleic acid sequence(s) including, or identical to, the amplicon(s) or nucleic acid sequence(s) obtainable by the set of primers listed in Table 1 (SEQ ID No.1 to SEQ ID No.36). Said amplicon or nucleic acid sequence used for the detection of the RNA editing sites and/or the isoforms of the RNA transcript of said biomarker could be the RNA transcript complete sequence itself.

In another aspect, the present invention is directed to a method for monitoring treatment for depression disorders, in a human subject from a blood sample of said subject, preferably for monitoring DEP, said method comprising:

A) diagnosing depression disorders in said human by the method for diagnosing depression disorders in a human patient according to the present invention before beginning of the treatment which is desired to be monitored.
B) repeating steps (a) to c) of the method for diagnosing depression disorders in a human patient according to the present invention, after a period of time during which said patient receives treatment for said depression, to obtain a post-treatment depression score;

C) comparing the post-treatment depression score from step (c) to:

- the depression score (i.e. Z value, probability value) obtained before the period of time during which said patient receives treatment for said depression, and
- to the depression score (control final value) for normal/healthy subjects, and classifying said treatment as being effective if the post-treatment depression score obtained in step B) is closer than the depression score obtained before the period of time during which said patient receives treatment to the depression score obtained for normal/healthy subjects.

In another aspect, the present invention is directed to a kit for determining whether a patient present a depression said kit comprising:

1) optionally, instructions to apply the method according to the invention as defined in the claims, in order to obtain the result value or depression score the analysis of which determining whether said patient present a mood disorder, preferably depression disorder; and

- a combination of at least two set of primers from the group of pairs of primers consisting of: SEQ ID No.1 to SEQ ID No.36, the selection of which being dependent of the biomarkers combination used for the mood disorder, preferably depression disorder, diagnostic;
or
- a combination of at least two sets of primers allowing to obtain amplicon(s) identical to, the amplicon(s) obtained by the at least two sets of primers from the group of pairs or primers consisting of SEQ ID NO: 1 to SEQ ID NO: 36.

The following examples and the figures and the legends hereinafter have been chosen to provide those skilled in the art with a complete description in order to be able to implement and use the present invention These examples are not intended to limit the scope of what the inventor considers to be its invention, nor are they intended to show that only the experiments hereinafter were carried out.

Other characteristics and advantages of the invention will emerge in the remainder of the description with the Examples, Figures and Tables, for which the legends are given herein below.

## BRIEF DESCRIPTION OF THE DRAWINGS AND THE TABLES

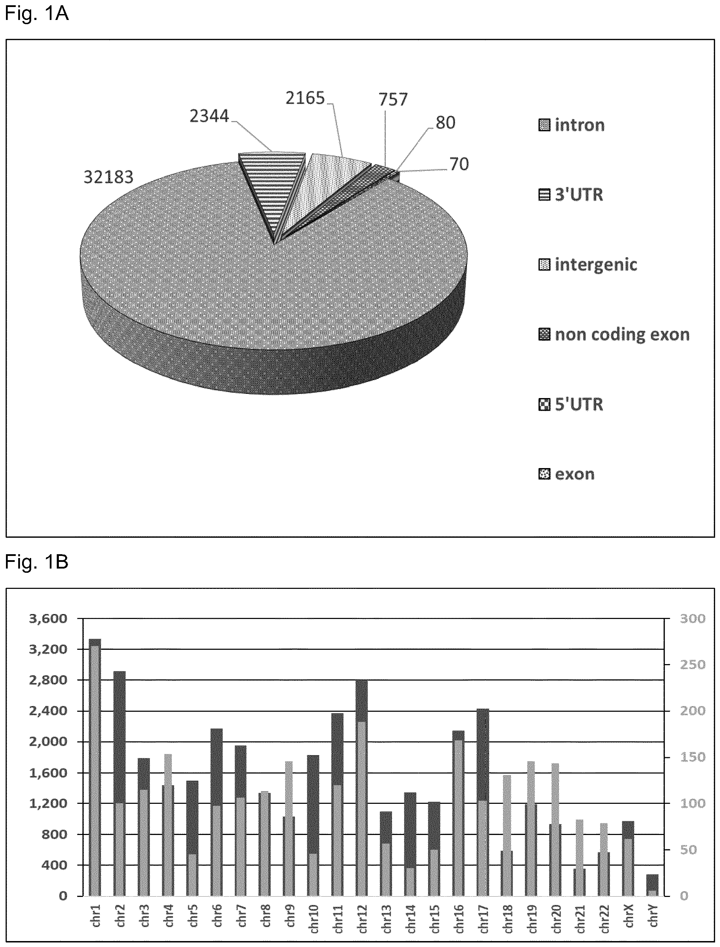

**Figures 1A-1D:** Identification and validation of differentially A-to-I RNA editing sites between healthy controls and DEP patients using RNA-seq data from human blood.

A: Genomic localization of detected editing sites
B: Repartition of A-to-I editing events or edited genes by chromosome.

Dark grey (left y-axis): Repartition of A-to-I editing events identified with a minimum coverage of 30 in the RNA-Seq study.
Light grey (right y-axis): Repartition of edited genes identified with a minimum coverage of 30 in the RNA-Seq study

C: Volcano plot of differentially edited sites between controls and DEP patients. The volcano plot shows the upregulated and downregulated differentially edited genes sites between depressed patients (DEP) group and the control group. For each plot, the x-axis represents the log (2)(fold change) (FC), and the y-axis represents -log 10(p values). Editing sites with an adjusted p value of less than 0.05 AND FC>5% were assigned as differentially edited and are indicated in green.
D: Alu Editing index between controls and depressed patients. Distribution of Alu editing index (AEI) values in controls and DEP patients. Data are the mean $\pm$ SEM. p-value of editing index was calculated using the Wilcoxon rank-sum test.

**Figure 2:** Principal component analysis of the 646 variants in 366 genes differentially edited between depressed patients and healthy controls identified in the RNA-Seq study.
The scatter plot visualizes the first, second and third principal components and respective variance percentages on the x-, y- and z-axis of ratio (fold change) of biomarkers. grey circles: DEP patients; black circle: Healthy Controls.

**Figure 3:** Protein-protein associations in the 14 differentially edited genes used in the prioritization cohorts.
Diagram shows the protein-protein interactions in the cluster of 14 genes selected in the prioritization cohort. Genes were annotated and colored regarding to their involvement in Biological processes.

: regulation of immune system process (GO:0002682) ;

: receptor signaling pathway via JAK-STAT (GO:0007259) ;

: regulation of immune response (GO:0050776)

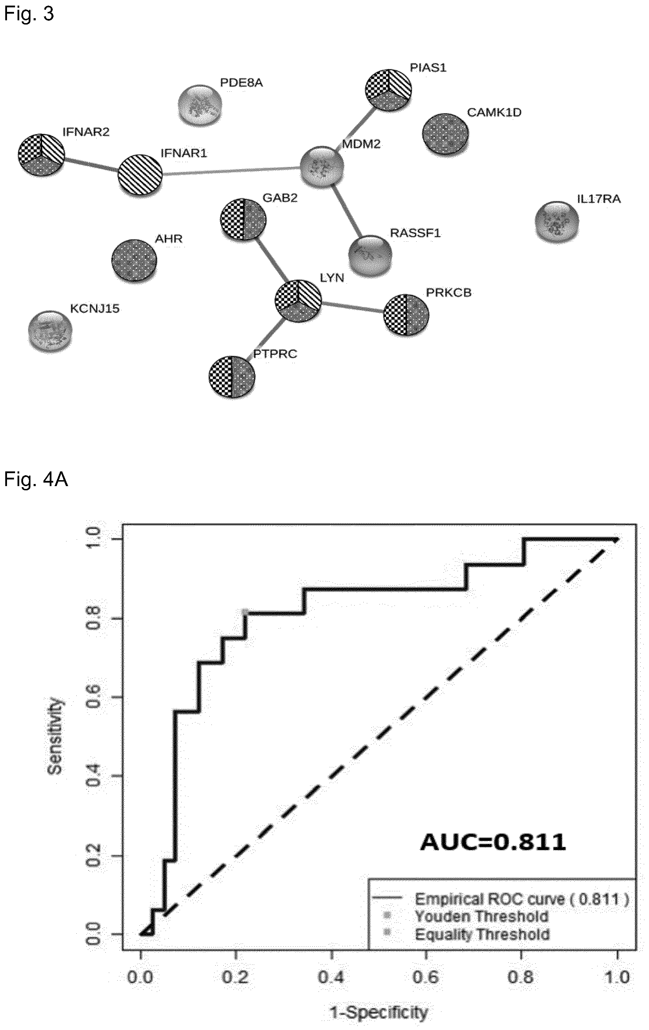

**Figures 4A-4D:** Examples of diagnostic performance of mRNA editing of the first prioritization cohort (n=76) analyzed by single-plex.

A: Example of diagnostic performances obtained by using 4 RNA editing sites in 3 different targets.
B: Example of diagnostic performances obtained by using 4 RNA editing sites in 2 different targets.
C: Example of diagnostic performances obtained by using 6 RNA editing sites in 4 different targets.
D: Example of diagnostic performances obtained by using 29 RNA editing sites present in 5 different targets.

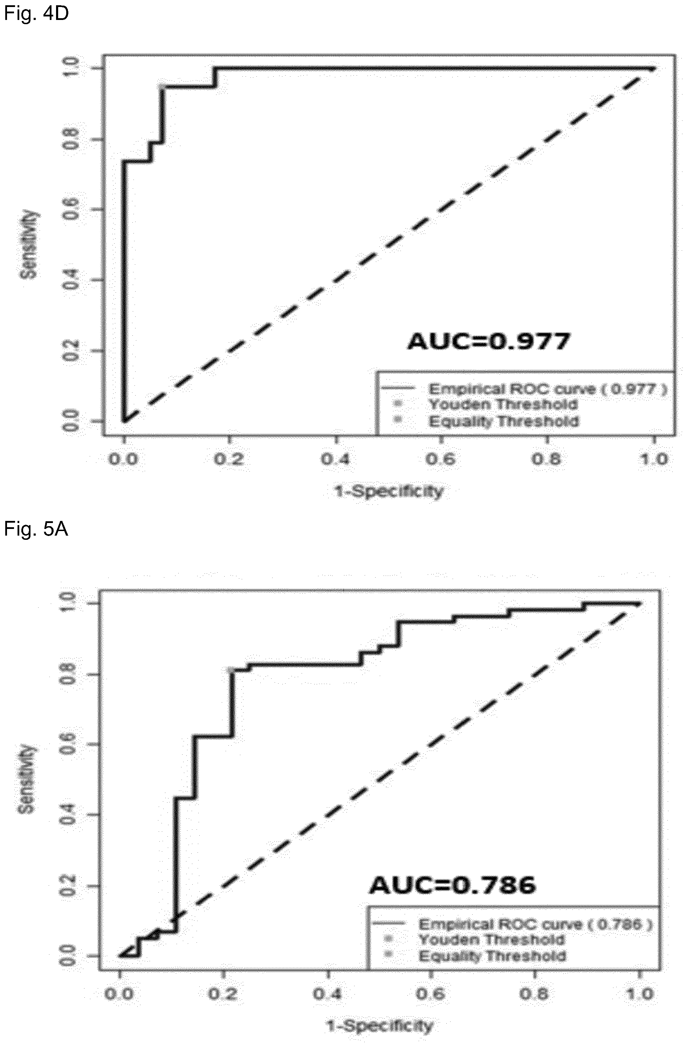

**Figures 5A**-4F: Diagnostic performance of mRNA editing of the second prioritization cohort (n=86) analyzed by multiplex sequencing.

A: Example of diagnostic performances obtained by using 2 RNA editing sites in 3 different targets.
B: Example of diagnostic performances obtained by using 3 RNA editing sites in 3 different targets.
C: Example of diagnostic performances obtained by using 4 RNA editing sites in 4 different targets.
D: Example of diagnostic performances obtained by using 5 RNA editing sites present in 4 different targets.
E: Example of diagnostic performances obtained by using 19 RNA editing sites in 8 different targets.
F: Example of diagnostic performances obtained by using 48 RNA editing sites present in 9 different targets.

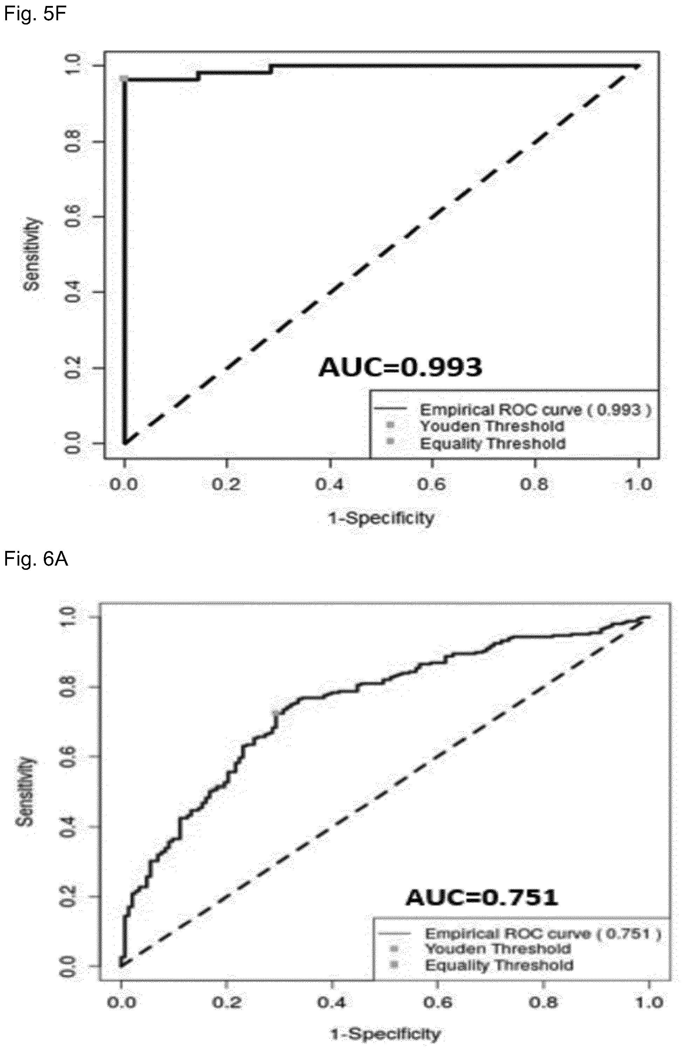

**Figures 6A-6C:** Diagnostic performance of mRNA editing of the validation cohort (n=411) analyzed by multiplex sequencing of 8 targets.

A: Example of diagnostic performances obtained by using 17 RNA editing sites in 5 different targets.
B: Example of diagnostic performances obtained by using 53 RNA editing sites in 8 different targets.
C: Example of diagnostic performances obtained by using 79 RNA editing sites in 8 different targets.

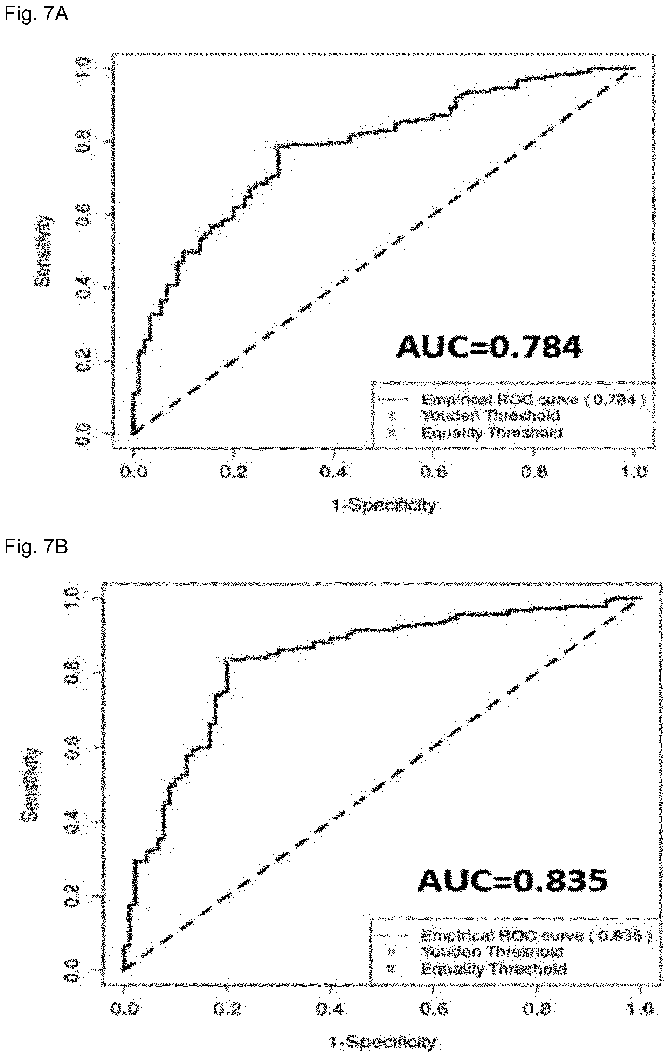

**Figures 7A-7E:** Diagnostic performance of mRNA editing of the female population of the validation cohort (n=277) analyzed by multiplex sequencing of 8 targets.

A: Example of diagnostic performances obtained by using 11 RNA editing sites in 4 different targets.
B: Example of diagnostic performances obtained by using 30 RNA editing sites in 6 different targets.
C: Example of diagnostic performances obtained by using 49 RNA editing sites in 8 different targets.
D: ROC curve obtained after machine-learning with the mROC method.
E: ROC curve obtained after machine-learning with the RandomForest method

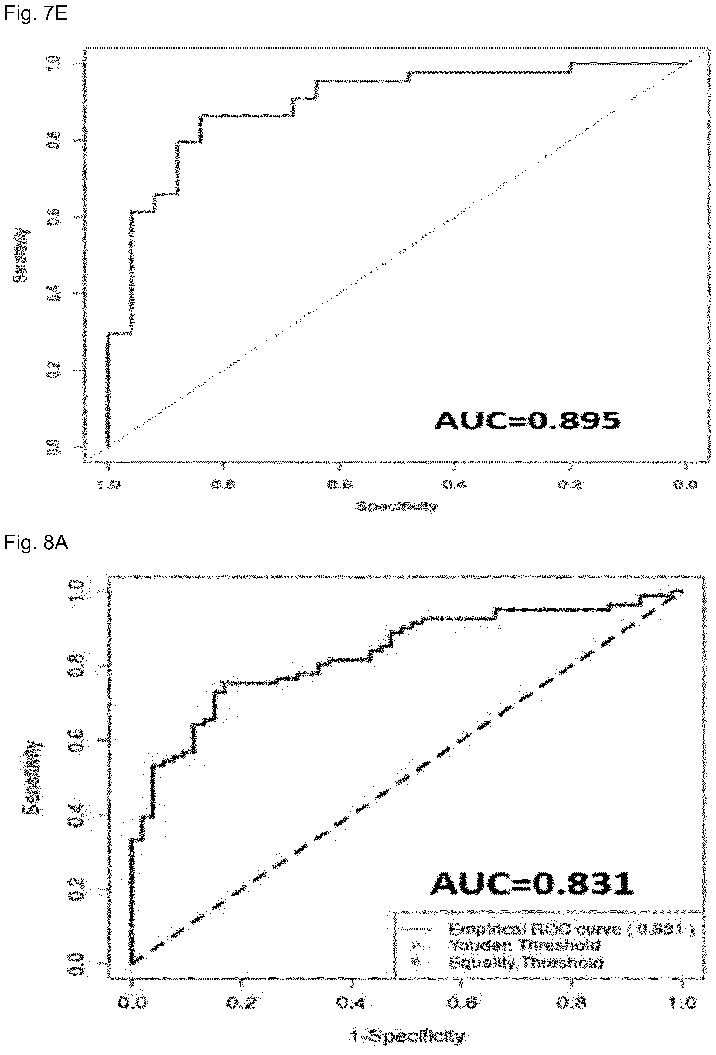

**Figures 8A-8E:** Diagnostic performance of mRNA editing of the male population of the validation cohort (n=134) analyzed by multiplex sequencing of 8 targets.

A: Example of diagnostic performances obtained by using 8 RNA editing sites in 6 different targets.
B: Example of diagnostic performances obtained by using 39 RNA editing sites in 7 different targets.
C: Example of diagnostic performances obtained by using 68 RNA editing sites in 8 different targets.
D: ROC curve obtained after machine-learning with the mROC method.

E: ROC curve obtained after machine-learning with the RandomForest method

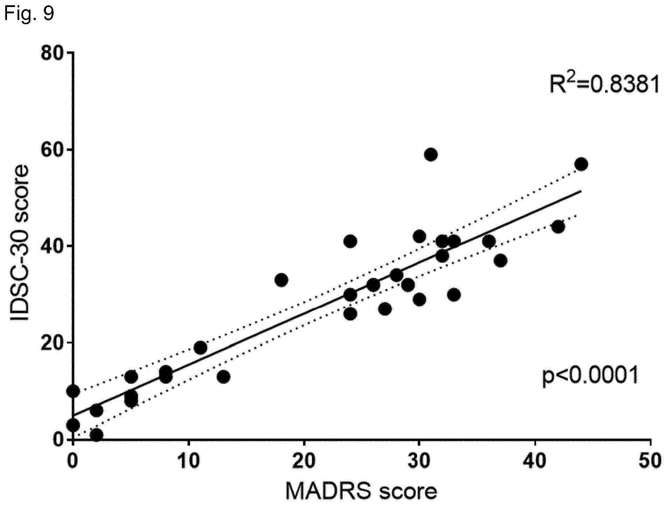

**Figure 9:** Correlation between clinical MADRS and IDS-C30 scores for the subjects included in the RNA-Seq study. Graph shows the 95% Intervals confidence. The Pearson correlation coefficient and p value are indicated.

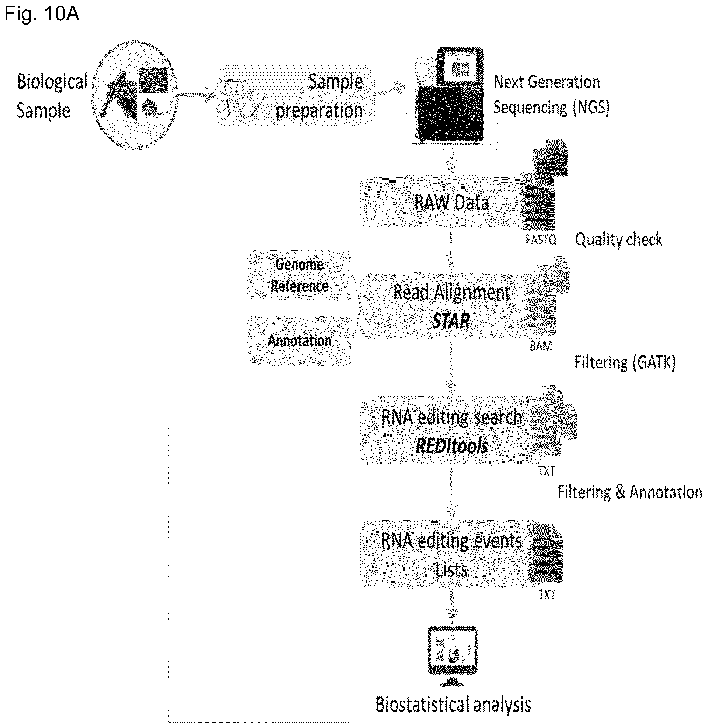

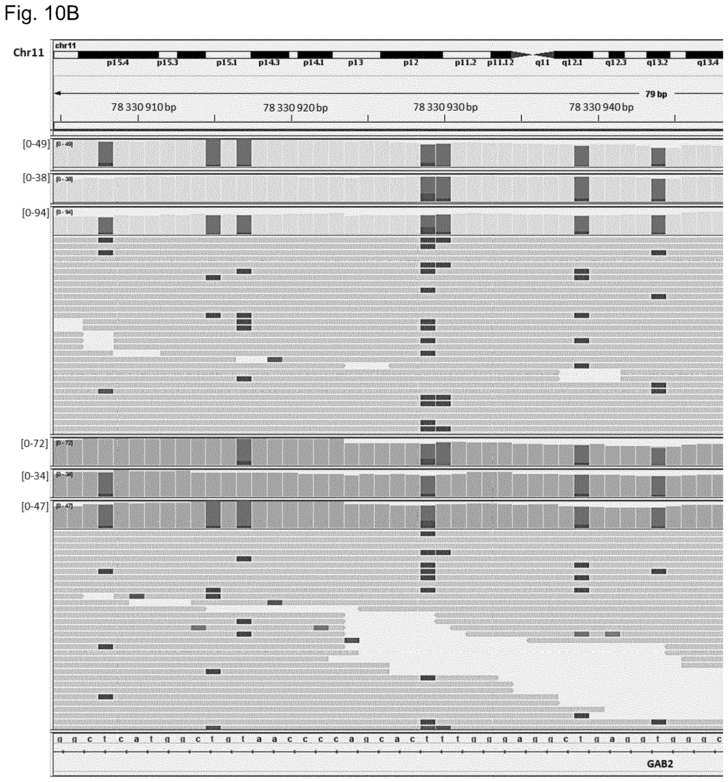

**Figure 10:** Overview of the Editome analysis pipeline

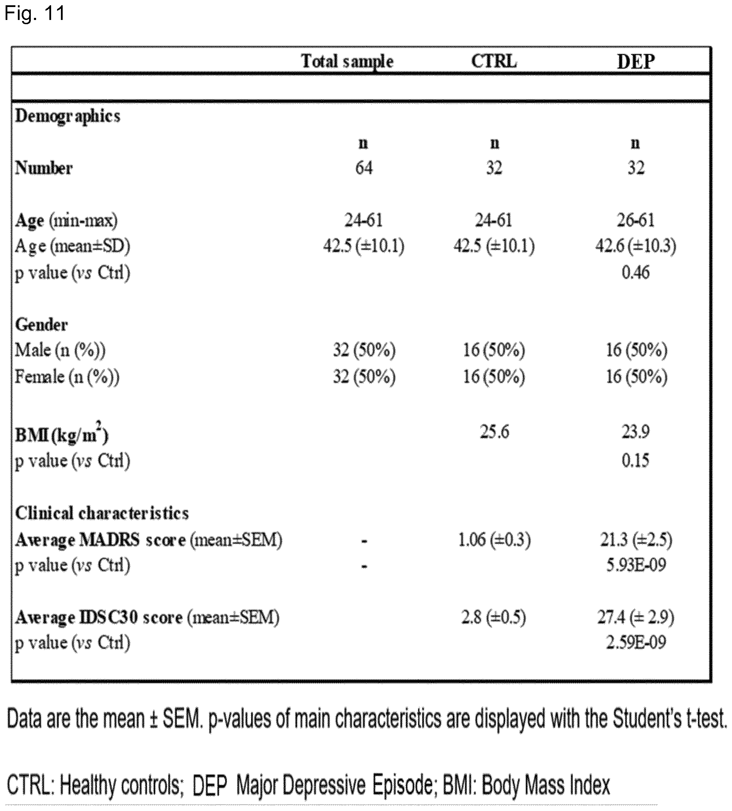

**Figure 11:** Demographic characteristics and psychiatric diagnosis of the study population included in the RNA-Seq study. Data are the mean ± SEM. p-values of main characteristics are displayed with the Student's t-test. CTRL: Healthy controls; DEP: Major depressive episode; BMI: Body Mass Index

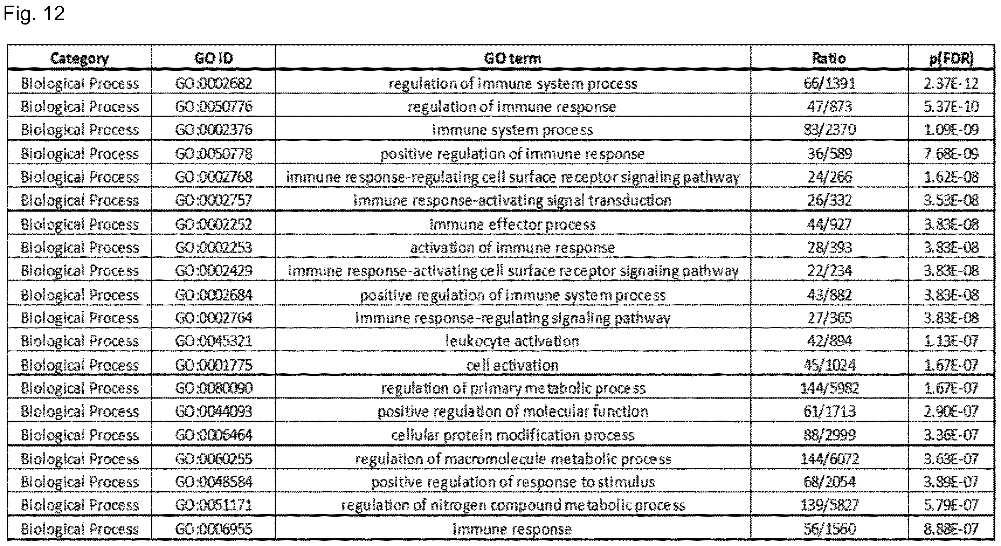

**Figure 12:** Functional categorization of the 366 genes differentially edited between DEP and controls based on gene ontology (GO) annotations.

Shown are the results of top 20 GO over-representation tests for enrichment of Biological Process GO terms with differentially edited genes. The input into the GO enrichment analysis tools was the list of 366 genes differentially edited between DEP and controls. The ratio of the number of genes/total number of genes included in the GO term and p-values corrected using the Benjamini-Hochberg method for multiple testing are presented. FDR, false discovery rate; GO, gene ontology.

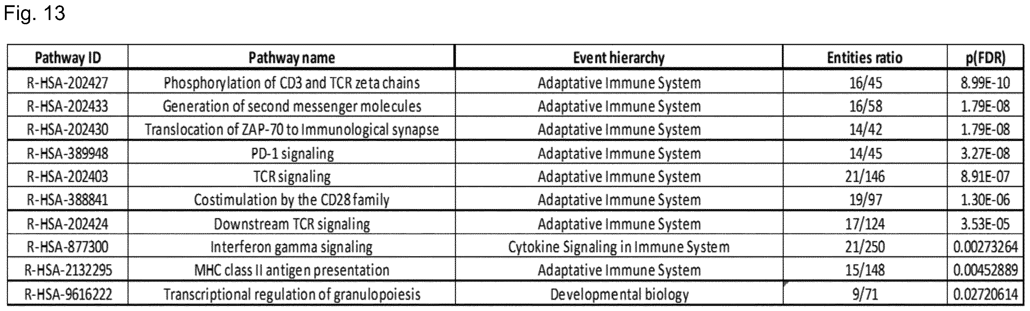

**Figure 13:** Signaling pathway enrichment analysis of the 366 genes differentially edited between DEP and controls.

Table 3 shows the significant pathways obtained performing over-representation analysis in Reactome. The ratio of the number of genes/total number of genes included in the pathway and p-value corrected using the Benjamini-Hochberg method for multiple testing are shown.

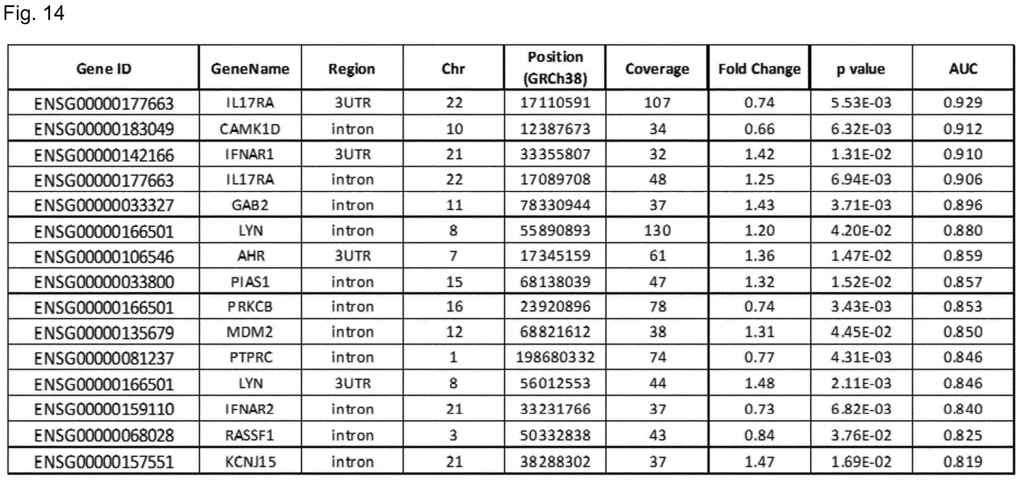

**Figure 14:** Gene and RNA editing variants analyzed in the prioritization cohort. Shown are the gene and position (GRCh38) of the editing variant, coverage following RNA-Seq, Fold Change between controls and DEP patients, p value and Area under the Curve calculated from RNA-Seq data. p-values were calculated using the Wilcoxon rank-sum test.

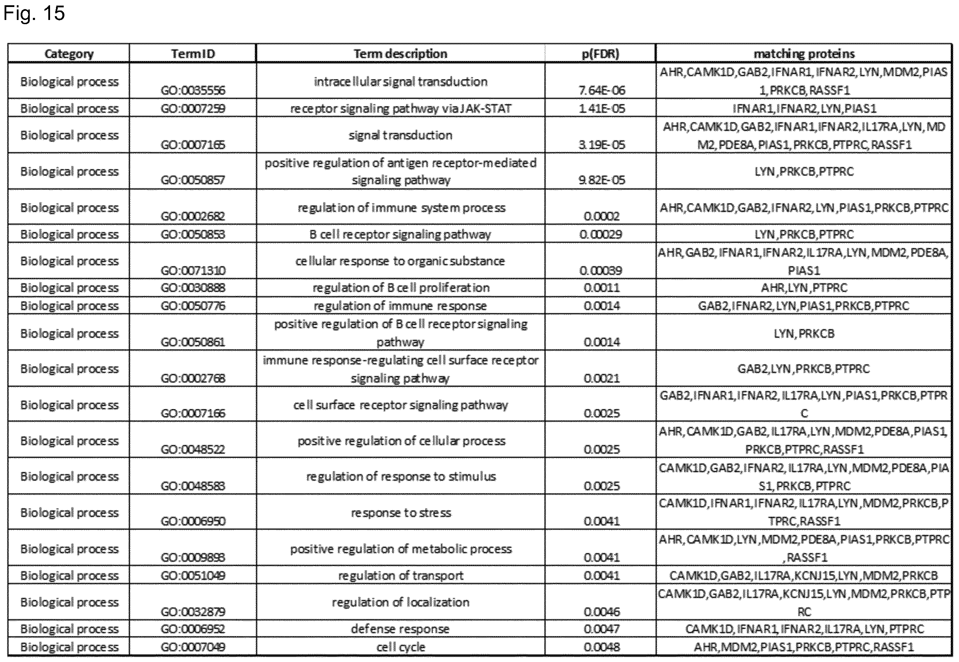

**Figure 15:** Functional categorization of the 14 differentially edited genes used in the prioritization cohorts. based on gene ontology (GO) annotations.

Shown are the results of top 20 GO over-representation tests for enrichment of Biological Process GO terms with differentially edited genes. The input into the GO enrichment analysis tools was the list of 14 genes differentially edited between DEP and controls analyzed in the prioritization cohorts. p-values corrected using the Benjamini-Hochberg method for multiple testing are presented. FDR, false discovery rate; GO, gene ontology.

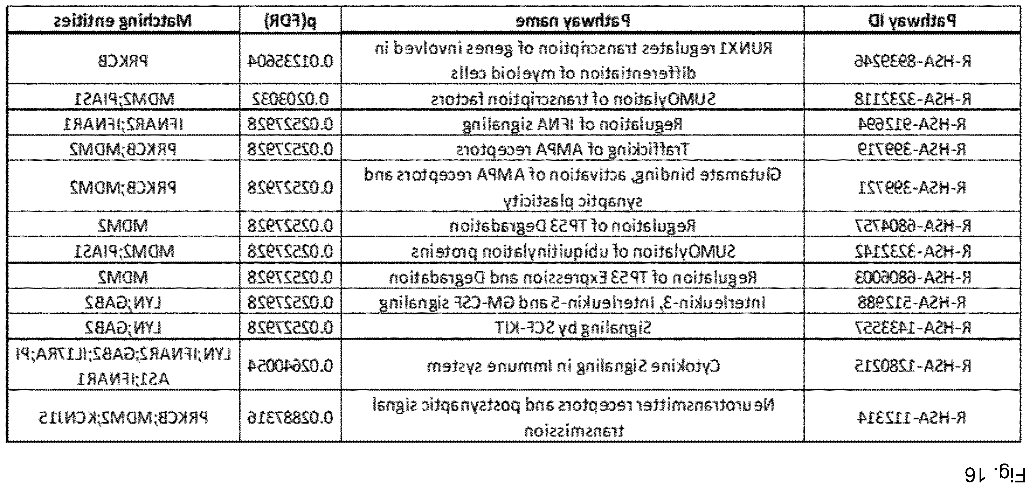

**Figure 16:** Signaling pathway enrichment analysis of the 14 genes analyzed in the prioritization cohorts.

Table shows the significant pathways obtained performing over-representation analysis in Reactome. p-value corrected using the Benjamini-Hochberg method for multiple testing are shown.

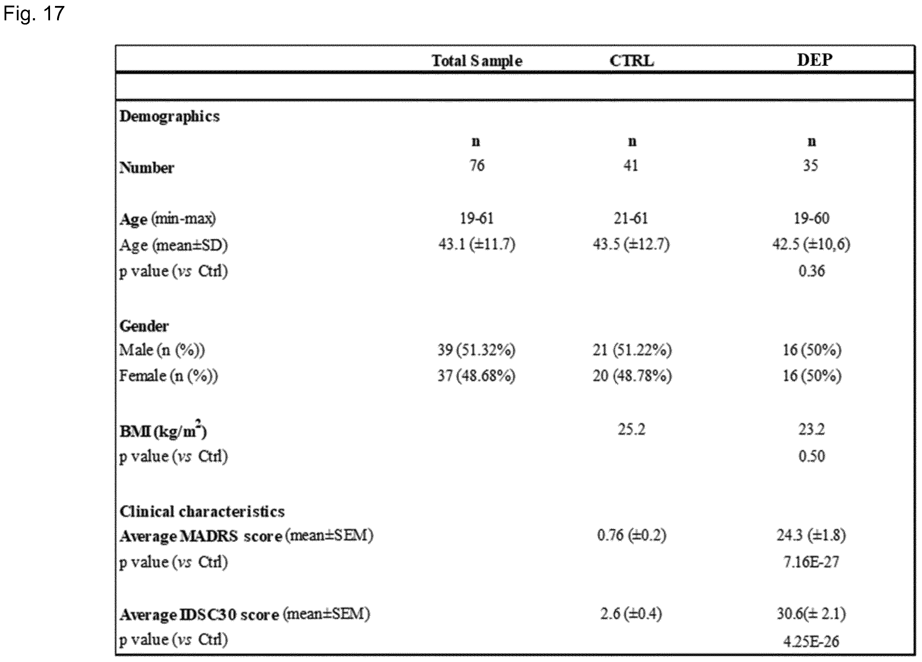

**Figure 17:** Demographic characteristics and psychiatric diagnosis of the study population included the first prioritization study analyzed in single-plex.

Data are the mean ± SEM. p-values of main characteristics are displayed with the Student's t-test. CTRL: Healthy controls; DEP: Major depressiveepisode; BMI: Body Mass Index

Testing are shown.

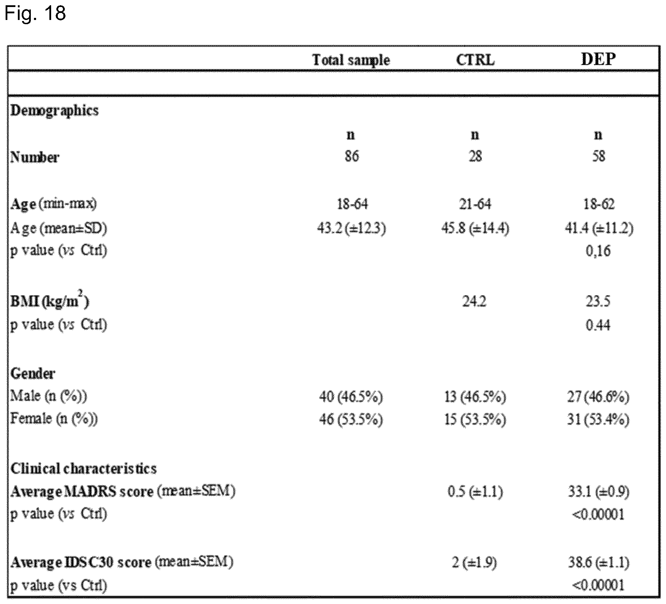

**Figure 18:** Demographic characteristics and psychiatric diagnosis of the study population included the second prioritization study analyzed by with multiplex sequencing.

Data are the mean ± SEM. p-values of main characteristics are displayed with the Student's t-test. CTRL: Healthy controls; DEP: Major depressive episode; BMI: Body Mass Index.

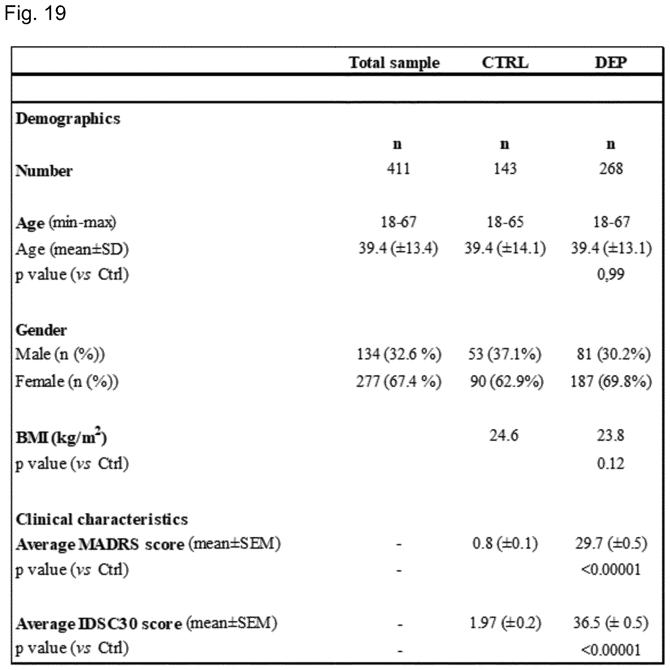

**Figure 19:** Demographic characteristics and psychiatric diagnosis of the study population included in the validation cohort. Data are the mean ± SEM. p-values of main characteristics are displayed with the Student's t-test. CTRL: Healthy controls; DEP: Major depressive episode; BMI: Body Mass Index

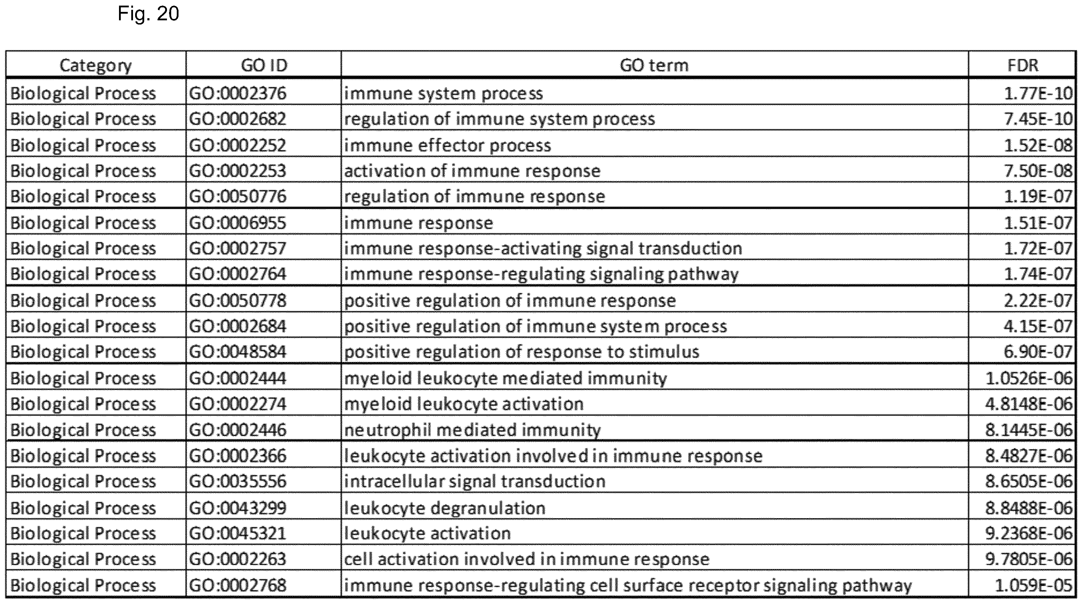

**Figure 20:** Functional categorization of the 366 genes differentially edited between DEP and controls based on g:Profiler annotations.

Shown are the results of top 20 GO over-representation tests for enrichment of Biological Process GO terms with differentially edited genes. The input into the g:Profiler was the list of 366 genes differentially edited between DEP and controls. The ratio of the number of genes/total number of genes included in the GO term and p-values corrected using the Benjamini-Hochberg method for multiple testing are presented. FDR, false discovery rate; GO, gene ontology.

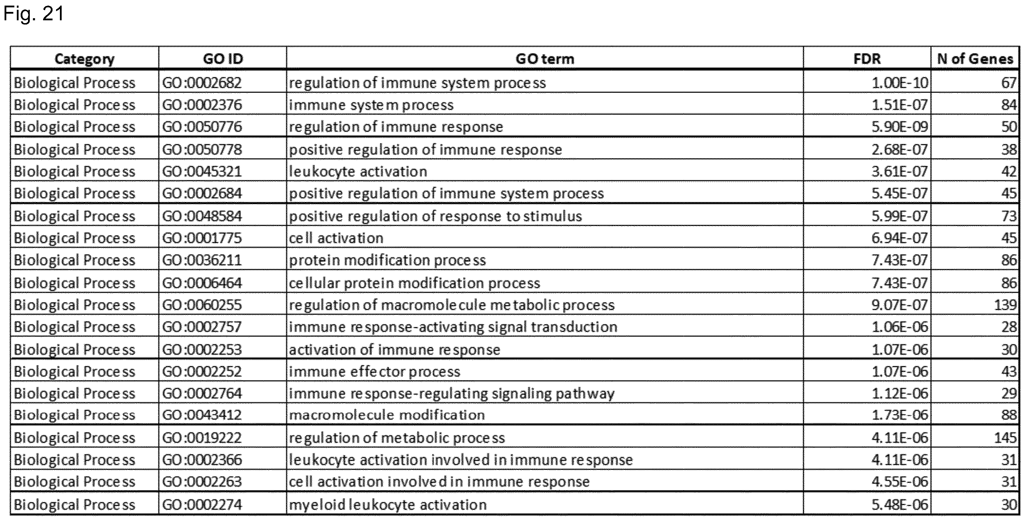

**Figure 21:** Functional categorization of the 366 genes differentially edited between DEP and controls based on GOnet annotations.

Shown are the results of top 20 GO over-representation tests for enrichment of Biological Process GO terms with differentially edited genes. The input into the GOnet was the list of 366 genes differentially edited between DEP and controls. The ratio of the number of genes/total number of genes included in the GO term and p-values corrected using the Benjamini-Hochberg method for multiple testing are presented. FDR, false discovery rate; GO, gene ontology.

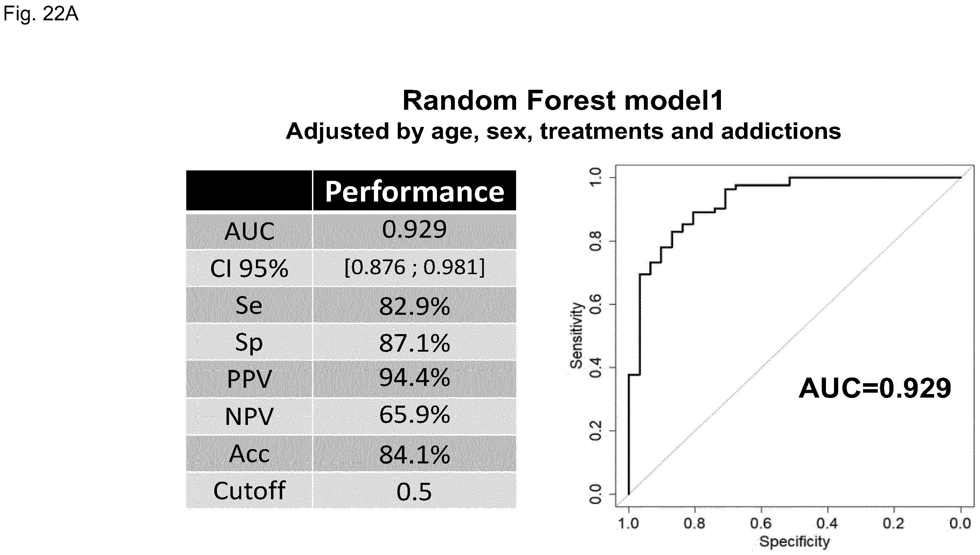

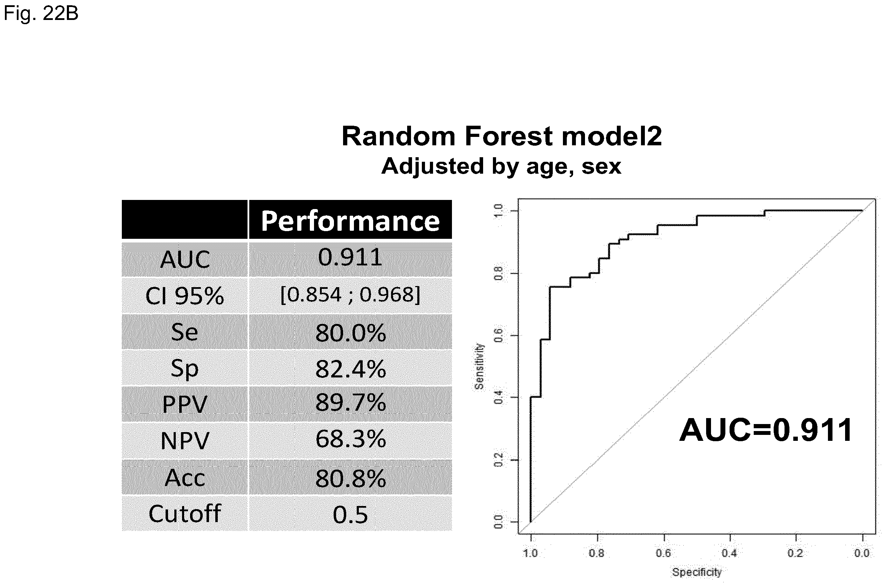

**Figures 22A and 22B:** Diagnostic performance of mRNA editing of the validation cohort (n=411) analyzed by multiplex sequencing of 8 targets using randomForest.

A. Receiver operating characteristic curve and diagnostic performances for depression with the first model adjusted by age, sex, psychiatric treatment and addiction. Plotted are the probabilities for the correct response of the tested sets
B. Receiver operating characteristic curve and diagnostic performances for depression with the second model adjusted by age and sex. Plotted are the probabilities for the correct response of the tested sets

## EXAMPLE 1: Materials and Methods

### Subjects and clinical assessment

Depressed patients (n=268) were recruited from the outpatients of the Department of Emergency Psychiatry and Post-Acute Care (CHRU of Montpellier) according to the principles of the Helsinki Declaration of 1975 and its successive updates. This study was approved by the French local Ethical Committee (CPP Sud-Méditerranée IV in Montpellier, CPP No.A01978-41). All participants, aged between 18 and 65 years, understood and signed a written informed consent before entering the study. The study was approved by the local Institutional Review Board, according to the approval requirements and good clinical practice. All patients met the DEP criteria in Diagnostic and Statistical Manual of Mental disorders IV (DSM-IV) using the Mini-International Neuropsychiatric Interview. The presence or absence of psychiatric diagnoses and mood states at time entering the study were confirmed by trained psychiatrists. During the standardized interview, psychiatrists managed the French version of the Montgomery-Åsberg Depression Rating Scale (MADRS) and the 30-item Inventory of Depressive Symptomatology, Clinician Rated (IDS-C30) to score depression. DEP patients, all Caucasian, were recruited among the outpatients of the Department of Emergency Psychiatry Post-Acute Care (CHRU of Montpellier). Age-, race- and sex-matched control subjects (n=143) were recruited from a list of volunteers from the Clinical Investigation Center (CHRU of Montpellier).

### RNA extraction and qualification from whole blood

A volume of 4 ml of whole blood from each patient was retrieved in PAXgene™ blood RNA tubes, which are optimized for stabilization of RNA, and stored at -20°C before being transferred to -80°C. Samples were distributed randomly in the different sets of extractions. Blood samples were thawed at 4°C overnight and centrifuged 10 min at 3000g. The pellet was washed with 4ml of DPBS 1X, resuspended in 400μl of DPBS 1X. Samples total RNAs were isolated using the MagNA Pure 96 Cellular RNA Large Volume Kit (LifeScience), according to the manufacturer's automated protocol. RNAs were eluted in 100μl MagNA Pure 96 Elution Buffer. During sample preparation and RNA extraction, standard precautions were taken to avoid RNA degradation by RNAses. Total RNA concentrations and quality levels were determined with Qubit Fluorometer (Invitrogen) and LabChip (Perkin-Elmer, HT RNA Reagent Kit) instruments, respectively.

### Library preparation for RNA-Sequencing

For RNA library preparation from blood derived RNAs, we chose a specific strategy aiming at depleting in a single step both ribosomal RNA and globin RNAs, which are two forms of abundant RNA, in order to enrich the library with total RNAs (mRNAs). Total RNAs were preferred to messenger RNAs, as it has been shown that RNA editing in blood cells was enriched in intronic region of repetitive elements, which are removed during splicing to produce messenger RNAs. We thus used a TruSeq Stranded Total RNA library kit (Illumina) with Ribo-Zero Globin, which is specifically tailored for blood samples, according to the manufacturer's instructions. Briefly, 300ng total RNAs were depleted in ribosomal RNA and globin RNAs using rRNA and globin depletion probes in combination with magnetic beads. Following purification, the RNAs were fragmented into 250bp fragments in average using divalent cations under elevated temperature. First strand cDNA synthesis was then achieved using Superscript II Reverse Transcriptase (Thermo Fisher Scientific) and random primers. Next, second strand cDNA synthesis is performed using DNA Polymerase I and RNase H. Importantly, during second strand cDNA synthesis dUTP is incorporated in place of dTTP to generate a strand-specific library. Following second strand cDNA synthesis, the 3' ends of the blunt fragments are 3' adenylated before adapter ligation. Importantly, each index has been attributed to all groups to prevent putative bias due to unspecific effect of index on the depth of sequencing. DNA fragments having adapter molecules on both ends were then amplified by PCR to increase the amount of DNA in the library. Quality controls were then performed using both Qubit Fluorometer (Invitrogen) and LabChip (Perkin-Elmer, HT DNA 5K Reagent Kit) instruments for quantification of the DNA library templates and quality control analysis, respectively. Samples were then pooled into a library and purified using Magbio PCR cleanup system. The library was denatured using 0.1M NaOH and sequenced on an Illumina NextSeq 500/550 High-Output run using paired-end chemistry with 75-bp read length.

**Processing of RNA-Seq data, differential expression analysis and editome analysis**

Paired-end reads were generated using an Illumina NextSeq 500 and demultiplexed using bcl2fastq (version 2;17.1.14, Illumina). Sequencing quality was performed using FastQC software (version 0.11.7, https://github.com/s-andrews/FastQC). Read alignments were done using the human genome version hg38 with STAR aligner[6], following by realignment by Genome Analysis Tool Kit (GATK version 4.1.4.0)[7] removal of PCR duplicates and final single nucleotides variants (SNV) calling. Identification and quantification of A-to-I editing events was performed using RNAEditor (Version 1.0)[8]. Further filtering and functional annotation of edited events was then performed with ANNOVAR[9], RepeatMasker[10] and BiomaRt[11].

**Targeted Next Generation Sequencing Library preparation and sequencing**

For Next Generation Sequencing (NGS) library preparation, we chose a multiplexed targeted approach to selectively sequence the region of interest within each relevant target. Validated PCR primers were used to amplify the region of interest by PCR (Table 20). For PCR amplification, the Q5 Hot Start High Fidelity enzyme (New England Biolabs) was used according to manufacturer guidelines. The PCR reaction was performed on a Peqstar 96x thermocycler using optimized PCR protocol. Both quantity and quality of the PCR product were assessed. Purity of the amplicon was determined with Nucleic Acid Analyzer (LabChipGx, Perkin Elmer) and quantification was performed using the fluorescence-based Qubit method. After quality control, the PCR reactions were purified using magnetic beads (High Prep PCR MAGbio system, Mokascience). DNA was then quantified using Qubit system and purification yield was calculated. Next, samples were individually indexed by PCR amplification using Q5 Hot start High fidelity PCR enzyme and the Illumina 96 Indexes kit (Nextera XT index kit; Illumina). Samples were then pooled into a library and purified using Magbio PCR cleanup system. The library was denatured using 0.1M NaOH and loaded onto a sequencing cartridge (Illumina MiSeq Reagent Kit V3 or Illumina NextSeq 500/550 Mid-Output) according to Illumina's guidelines. A commercial total RNA pool from human blood peripheral leukocytes (Clontech, #636592) was incorporated into the libraries to determine variability between different sequencing flow cells during the course of the experiment. NGS libraries were spiked in to introduce library diversity using PhiX Control V3 (Illumina) and sequenced (single-read sequencing, read length 150bp) at standard concentrations using deep sequencing (>50K sequences per sample).

**Bioinformatics analysis of targeted sequencing data**

The sequencing data were downloaded from the NextSeq or MiSeq sequencers (Illumina). Sequencing quality was performed using FastQC software (version 0.11.7 https://github.com/s-andrews/FastQC/). A minimal sequencing depth of 20 000 reads for each sample was considered for further analysis. A pretreatment step was performed consisting of removing adapter sequences and filtering of the sequences according to their size and quality score. Short reads (<100nts) and reads with an average QC<20 were removed. To improve sequence alignment quality of the sequences, flexible read trimming and filtering tools for Illumina NGS data were used (fastx_toolkit v0.0.14 and prinseq version 0.20.4). After performing pre-processing steps, an additional quality control of each cleaned fastq file was carried out prior further analysis.

Alignment of the processed reads was performed using bowtie2[12] (version 2.2.9) with end-to-end sensitive mode. The alignment was done to the latest reference human genome sequence (GRCh38). Non-unique alignments, reads, unaligned or reads containing insertion/deletion (INDEL) were removed from downstream analysis by SAMtools software[13] (version 1.7). SAMtools mpileup[14] was used for SVN calling. Finding edited position in the alignment was done by using in-house script's in order to count the number of different nucleotides in each genomic location. For each position the percentage of reads supporting nucleotide 'G' instead of nucleotide 'A' is calculated [Number of 'G' reads/ (Number of 'G' reads + Number of 'A' reads)*100]. An editing rate > 0.1 are automatically detected by the script and are considered as 'A-to-I edition site'. The last stage is to compute the percentage of all possible isoforms of each transcript. By definition the relative proportion of RNA editing at a given editing 'site' represents the sum of editing modifications measured at this unique genomic coordinate. Conversely, an mRNA isoform is a unique molecule that may or may not contain multiple editing modifications on the same transcript. For example for a given transcript, the mRNA isoform BC contains an A-to-I modification on both site B and site C within the same transcript. A relative proportion of at least 0.1% was set as the threshold in order to be included in the analysis.

**Statistical analysis of data**

All statistics and figures were computed with the "R/Bioconductor" statistical open source software[15] or GraphPad Prism software (version 7.0). Biomarkers (i.e RNA editing sites and isoforms of target genes and mRNA expression of ADARs) values are usually presented as mean $\pm$ standard error of the mean (SEM). In order to guarantee normally distributed data, all biomarker could be transformed using bestNormalize R package (version 1.4.2). A differential analysis was carried out using the most appropriate test between the Mann-Whitney rank-sum test, Student's t-test or Welch's t-test according to normality and sample variance distribution. A p-value below 0.05 was considered as statistically significant.

The biomarker diagnostic performance could be characterized by: sensitivity, which represents its ability to detect the 'depressed' group and specificity which represents its ability to detect the 'control' group. The results of the evaluation of a diagnostic test can be summarized in a 2x2 contingency table comparing these two well-defined groups. By fixing a cut-off, the two groups could be classified into categories according to the results of the test, categorized as either positive or negative. Given a particular biomarker, one can identify a number of A patients with a positive test result among the depressed group (the "True Positive": TP) and B patients with a negative test result among the 'control' group (the "True Negative": TN). In the same fashion, C patients with a negative test result among the 'depressed' group (the "False Negative": FN) and D patients with a positive test result among the 'control' group (the "False Positive": FP) are observed. Sensitivity is defined as TP/(TP+FN); which is herein referred to as the "true positive rate". Specificity is defined as TN/(TN+FP); which is herein referred to as the "true negative rate".

The accuracy of each biomarker and its discriminatory power was evaluated using a Receiving Operating Characteristics (ROC) analysis[16]. ROC curves are the graphical visualization of the reciprocal relation between the sensitivity (Se) and the specificity (Sp) of a test for various values. In addition, all biomarkers were combined with each other to evaluate the potential increase in sensibility and specificity using multiple approaches as for example mROC program[17] or RandomForest[18].

mROC is a dedicated program to identify the linear combination, which maximizes the AUC (Area Under the Curve) ROC. The equation for the respective combination is provided and can be used as a new virtual marker Z, as follows:

$$Z = a \times \text{biomarker } 1 + b \times \text{biomarker } 2 + c \times \text{biomarker } 3$$

where a, b and c are calculated coefficients and biomarkers 1,2 and 3 are the level of the considered biomarker.

Random Forest (RF) was applied as previously to assess the RNA editing isoforms/sites combinations. This method combines Breiman's "bagging" idea and the random selection of features in order to construct a collection of decision trees with controlled variance. Random forests can be used to rank the importance of editing isoforms/sites and to combine the best isoforms. Multiple down-sizing (MultDS) asymmetric bagging with embedded variable classifier was implemented. This method tries to take advantage of the whole information of majority calls by multiple down sampling but keeping the minority class fix. Our algorithm generated 100 random forest from the training set, each containing the same fix number of the minority class and equal or adjusted number of the randomly sampled, generating nearly balanced trees. For classification of new data, the results afterwards were combined by majority voting of the trained 100 random forest. To estimate RF parameters for each trained 100 randomForest, a 10-fold cross-validation were applied. The implementation was done using the R random Forest package (version 4.6-14) and R caret package (version 6.0-84).

## EXAMPLE 2: Results

**Characteristics of the Discovery Study Samples** Patients and control subjects included in the RNA-Seq samples (n=32 for each condition) were matched with respect to all variables, e.g. sex, age, or BMI, which were not statistically different between groups (Figure 11). We chose two different clinical evaluations to confirm the presence or absence of psychiatric diagnoses, namely MADRS and IDSC-30, both designed by the American Psychiatry Association Diagnostic and Statistical Manual of Mental Disorders. Healthy controls did not show any sign of depressed mood at the time on sample collection (MADRS<7 and IDSC-30<10). Depressed patients (DEP) had significantly higher scores in both MADRS (p-value 5.93E-09) and IDSC-30 (p-value 2.59E-09) (Table 1). Moreover, we observed a significant association (r2=0.84, p<0.0001) between the two clinical evaluations (Figure 9), suggesting a correct assignment of patients and a relative homogeneity in the scoring of depression.

## Editome analysis

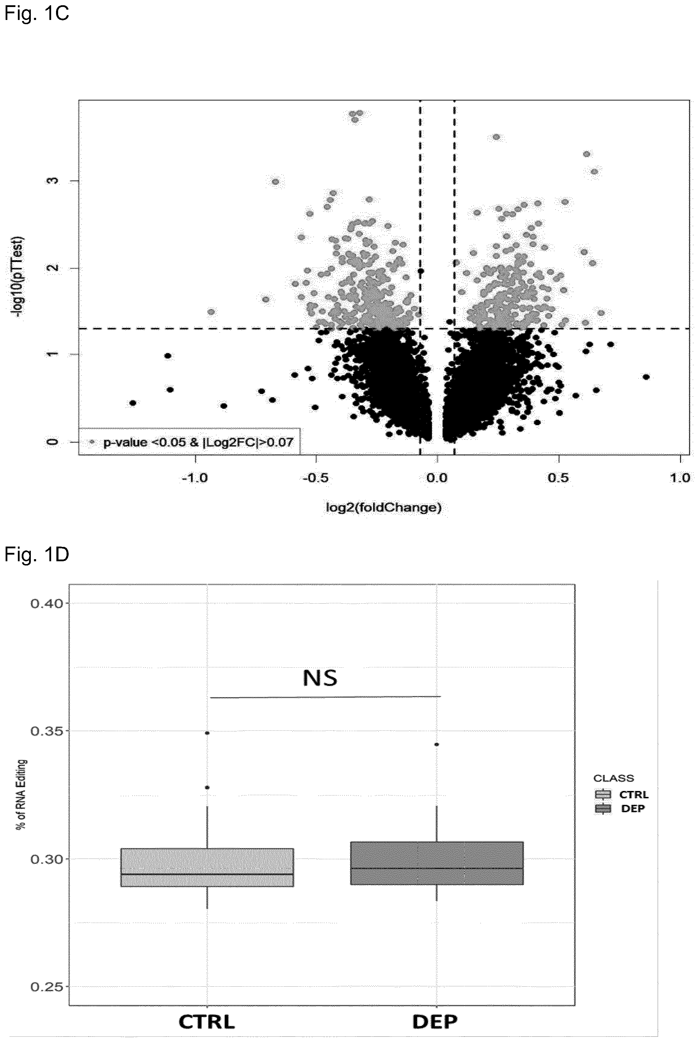

To obtain a global landscape of the modification of RNA editing events in depressed patients, we analyzed the RNA-Seq dataset using an in-house editome analysis pipeline (see methods and figure 10). We identified 37, 599 A-to-I differentially edited positions with a minimum coverage of 30x, which ensures a high degree of confidence at particular base positions. The coverage describes the average number of reads that align to, or "cover," known reference bases. Most of the edited position (32, 183 sites, 85.6% of total) were present in introns (Figure 1A), consistent with other studies in human tissues. The percentage of edited position in 3' untranslated regions (3'UTR) of mRNAs was 6.2% (2344 sites), in line with previous reports using RNA-Seq. The number of editing sites had a homogeneous repartition all over the genome, though edited genes on chromosomes 4, 9, 18, 19, 20, 21 and 22 were slightly overrepresented (Figure 1B). We then performed a differential analysis to identify sites whose editing could be specifically different between DEP and healthy controls. After applying pre-specified quality criteria (exclusion of intergenic sites, coverage >30; AUC>0.6; 0.95>FoldChange>1.05 and p<.05,), we identified 646 variants differentially edited between depressed patients and healthy controls (Figure 1C), representing 366 genes. The quality criteria were set in order to provide potential diagnostic biomarkers (BMKs) for depressive disorders with clinical usefulness. Importantly, no difference in global RNA editing was observed between patients and controls. Indeed, we calculated the global Alu editing index (AEI), which is a measure of the overall rate of RNA editing in Alu repeats. Alu repeats are by far the most common edited region in the human genome, and thus are indicative of the global editing rate. We found no significant differences in AEI between controls and depressed patients (AEI control mean=0.300±0.002, AEI DEP mean=0.299±0.003, p value=0.57, figure 1D). As the Alu Editing index characterizes the weighted average editing level across all expressed Alu sequences, these data suggest that differential RNA editing was target-specific rather than dependent on the disease status.

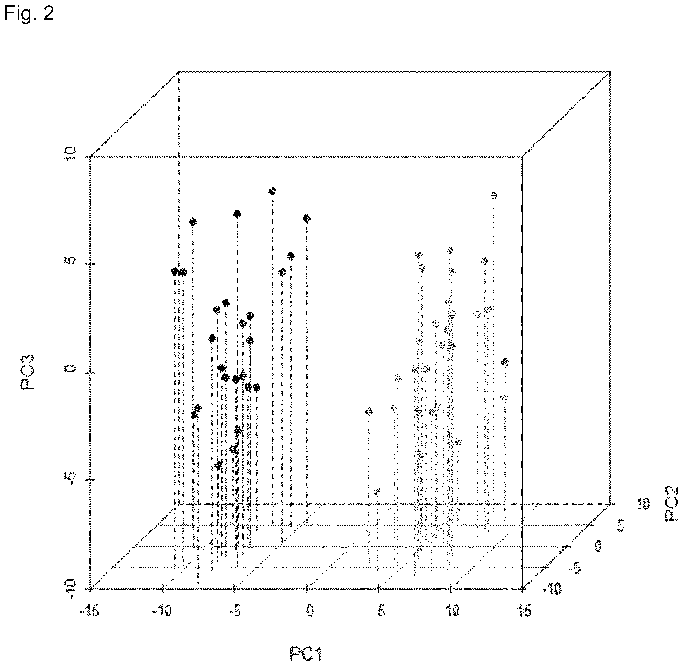

GSEA (enrichment analysis on gene sets) on these 366 genes using gene ontology tools[19] displayed a strong term enrichment (false discovery rate <0.05) for biological processes including multiple immune categories (response, activation and regulation), cell activation and metabolic processes (Figure 12). This analysis was replicated with other tools, such as G:profiler[20] (Figure 9) or GOnet[21] (Figure 10), and consistently indicated a strong enrichment in biological processes related to immune system. Moreover the reactome pathways analysis of those 366 genes revealed a strong enrichment in pathways related to the immune system, i.e. TCR signaling, Translocation of ZAP-70 to Immunological synapse or Interferon gamma signaling (Figure 13). Noteworthy, Principal Component Analysis (PCA) showed that this subset of the 646 editing variants representing 366 genes clearly discriminated controls and depressed patients in 2 separate groups (Figure 2), suggesting that editing variants in those genes could be used in combination to selectively discriminate between depressed patients and healthy controls.

To further narrow down the list of genes, much more stringent quality criteria were used (coverage >30; AUC>0.8; 0.8>FoldChange>1.20 and p<0.05). The database of these markers was then manually curated to reach a combination of several biomarkers representing different biological mechanisms. Using these secondary criteria, a list of 15 variants, representing 13 genes (Figure 14), was selected for both diagnostic performance and for their potential role in the immune system or psychiatric disease biology and to be studied in a subsequent prioritization cohort. Additionally, we nominated one other target, PDE8A, for which previous data indicated altered editing in suicide brain and in blood of drug-induced depression in HCV patients[5,22]. Protein-protein association networks analysis with STRING database[23] revealed a network with strong enrichment in protein-protein interaction (PPI pvalue <4.5.10-6). The interactions inside STRING were based on evidence and consist of direct (physical) and indirect (functional) interactions; we focused here on a minimum required interaction score of 0.7, indicative of high confidence in actual protein-protein interactions. Functional protein association network analysis of the 14 selected genes using gene ontology tools[19] also showed a strong enrichment in genes related to: regulation of immune system process (8 genes, GO:0002682; p(FDR=4.10-4), regulation of immune response, (6 genes, (GO:0050776, p(FDR=2.2.10-3)) or receptor signaling pathway via JAK-STAT (4 genes, GO:0007259), which is typically triggered following interferon-alpha stimulation (Figure 3 and Figure 15). Furthermore, Reactome pathways analysis indicated that those 14 genes were enriched in pathways related to immune system, (e.g. Regulation of IFNA signaling (HSA-912694, p(FDR)<0.025) or Cytokine signaling (HSA-1280215, p(FDR)<0.026)) and neurotransmitter regulation (e.g. Trafficking of AMPA receptors (HSA-399719, p(FDR)<0.025), glutamate binding and synaptic plasticity (HAS-399721, p(FDR)<0.025) or Neurotransmitter receptors and postsynaptic signal transmission (HSA-112314, p(FDR)<0.029)) (Figure 16). Interestingly, the Top pathway identified in the Reactome analysis (p(FDR)<0.012) is linked to the transcription factor Runx1, which has recently been shown to mediate the effect of chronic social defeat stress in a mouse depression model through regulation of the miR-30 Family of miRNAs.

**Target description**

**PRKCB:** Protein Kinase C Beta (PRKCB), which belongs to the family of serine- and threonine-specific protein kinases, is well known to play a crucial role in the immune system, as Mice knockout for PKCB develop immunodeficiency characterized by impaired B-cell activation, B cell Receptor response, and defects in T-cell-independent immune responses. PRKCB also act as a regulator of the HPA axis response to stress[24]. An association study of 44 candidate genes with depressive and anxiety symptoms in post-partum women revealed an association between PRKCB and major depression. Indeed, PRKCB interacts with and phosphorylates CREB1, which directly regulates the expression of several genes involved in stress response in the brain, such as BDNF, the BDNF receptor Trk-b, and the glucocorticoid receptor[25].

**MDM2:** MDM2 is a primary cellular inhibitor of p53, and might be a target for anticancer treatment. However, MDM2 also catalyzes ubiquitination of $\beta$-arrestin, which is a target for antidepressants. In the CNS, MDM2 is involved in AMPAR (glutamate receptor) surface expression during synaptic plasticity[26]. Interestingly, RNA editing in MDM2 has been shown to regulate protein levels. Indeed, increased RNA editing at the 3' UTR of MDM2 abolishes microRNA-mediated repression, which may increase its mRNA levels.

**PIAS1:** PIAS1 (Protein Inhibitor of Activated STAT 1) function as a SUMO ligase which blocks the DNA binding activity of Stat1 and inhibited Stat1-mediated gene activation in response to interferon. Sumoylation of transcription factors (HSA-3232118) is one of the major Reactome pathways in the 14 genes selected for analysis in the prioritization cohort (Figure 16). PIAS1 selectively inhibits interferon-inducible genes and is an important player in innate immunity[27]. In addition, Pias1 interacts with the presynaptic Metabotropic Glutamate Receptor 8 (mGluR8) and add a post transcriptional modification by sumoylation, suggesting that PIAS1 might have regulatory functions in targeting and modulating mGluR8 receptor signaling.

**IFNAR1:** A wealth of studies have linked interferons to inflammation-induced changes in brain function and depression, at least in part though the effect of IL-6, CXCL10 or by induction of indoleamine 2,3-dioxygenase1 (IDO1)[28]. Moreover, it has been shown that polymorphisms in the promoter region of the IFN-alpha/beta receptor 1 (IFNAR1) can influence the risk of developing depression. Interferons are used for the treatment of viral hepatitis, hemato-proliferative disorders, autoimmune disorders and malignancies. However, irrespective of the indication for IFN therapy, IFNs are associated with a 30-70% risk of treatment-emergent depression. In a previous work we have shown that RNA editing biomarkers allowed discrimination between patients who developed a treatment-emergent depression and those who did not.

**IFNAR2:** Interferons act through their receptors; the interferon type I receptor (IFNaR) is composed of two subunits, IFNAR1 and IFNAR2. A polymorphism in the IFNAR2 gene (SNP 11876) showed an association with multiple sclerosis susceptibility (p = 0.035). Interestingly, the same study also identified a polymorphism in the IFNAR1 gene (SNP 18417), which is a member of the same receptor.

**LYN:** LYN encodes a non-receptor tyrosine-protein kinase that transmits signals from cell surface receptors. Lyn plays an important role in the regulation of innate and adaptive immune responses[29]. In the mammalian central nervous system, Lyn is highly expressed in the nucleus accumbens, cerebral cortex and thalamus. Moreover, Lyn participates in the control of N-methyl-D-aspartate receptor (NMDAR, an ionotropic glutamate receptor) receptor pathway. Indeed, behavioral tests showed that spontaneous motor activity is suppressed in Lyn-/- mice, due to enhancement of NMDA receptor signaling. Furthermore, Lyn physically interact with AMPA receptor, a member of the glutamate receptor family[30]. Following stimulation of the receptor, Lyn activates the mitogen-activated protein kinase (MAPK) signalling pathway, which in turn increases expression of brain-derived neurotrophic factor (BDNF). Importantly, activation of AMPA receptors along with release of BDNF and activation of downstream synaptogenic signaling pathways mediate the anti-depressant effects of ketamine79. Lyn kinase-glutamate receptor interactions undergo drastic adaptations in mood disorders such as major depressive disorder.

**AHR:** The aryl hydrocarbon receptor (AHR) is a highly conserved ligand-dependent transcription factor that senses environmental toxins and endogenous ligands. AHR also modulates immune cell differentiation and response[31]. AHR also controls both T(reg) and Th17 cell differentiation in a ligand-specific fashion. Activation AHR induces IDO and thus increases kynurenine levels. The role of Kynureine is well documented in the CNS, where it plays important roles in the pathophysiology of inflammation-induced depression. Pharmacological blockade of AHR rescued systemic inflammation-induced monocyte trafficking, neuroimmune disturbance, and depressive-like behavior in mice. RNA Editing affects the 3'UTR of the human AHR mRNA, thereby creating a microRNA recognition sequence which modulates AHR expression.

**RASSF1:** Ras association domain family member 1 (RASSF1) RASSF1A is a tumor suppressor gene whose inactivation has been implicated in a wide variety of sporadic human cancers and is epigenetically inactivated at high frequency in a variety of solid tumors. RASSF1A associates with MDM2 and enhances the self-ubiquitin ligase activity of MDM2[32]. Interestingly, a microarray analysis recently identified RASSF1A as a significantly downregulated gene in women with history of postpartum depression.

**GAB2:** Gab proteins function downstream of multiple receptors, such as the receptors for EGF, hepatocyte growth factor, and FGFs. GAB2 is an important component of neuronal differentiation induced by retinoic acid, partly by acting downstream of bFGF to mediate survival through PI3 kinase-AKT. GAB2 positively upregulated IL-4 and IL-13 expression in activated T cells, suggesting that GAB2 might be an important regulator of the human Th2 immune response[33].

**CAMK1D:** CAMK1D is a member of the calcium/calmodulin-dependent protein kinase 1 family and is involved in the chemokine signal transduction pathway that regulates granulocyte function[34]. In the CNS, CAMK1D is mostly expressed in the CA1 region of the hippocampus, where it is induced following Long-term potentiation (LTP), a long lasting enhancement of synaptic efficacy in the central nervous system. It could translocate to the nucleus in response to stimuli that trigger intracellular Ca2+ influx, e.g. glutamate, and activate CREB-dependent gene transcription. Noteworthy, SNP rs2724812 in CAMK1D has been associated to remission after selective serotonin reuptake inhibitors treatment by a deep learning approach[35].

**KCNJ15:** Potassium voltage-gated channel subfamily J member 15 (KCNJ15) encodes an inward-rectifier type potassium channel, Kir4.2. In the CNS, the KIR4.2 channel is expressed late in development in both mice and human brain. Inwardly rectifying potassium channels (Kir channels) are important regulators of neuronal signaling and membrane excitability. Gating of Kir channel subunits plays crucial role in polygenic CNS diseases. Moreover, rs928771 in KCNJ15 was found to be associated with alterations in KCNJ15 transcript levels in whole blood; network analysis of hippocampus and blood transcriptome datasets suggested that suggests that the risk variants in the KCNJ15 locus might act through their regulatory effects on immune-related pathways[36].

**IL17RA:** The Interleukin 17 Receptor A (IL17RA) is a type I membrane glycoprotein, which is a similar structure to IFNAR1, that binds with low affinity to interleukin 17A. Interleukin 17A (IL17A) is a proinflammatory cytokine primarily secreted by activated Th17 lymphocytes. Upon biding to its cognate receptor, IL17 signals through ERK, MAPK and NFKB pathways, all of which have been implicated in depressive disorder. Th17 cells are potent regulators of brain function as they target various brain resident cells including neurons, astrocytes, and microglia and participate in neuroinflammation[37]. IL17RA is highly expressed in blood-brain barrier (BBB) endothelial cells in multiple sclerosis lesions, where IL17 increases BBB permeability by disrupting tight junctions, thereby promoting CNS inflammation. Pro-inflammatory IL17 is also secreted by activated astrocytes and microglia and has detrimental effect on neurons. Flow cytometry analysis of PBMCs in unmedicated depressed subjects revealed a significant increase in peripheral Th17 cell number, and elevated serum concentration of IL-17, suggesting a potential role of Th17 cells in MDD patients. Similarly, Th17 cells are increased in the brain of mice after learned helplessness or chronic restraint stress, which are two common depression-like models. Inhibiting the production or function of Th17 cells reduces vulnerability to depression-like behavior in the same mouse models

**PTPRC:** Protein Tyrosine Phosphatase, Receptor Type C (PTPRC), also known as leukocyte-common antigen, or CD45, is an integral membrane protein tyrosine phosphatase, which contains an external segment and is capable of initiating transmembrane signaling in response to external ligands[38]. In the immune system, PTPRC is essential for activation of T and B cells by mediating cell-to-cell contacts and regulating protein-tyrosine kinases involved in signal transduction. PTPRC suppresses negatively regulates cytokine receptor signaling; selective disruption of PTPRC expression leads to enhanced interferon-receptor-mediated activation of JAK and STAT kinases proteins[39]. Remarkably, a point mutation in PTPRC (C-to-G nucleotide transition in position 77 of exon 4) has been associated with the development of multiple sclerosis, the most common demyelinating disease of the CNS. In the brain, anxiety-like behavior induced by repeated social defeat, a mouse model of depression, corresponded with an increase in brain macrophages overexpressing PTPRC. In the same mouse model of depression, classical benzodiazepines were shown to reverse symptoms and to block stress-induced accumulation of macrophages expressing high levels of PTPRC in the CNS. Furthermore, in patients diagnosed with major depressive disorder, PTPRC was higher in patients than in healthy subjects. PTPRC ratio decreased with antidepressant treatment such as venlafaxine or fluoxetine and was positively correlated with the severity of depression[40], suggesting that antidepressant treatment contributes to immune regulation in patients with major depressive disorder.

**PDE8A:** Glutamate or serotonin receptors are G-protein-coupled receptors (GPCRs) involved in a variety of psychiatric disorders, for which the spatial and temporal regulation of second messenger concentration is crucial for subsequent signaling. Phosphodiesterases (PDE) are key modulators of signal transduction and are involved in inflammatory cell activation, behavior, memory and cognition. PDE8A is a cAMP-specific phosphodiesterase expressed in the brain and blood. Altered RNA editing in PDE8A transcripts has been identified in in T cells of patients suffering from systemic lupus erythematosus, a chronic autoimmune disorder, as in normal T cells with IFN-$\alpha$. There is a two-fold decrease in the expression of phosphodiesterase 8A (PDE8A) in the temporal cortex of major depressive disorder (MDD) patients[41]. We have recently shown brain region-specific alterations of RNA editing in PDE8A mRNA in suicide decedents, which provided the first immune response-related brain marker for suicide[5]. PDE8A RNA editing is also increased in SHSY-5Y neuroblastoma cells treated with interferon alpha, as in HCV patients undergoing antiviral combination therapy with IFN-$\alpha$ and ribavirin. Importantly, measurement of RNA editing in PDE8A allowed discrimination between the group of patients who developed a treatment-emergent depression and those who did not, suggesting that A-I RNA editing biomarkers could be useful to monitor treatment-emergent depression[22].

In order to prioritize a subset of variants clinically useful for a diagnostic assay, each of the target genes described above was tested individually in two separate 'prioritization' cohorts (n=76, Figure 17 and n=86, Figure 18) by measuring editing rate in controls and depressed patients by NGS. In the first cohort each target was sequenced individually, while in the second cohort editing rates of biomarkers were measured by multiplexing 4 BMKs. A multivariate analysis statistical method, evaluating for each combination of RNA edited sites and/or isoforms, was performed to find the best combination of biomarkers. To potentiate diagnostic performance, while minimizing biomarker number, the best combination was calculated with different numbers of biomarkers.

The list of primers used for the detection of RNA editing sites is indicated in Table 1

Table 1: Primers used for the detection of RNA editing sites

| PRIMER NAME | Gene Name | PCR product Size (bp) | Primer Sequence (5'->3') | Primer length (bp) | SEQ ID NO. |
|---|---|---|---|---|---|
| IFNAR1 FORWARD_1 | IFNAR1 | 259 | ATACGGGCAAGCTCTTAACTATT | 23 | 1 |
| IFNAR1 REVERSE_1 | | | AACCTCCACCTCCTGTTTTC | 20 | 2 |
| IFNAR1_MiSeqF | IFNAR1 | 472 | AAAATACGGGCAAGCTCTTAACT | 23 | 3 |
| IFNAR1_MiSeqR | | | CTGCACTTAGGCTCACAGGA | 20 | 4 |
| | | | | | |
| PDE8A Forward | PDE8A | 225 | ATGCAAGTTGTGGACATGGAG | 21 | 5 |
| PDE8A Reverse | | | TTCTGAAAACAATGGGCACCA | 21 | 6 |
| | | | | | |
| CAMK1DPCR0_F | CAMK1D | 577 | TGTGCAAAGGGCTCCATAC | 19 | 7 |
| CAMK1DPCR0_R | | | CCTTGCAAGTGTCCAGTAAAC | 21 | 8 |
| CAMK1D MPx_F1 | | 195 | CTAGGGTGAGGTGGTGCAAT | 20 | 9 |
| CAMK1D MPx R1 | | | CAAGGCCAGGAGTTCAAGAC | 20 | 10 |
| | | | | | |
| LYN_F_PCR0 | LYN | 358 | TTTAAAAGCTTCTCCAGTCAGC | 22 | 11 |
| LYN_R_PCR0 | | | CAAGACTGAGCATACATTATATTCACC | 27 | 12 |
| LYN MPx_F1 | | 193 | TTTAAAAGCTTCTCCAGTCAGC | 22 | 13 |
| LYN MPx_R1 | | | TCCTGAGTAGCTGGGACCAT | 20 | 14 |
| | | | | | |
| PRKCB_MPx_F1 | PRKCB | 176 | CAGCACATTCTGTTGCCTTC | 20 | 15 |
| PRKCB MPx R2 | | | GTGACTTTGCCCCTCATTTG | 20 | 16 |
| | | | | | |
| KCNJ15_MPx_F1 | KCNJ15 | 160 | GGCCACTCTGTACTATGCAAATC | 23 | 17 |
| KCNJ15 MPx R2 | | | TGAATTTCAGATGGACAGCAA | 21 | 18 |
| | | | | | |
| MDM2_F5 MPx-Rev | MDM2 | 302 | AAGCCCATCCTCGAACTACAG | 21 | 19 |
| MDM2_R5 MPx-Rev | | | TTGCTCTGTCACCTGGACTG | 20 | 20 |
| | | | | | |
| GAB2 MPx_F1 | GAB2 | 154 | GAACCAGGCTAGGTGCAATG | 20 | 21 |

| PRIMER NAME | Gene Name | PCR product Size (bp) | Primer Sequence (5'->3') | Primer length (bp) | SEQ ID NO. |
|---|---|---|---|---|---|
| GAB2 MPx_R2 | | | CCATGCCCTGGTAATTTTTG | 20 | 22 |
| | | | | | |
| AHR-F1 | AHR | 409 | TCTGATGGAATCCCAAACTGAGA | 23 | 23 |
| AHR_F2 | | | TCAGAGATATTTTGTCCGAAGCCA | 24 | 24 |
| | | | | | |
| IFNAR2 MPx_F1_rev | IFNAR2 | 344 | CCCACAGTTTCAGAGGTGGT | 18 | 25 |
| IFNAR2 MPx_R1_rev | | | GGCTCACCCCTGTAATCC | 20 | 26 |
| | | | | | |
| IL17RA_Forward1 | IL17RA | 172 | GGGATGTAAACGCTGAATGG | 20 | 27 |
| IL17RA_Reverse1 | | | ATGTGCCACCATGACTGACT | 20 | 28 |
| | | | | | |
| RASSF1_F1 | | 480 | CCCTTCCGGCAAGAATCCAG | 20 | 29 |
| RASSF1_R1 | | | ACCCAAGCTGCATAAAAGGC | 20 | 30 |
| | | | | | |
| PIAS1_F1 | PIAS1 | 407 | GCAACCTTAGATCTCAATG GCTAA | 24 | 31 |
| PIAS1_R2 | | | AGCAGAGTCACAGTCTACAGC | 21 | 32 |
| | | | | | |
| PTPRC-MiSeqF | PTPTC | 505 | CCGGGGCTCATTTATAGGAT | 20 | 33 |
| PTPRC-MiSeqR | | | TGCTAGATACCGCCACATTG | 20 | 34 |
| PTPRC F rd2-1 | PTPRC | 215 | TAAGGACGGAGTTCGAGACC | 20 | 35 |
| PTPRC R rd2-1 | | | TGAGAGAGTTTTGCTCTGTGG | 21 | 36 |

EP 4 055 190 B1

17

As it was important to validate the results obtained in the RNA-Seq study, we first ran a differential analysis of each target on the prioritization cohorts. As shown in the tables below (Tables 2A-2B and3), all the sites and isoforms identified in the RNA-Seq study were found significant in a subsequent analysis on a larger population.

**Tables 2A and 2B:** Differential analysis of editing sites (2A) and isoforms (2B) identified in the RNA-Seq study in the patients of the first prioritization cohort analyzed in single-plex.

Shown are examples of RNA-Seq data of different targets with target name, differentially edited site or isoform, p-value and fold change of the edited site or isoform, AUC ROC, the mean value of editing at the considered site and population number.

**Table 2A**

| ID | Target | Chr:Pos (hg38) | Sites | p-Value | foldChange | AUC ROC | %Editing (Mean) | Population |
|---|---|---|---|---|---|---|---|---|
| 1 | AHR | 7:17345070 | X118 | 0.0318 | 1.80 | 0.635 | 0.52 | N= 76 [ 41 (0) ; 35 (1)] |
| 3 | IFNAR1 | 21: 33355789 | X111 | 0.0239 | 1.07 | 0.651 | 19.16 | N= 76 [ 41 (0); 35 (1)] |
| 4 | IFNAR1 | 21: 33355793 | X115 | 0.0098 | 1.11 | 0.678 | 10.89 | N= 76 [ 41 (0); 35 (1)] |
| 5 | IFNAR1 | 21: 33355807 | X129 | 0.0337 | 1.06 | 0.647 | 20.53 | N= 76 [ 41 (0); 35 (1)] |
| 6 | IFNAR1 | 21: 33355838 | X160 | 0.0030 | 1.13 | 0.682 | 7.60 | N= 76 [ 41 (0); 35 (1)] |
| 7 | IFNAR1 | 21: 33355840 | X162 | 0.0069 | 1.10 | 0.681 | 10.05 | N= 76 [ 41 (0); 35 (1)] |
| 8 | IFNAR1 | 21: 33355854 | X 176 | 0.0004 | 1.18 | 0.745 | 0.54 | N= 76 [ 41 (0); 35 (1)] |
| 9 | IFNAR1 | 21: 33355860 | X182 | 0.0142 | 1.13 | 0.652 | 4.30 | N= 76 [ 41 (0); 35 (1)] |
| 10 | IFNAR1 | 21: 33355865 | X187 | 0.0019 | 1.15 | 0.698 | 1.96 | N= 76 [ 41 (0); 35 (1)] |
| 11 | IFNAR1 | 21: 33355869 | X191 | 0.0059 | 1.14 | 0.688 | 7.47 | N= 76 [ 41 (0); 35 (1)] |
| 12 | IFNAR1 | 21: 33355905 | X227 | 0.0066 | 1.10 | 0.681 | 5.45 | N= 76 [ 41 (0); 35 (1)] |
| 13 | IFNAR1 | 21: 33355909 | X231 | 0.0121 | 1.10 | 0.661 | 10.39 | N= 76 [ 41 (0); 35 (1)] |
| 14 | IFNAR1 | 21: 33355762 | X84 | 0.0056 | 1.12 | 0.678 | 10.56 | N= 76 [ 41 (0); 35 (1)] |
| 15 | IFNAR1 | 21: 33355763 | X85 | 0.0067 | 1.12 | 0.681 | 6.25 | N= 76 [ 41 (0); 35 (1)] |
| 16 | IL17RA | 22: 17110647 | X151 | 0.0278 | 1.05 | 0.637 | 8.40 | N= 76 [ 41 (0); 35 (1)] |
| 17 | IL17RA | 22: 17110680 | X184 | 0.0049 | 1.13 | 0.674 | 0.83 | N= 76 [ 41 (0); 35 (1)] |
| 18 | IL17RA | 22: 17110551 | X55 | 0.0164 | 1.14 | 0.655 | 1.41 | N= 76 [ 41 (0); 35 (1)] |
| 20 | IL17RA | 22: 17110574 | X78 | 0.0049 | 1.09 | 0.675 | 1.95 | N= 76 [ 41 (0); 35 (1)] |

(continued)

| ID | Target | Chr:Pos (hg38) | Sites | p-Value | foldChange | AUC ROC | %Editing (Mean) | Population |
|----|--------|----------------|-------|---------|------------|---------|-----------------|-----------|
| 21 | IL17RA | 22: 17110591 | X95 | 0.0411 | 1.05 | 0.629 | 15.97 | N= 60 [ 41 (0) ; 19 (1)] |
| 22 | LYN | 8:56012521 | X116 | 0.0266 | 1.12 | 0.652 | 0.97 | N= 76 [ 41 (0); 35 (1)] |
| 23 | LYN | 8:56012553 | X148 | 0.0238 | 1.06 | 0.676 | 18.79 | N= 60 [ 41 (0) ; 19 (1)] |
| 24 | LYN | 8:56012558 | X153 | 0.0074 | 1.10 | 0.661 | 3.81 | N= 76 [ 41 (0); 35 (1)] |
| 25 | LYN | 8:56012689 | X284 | 0.0140 | 1.10 | 0.633 | 4.34 | N= 76 [ 41 (0); 35 (1)] |
| 26 | LYN | 8:56012705 | X300 | 0.0161 | 1.10 | 0.652 | 3.03 | N= 76 [ 41 (0); 35 (1)] |
| 27 | LYN | 8:56012490 | X85 | 0.0049 | 1.13 | 0.679 | 0.75 | N= 76 [ 41 (0); 35 (1)] |
| 28 | MDM2 | 12: 68821545 | X166 | 0.0142 | 0.91 | 0.661 | 2.91 | N= 76 [ 41 (0); 35 (1)] |
| 29 | MDM2 | 12: 68821629 | X250 | 0.0215 | 0.89 | 0.649 | 4.42 | N= 76 [ 41 (0); 35 (1)] |
| 30 | PIAS1 | 15: 68138001 | X201 | 0.0446 | 1.09 | 0.664 | 0.64 | N= 60 [ 41 (0) ; 19 (1)] |
| 31 | PTPRC | 1: 198680346 | X240 | 0.0216 | 1.06 | 0.689 | 25.83 | N= 60 [ 41 (0) ; 19 (1)] |
| 32 | PTPRC | 1: 198680405 | X299 | 0.0263 | 1.08 | 0.675 | 4.30 | N= 60 [ 41 (0) ; 19 (1)] |
| 33 | RASSF1 | 3:50333653 | X192 | 0.0204 | 1.09 | 0.654 | 1.32 | N= 60 [ 41 (0) ; 19 (1)] |
| 35 | PDE8A | 15: 85212876 | X116246 | 0.0055 | 0.92 | 0.668 | 19.81 | N= 76 [ 41 (0); 35 (1)] |

**Table 2B**

| Target | Isoform | p-Value | Fold-change | AUC ROC | %Editing (Mean) | Population |
|--------|---------|---------|-------------|---------|-----------------|-----------|
| IFNAR1 | A | 0.0002 | 1.13 | 0.735 | 0.53 | N= 76 [ 41 (0) ; 35 (1)] |
| IFNAR1 | ABCDEFGH | 0.0042 | 1.19 | 0.686 | 0.99 | N= 76 [ 41 (0) ; 35 (1)] |
| IFNAR1 | GH | 0.0002 | 1.16 | 0.742 | 0.73 | N= 76 [ 41 (0) ; 35 (1)] |
| LYN | DO | 0.0275 | 0.94 | 0.660 | 1.45 | N= 76 [ 41 (0) ; 35 (1)] |
| MDM2 | GVA | 0.0023 | 1.09 | 0.710 | 0.94 | N= 76 [ 41 (0) ; 35 (1)] |
| MDM2 | W | 0.0154 | 0.93 | 0.669 | 0.45 | N= 76 [ 41 (0) ; 35 (1)] |
| PDE8A | B | 0.0059 | 0.92 | 0.660 | 19.00 | N= 76 [ 41 (0) ; 35 (1)] |
| PIAS1 | F | 0.0359 | 1.04 | 0.641 | 1.10 | N= 76 [ 41 (0) ; 35 (1)] |
| PTPRC | HI | 0.0331 | 1.07 | 0.623 | 1.61 | N= 76 [ 41 (0) ; 35 (1)] |
| RASSF1 | ABE | 0.0224 | 1.04 | 0.637 | 1.87 | N= 76 [41 (0) ; 35 (1)] |

(continued)

| Target | Isoform | p-Value | Fold-change | AUC ROC | %Editing (Mean) | Population |
|--------|---------|---------|-------------|---------|-----------------|-----------|
| RASSF1 | ABEH | 0.0290 | 1.07 | 0.695 | 0.69 | N= 76 [41 (0) ; 35 (1)] |
| RASSF1 | HJ | 0.0260 | 0.93 | 0.624 | 0.83 | N= 76 [41 (0) ; 35 (1)] |

**Table 3:** Differential analysis of editing sites and isoforms identified in the RNA-Seq study in the patients of the second prioritization cohort analyzed with multiplex sequencing.

Shown are examples of RNA-Seq data of different targets with target name, differentially edited site, p-value and fold change of the edited site, AUC ROC, the mean value of editing at the considered site and population number.

| Target | Chr:Pos (hg38) | Sites | p-Value | Foldchange | AUC ROC | % Editing (Mean) | Population |
|--------|----------------|-------|---------|------------|---------|------------------|-----------|
| CAMK1D | 10:12378423 | X131 | 0.0136 | 0.90 | 0.659 | 1.33 | N= 86 [ 28 (0) ;58 (1)] |
| CAMK1D | 10:12378341 | X49 | 0.0303 | 0.92 | 0.639 | 5.59 | N= 86 [ 28 (0) ;58 (1)] |
| CAMK1D | 10:12378415 | X123 | 0.0387 | 0.93 | 0.698 | 25.23 | N= 30 [ 15 (0) ;15 (1)] |
| GAB2 | 11:78330952 | X88 | 0.0004 | 0.88 | 0.705 | 1.5 | N= 86 [ 28 (0) ;58 (1)] |
| GAB2 | 11:78330914 | X126 | 0.0260 | 0.96 | 0.610 | 17.18 | N= 86 [ 28 (0) ;58 (1)] |
| IFNAR1 | 21:33355779 | X101 | 0.0033 | 0.87 | 0.717 | 0.87 | N= 86 [ 28 (0) ;58 (1)] |
| IFNAR1 | 21:33355820 | X142 | 0.0159 | 0.89 | 0.663 | 1.3 | N= 86 [ 28 (0) ;58 (1)] |
| IFNAR1 | 21:33355754 | X76 | 0.0330 | 0.91 | 0.643 | 4.4 | N= 86 [ 28 (0) ;58 (1)] |
| KCNJ15 | 21:38287590 | X134 | 0.0099 | 0.89 | 0.673 | 3.6 | N= 86 [ 28 (0) ;58 (1)] |
| KCNJ15 | 21:38287586 | X130 | 0.0308 | 0.93 | 0.650 | 25.2 | N= 86 [ 28 (0) ;58 (1)] |
| KCNJ15 | 21:38287585 | X129 | 0.0237 | 0.91 | 0.647 | 3.2 | N= 86 [ 28 (0) ;58 (1)] |
| KCNJ15 | 21:38287589 | X133 | 0.0197 | 0.90 | 0.644 | 14.53 | N= 86 [ 28 (0) ;58 (1)] |
| KCNJ15 | 21:38287587 | X131 | 0.0488 | 0.91 | 0.634 | 0.9 | N= 86 [ 28 (0) ;58 (1)] |

| Target | Chr:Pos (hg38) | Sites | p-Value | Foldchange | AUC ROC | % Editing (Mean) | Population |
|--------|----------------|-------|---------|------------|---------|------------------|-----------|
| LYN | 8:56012495 | X90 | 0.0040 | 0.89 | 0.696 | 1.2 | N= 86 [ 28 (0) ;58 (1)] |
| LYN | 8:56012560 | X155 | 0.0443 | 1.09 | 0.635 | 0.6 | N= 86 [ 28 (0) ;58 (1)] |
| LYN | 8:56012553 | X148 | 0.0435 | 0.99 | 0.532 | 15.7 | N= 57 [28 (0) ;29 (1)] |
| MDM2 | 12:68821586 | X207 | 0.0004 | 0.89 | 0.746 | 12.58 | N= 86 [ 28 (0) ;58 (1)] |
| MDM2 | 12:68821522 | X143 | 0.0018 | 0.79 | 0.699 | 0.76 | N= 86 [ 28 (0) ;58 (1)] |
| MDM2 | 12:68821570 | X191 | 0.0296 | 0.92 | 0.652 | 1.09 | N= 86 [ 28 (0) ;58 (1)] |
| PDE8A | 15:85212876 | X116248 | 0.0032 | 0.90 | 0.680 | 20.3 | N= 86 [ 28 (0) ;58 (1)] |
| PRKCB | 16:23920909 | X103 | 0.0236 | 0.94 | 0.649 | 5.7 | N= 86 [ 28 (0) ;58 (1)] |
| PRKCB | 16:23920932 | X126 | 0.0235 | 0.94 | 0.649 | 1.5 | N= 86 [ 28 (0) ;58 (1)] |
| PRKCB | 16:23920938 | X132 | 0.0360 | 0.96 | 0.637 | 17.3 | N= 86 [ 28 (0) ;58 (1)] |
| PTPRC | 1:198680365 | X259 | 0.0466 | 0.94 | 0.625 | 0.8 | N= 86 [ 28 (0) ;58 (1)] |
| PTPRC | 1:198680332 | X226 | 0.0295 | 0.95 | 0.664 | 13.0 | N= 57 [28 (0) ;29 (1)] |
| MDM2 | 12:68848833 | X76 | 0.0035 | 0.83 | 0.704 | 0.5 | N= 86 [ 28 (0) ;58 (1)] |
| MDM2 | 12:68848843 | X86 | 0.0410 | 0.89 | 0.664 | 1.0 | N= 86 [ 28 (0) ;58 (1)] |

(continued)

| MDM2 | 12:68848844 | X87 | 0.0388 | 0.95 | 0.639 | 4.8 | N= 86 [ 28 (0) ;58 (1)] |
| --- | --- | --- | --- | --- | --- | --- | --- |

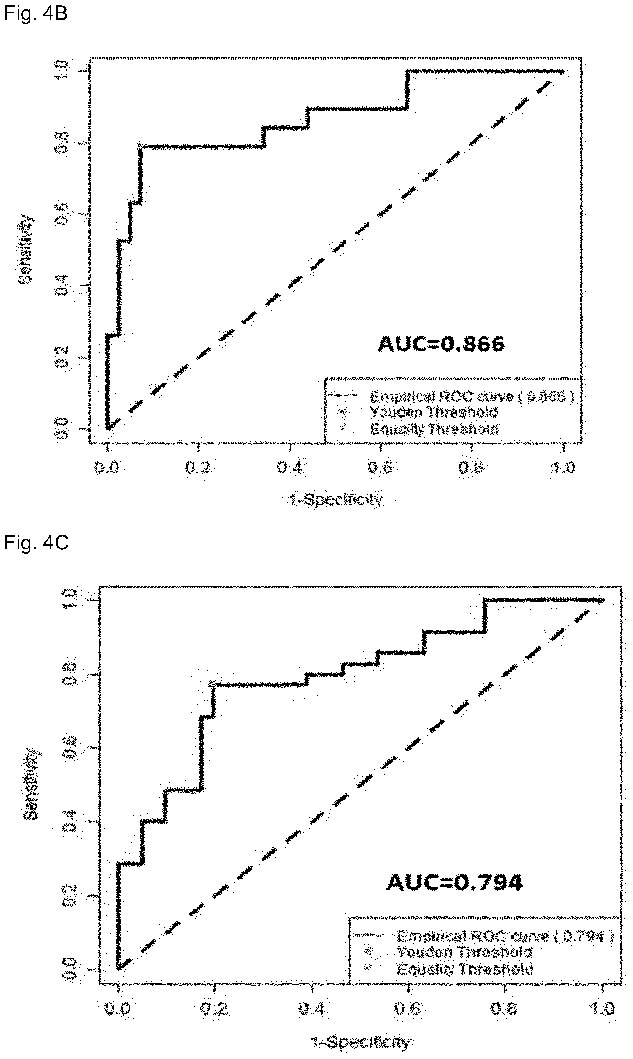

Combinations of biomarkers were then analyzed to identify the best diagnostic performance with different numbers of biomarkers. In the first cohort, when only 4 sites from 3 different targets were used, we obtained an AUC of 0.811 (Sp 78.1%; Se 81.3%, Figure 4A). Another combination of 4 sites, using 2 BMKs, gave even better performances, with an AUC of 0.866 (Sp 92.7%; Se 79.0%, Figure 4B). The equation used for this combination is the following:

Z= + (0,361161202728045 )* IFNAR1_X227_H + ( -0,0763586204473731 )* IFNAR1_X162_E + ( 1,35409123139041 )* LYN_X284_P + ( -2,2905526156995 )* LYN_X207_M.

Though measurement of RNA editing variants gave good performances with all the combinations tested, the performance was not necessarily reflected by the number of biomarkers: when the number of biomarkers was increased to 6, with 4 different targets, the AUC was 0.794 (Sp 80.5%; Se 77.1%, Figure 4C). However, when 5 BMKs were combined and analyzed all the differentially edited sites in those targets, we obtained an outstanding performance, with an AUC of 0.977, with excellent specificity and sensitivity (92.7% and 94.7%, respectively, Figure 4D). The equation used for this combination is the following:

Z= + (1,242350672584)* IFNAR1 X227_H + (1,36013909308819)* IFNAR1_X115_G + ( -0,739944712498771 )* IFNAR1_X84_F + ( 1,42700898962888 )* IFNAR1_X162_E + ( -1,66838875199491 )* IFNAR1_X231_D + ( -0,887010245248778 )* IFNAR1_X129_B + ( 0,0876014897108424 )* IFNAR1_X111_A + ( 0,985129912485808 )* LYN_X153_G + ( 4,33197064304577 )* LYN_X284_P + ( -0,200203331256123 )* IFNAR1_X152_C + ( -15,708397165937 )* IL17RA X78_D + ( 2,50160465829557 )* LYN_X116_T + ( 5,53594778946524 )* IL17RA_X77_C + ( -3,04072407838489 )* LYN_X47_Q + ( 1,82611854035731 )* IL17RA_X184_H + ( 1,14130391422593 )* LYN X85_R + ( -1,36538923404951 )* LYN_X63_C + ( -1,52552008654041 )* LYN_X172_J + ( 3,47820883385469 )* LYN_X203_L + ( -1,80085106045942 )* IL17RA X55_B + ( -1,25213023530841 )* LYN_X163_Z + ( -1,01733883175651 )* PTPRC X240_I + ( 122,471673425459 )* IL17RA_X151_G + ( 1,09079061375429 )* LYN_X64_D + ( 2,32804478266105 )* LYN_X255_N + ( -3,86751458009041 )* LYN_X167_I + ( -12,6254508356666 )* LYN_X148_A + ( 3,00274639389995 )* PTPRC_X299_O - ( 1,08899988893022 )* PDE8A_SiteB.

The list of all combination tested in this cohort is presented in Table 4. Noteworthy, in the first cohort some samples were not analyzed and the total population in the combination may be lower than the total population in the cohort. All combinations were statistically significant between control and cases with a p value $<10^{-4}$.

**Table 4:** Diagnostic performances obtained with patients of the first prioritization cohort analyzed in single-plex.

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | | | | | | | | | | | | |
| 1 | RASSF1_X263 + AHR_X156 | C2 | 2 | 0.782 | [0,628 ; 0,936] | -1.04 | 80.7 | 82.9 | 75.0 | 63.2 | 89.5 | N= 57 [41 (1); 16 (2)] |
| 2 | AHR_X118 + AHR_X156 | C2 | 4 | 0.745 | [0,599 ; 0,892] | 0.12 | 80.7 | 92.7 | 50.0 | 72.7 | 82.6 | N= 57 [41 (1); 16 (2)] |
| 3 | AHR_X156 + PDE8A_X116246 | C2 | 6 | 0.773 | [0,633 ; 0,913] | -6.50 | 77.2 | 78.1 | 75.0 | 57.1 | 88.9 | N= 57 [41 (1); 16 (2)] |
| 4 | IFNAR1_X227 + PTPRC_X299 | C2 | 28 | 0.815 | [0,698 ; 0,932] | 3.53 | 76.7 | 68.3 | 94.7 | 58.1 | 96.6 | N= 60 [41 (1); 19 (2)] |
| 5 | IFNAR1_X84 + PTPRC_X299 | C2 | 83 | 0.810 | [0,694 ; 0,926] | 3.95 | 76.7 | 68.3 | 94.7 | 58.1 | 96.6 | N= 60 [41 (1); 19 (2)] |
| 6 | RASSF1_X263 + AHR_X118 | C2 | 1 | 0.767 | [0,626 ; 0,907] | -1.42 | 75.4 | 73.2 | 81.3 | 54.2 | 90.9 | N= 57 [41 (1); 16 (2)] |
| 7 | IFNAR1_X227 + PDE8A_X116246 | C2 | 36 | 0.764 | [0,655 ; 0,873] | -3.28 | 75.0 | 73.2 | 77.1 | 71.1 | 79.0 | N= 76 [41 (1); 35 (2)] |
| 8 | IFNAR1_X84 + PDE8A_X116246 | C2 | 55 | 0.748 | [0,638 ; 0,859] | -2.70 | 73.7 | 73.2 | 74.3 | 70.3 | 76.9 | N= 76 [41 (1); 35 (2)] |
| 9 | IFNAR1_X227 + PTPRC_X240 | C2 | 22 | 0.813 | [0,692 ; 0,933] | 2.05 | 73.3 | 65.9 | 89.5 | 54.8 | 93.1 | N= 60 [41 (1); 19 (2)] |
| 10 | IL17RA_X77 + IFNAR1_X162 | C2 | 1 | 0.703 | [0,584 ; 0,823] | -0.36 | 72.4 | 73.2 | 71.4 | 69.4 | 75.0 | N= 76 [41 (1); 35 (2)] |
| 11 | LYN_X153 + PTPRC_X299 | C2 | 203 | 0.793 | [0,679 ; 0,908] | 4.08 | 71.7 | 61.0 | 94.7 | 52.9 | 96.2 | N= 60 [41 (1); 19 (2)] |
| 12 | PTPRC_X299 + PDE8A_X116246 | C2 | 435 | 0.791 | [0,679 ; 0,903] | -1.73 | 71.7 | 61.0 | 94.7 | 52.9 | 96.2 | N= 60 [41 (1); 19 (2)] |
| 13 | LYN_X85 + PDE8A_X116246 | C2 | 46 | 0.749 | [0,639 ; 0,859] | -5.33 | 71.1 | 58.5 | 85.7 | 63.8 | 82.8 | N= 76 [41 (1); 35 (2)] |
| 14 | IFNAR1_X162 + PDE8A_X116246 | C2 | 25 | 0.743 | [0,632 ; 0,854] | -2.21 | 71.1 | 73.2 | 68.6 | 68.6 | 73.2 | N= 76 [41 (1); 35 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 15 | IFNAR1_X231 + PDE8A_X116246 | C2 | 85 | 0.734 | [0,622 ; 0,847] | -2.80 | 71.1 | 61.0 | 82.9 | 64.4 | 80.7 | N= 76 [41 (1); 35 (2)] |
| 16 | IL17RA_X77 + IFNAR1_X227 | C2 | 2 | 0.713 | [0,595 ; 0,831] | -0.74 | 71.1 | 70.7 | 71.4 | 67.6 | 74.4 | N= 76 [41 (1); 35 (2)] |
| 17 | IFNAR1_X84 + IFNAR1_X231 | C2 | 51 | 0.702 | [0,582 ; 0,821] | 1.68 | 71.1 | 75.6 | 65.7 | 69.7 | 72.1 | N= 76 [41 (1); 35 (2)] |
| 18 | AHR_X118 + PDE8A_X116246 | C2 | 5 | 0.755 | [0,626 ; 0,883] | -7.44 | 68.4 | 61.0 | 87.5 | 46.7 | 92.6 | N= 57 [41 (1); 16 (2)] |
| 19 | RASSF1_X263 + PDE8A_X116246 | C2 | 3 | 0.753 | [0,621 ; 0,885] | -6.76 | 64.9 | 53.7 | 93.8 | 44.1 | 95.7 | N= 57 [41 (1); 16 (2)] |
| 20 | RASSF1_X263 + AHR_X156 + PDE8A X116246 | C3 | 3 | 0.793 | [0,65 ; 0,936] | -6.42 | 84.2 | 87.8 | 75.0 | 70.6 | 90.0 | N= 57 [41 (1); 16 (2)] |
| 21 | RASSF1_X263 + AHR_X118 + AHR_X156 | C3 | 1 | 0.788 | [0,638 ; 0,938] | -1.10 | 84.2 | 90.2 | 68.8 | 73.3 | 88.1 | N= 57 [41 (1); 16 (2)] |
| 22 | IFNAR1_X227 + LYN_X284 + LYN_X63 | C3 | 184 | 0.861 | [0,754 ; 0,969] | 2.16 | 83.3 | 87.8 | 73.7 | 73.7 | 87.8 | N= 60 [41 (1); 19 (2)] |
| 23 | IFNAR1_X227 + LYN_X284 + LYN_X172 | C3 | 185 | 0.874 | [0,787 ; 0,962] | 3.81 | 80.0 | 80.5 | 79.0 | 65.2 | 89.2 | N= 60 [41 (1); 19 (2)] |
| 24 | IFNAR1_X227 + PTPRC_X299 + PDE8A_ X116246 | C3 | 406 | 0.859 | [0,763 ; 0,955] | -0.10 | 80.0 | 75.6 | 89.5 | 63.0 | 93.9 | N= 60 [41 (1); 19 (2)] |
| 25 | IFNAR1_X227 + LYN_X284 + LYN_X64 | C3 | 191 | 0.852 | [0,748 ; 0,957] | 2.13 | 80.0 | 80.5 | 79.0 | 65.2 | 89.2 | N= 60 [41 (1); 19 (2)] |
| 26 | IFNAR1_X84 + LYN_X284 + LYN_X172 | C3 | 914 | 0.850 | [0,754 ; 0,945] | 4.30 | 78.3 | 78.1 | 79.0 | 62.5 | 88.9 | N= 60 [41 (1); 19 (2)] |
| 27 | IFNAR1_X227 + IFNAR1_X111 + PDE8A_ X116246 | C3 | 199 | 0.769 | [0,66 ; 0,878] | -3.55 | 77.6 | 73.2 | 82.9 | 72.5 | 83.3 | N= 76 [41 (1); 35 (2)] |
| 28 | AHR_X118 + AHR_X156 + PDE8A_X116246 | C3 | 4 | 0.777 | [0,649 ; 0,905] | -6.99 | 77.2 | 82.9 | 62.5 | 58.8 | 85.0 | N= 57 [41 (1); 16 (2)] |

EP 4 055 190 B1

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 29 | IFNAR1_X84 + IFNAR1_X231 + PDE8A_X116246 | C3 | 274 | 0.771 | [0,665 ; 0,878] | -3.58 | 76.3 | 78.1 | 74.3 | 74.3 | 78.1 | N= 76 [41 (1); 35 (2)] |
| 30 | RASSF1_X263 + AHR_X118 + PDE8A_X116246 | C3 | 2 | 0.793 | [0,664 ; 0,921] | -7.18 | 75.4 | 73.2 | 81.3 | 54.2 | 90.9 | N= 57 [41 (1); 16 (2)] |
| 31 | IFNAR1_X227 + IL17RA_X55 + PDE8A_X116246 | C3 | 196 | 0.771 | [0,663 ; 0,88] | -3.21 | 75.0 | 75.6 | 74.3 | 72.2 | 77.5 | N= 76 [41 (1); 35 (2)] |
| 32 | IFNAR1_X162 + IFNAR1_X227 + PDE8A_X116246 | C3 | 89 | 0.766 | [0,657 ; 0,875] | -3.40 | 75.0 | 73.2 | 77.1 | 71.1 | 79.0 | N= 76 [41 (1); 35 (2)] |
| 33 | IFNAR1_X84 + PTPRC_X299 + PDE8A_X116246 | C3 | 1135 | 0.851 | [0,757 ; 0,945] | -0.03 | 73.3 | 63.4 | 94.7 | 54.6 | 96.3 | N= 60 [41 (1); 19 (2)] |
| 34 | IFNAR1_X227 + IFNAR1_X162 + LYN_X284 + LYN X207 | C4 | 831 | 0.866 | [0,761 ; 0,972] | -0.44 | 88.3 | 92.7 | 79.0 | 83.3 | 90.5 | N= 60 [41 (1); 19 (2)] |
| 35 | IFNAR1_X227 + IFNAR1_X129 + LYN_X284 + LYN X207 | C4 | 1407 | 0.864 | [0,752 ; 0,976] | -3.03 | 88.3 | 92.7 | 79.0 | 83.3 | 90.5 | N= 60 [41 (1); 19 (2)] |
| 36 | IFNAR1_X227 + IFNAR1_X115 + LYN_X284 + LYN X207 | C4 | 155 | 0.863 | [0,756 ; 0,97] | -0.20 | 88.3 | 92.7 | 79.0 | 83.3 | 90.5 | N= 60 [41 (1); 19 (2)] |
| 37 | IFNAR1_X227 + LYN_X284 + LYN_X207 + PTPRC_X240 | C4 | 2239 | 0.858 | [0,748 ; 0,967] | -1.45 | 88.3 | 92.7 | 79.0 | 83.3 | 90.5 | N= 60 [41 (1); 19 (2)] |
| 38 | IFNAR1_X227 + LYN_X255 + PTPRC_X299 + PDE8A_X116246 | C4 | 3650 | 0.860 | [0,763 ; 0,957] | -0.25 | 83.3 | 80.5 | 89.5 | 68.0 | 94.3 | N= 60 [41 (1); 19 (2)] |
| 39 | IFNAR1_X227 + IFNAR1_X231 + PTPRC_X299 + PDE8A_X116246 | C4 | 1354 | 0.861 | [0,762 ; 0,961] | -1.14 | 81.7 | 75.6 | 94.7 | 64.3 | 96.9 | N= 60 [41 (1); 19 (2)] |
| 40 | IFNAR1_X227 + LYN_X255 + LYN_X148 + PTPRC_X299 | C4 | 3648 | 0.845 | [0,731 ; 0,958] | 40.28 | 81.7 | 75.6 | 94.7 | 64.3 | 96.9 | N= 60 [41 (1); 19 (2)] |
| 41 | RASSF1_X263 + AHR_X118 + AHR_X156 + PDE8A X116246 | C4 | 1 | 0.811 | [0,677 ; 0,945] | -7.18 | 79.0 | 78.1 | 81.3 | 59.1 | 91.4 | N= 57 [41 (1); 16 (2)] |
| 42 | IFNAR1_X84 + IFNAR1_X231 + IFNAR1_X111 + PDE8A_X116246 | C4 | 871 | 0.775 | [0,669 ; 0,881] | -3.43 | 77.6 | 80.5 | 74.3 | 76.5 | 78.6 | N= 76 [41 (1); 35 (2)] |

EP 4 055 190 B1

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 43 | IFNAR1_X227 + IL17RA_X55 + IFNAR1_X111 + PDE8A_X116246 | C4 | 670 | 0.774 | [0,666 ; 0,882] | -3.60 | 76.3 | 68.3 | 85.7 | 69.8 | 84.9 | N= 76 [ 41 (1); 35 (2)] |
| 44 | IFNAR1_X227 + IFNAR1_X84 + IFNAR1_X111 + PDE8A_X116246 | C4 | 587 | 0.772 | [0,666 ; 0,878] | -3.00 | 76.3 | 73.2 | 80.0 | 71.8 | 81.1 | N= 76 [ 41 (1); 35 (2)] |
| 45 | IFNAR1_X84 + IL17RA_X184 + IFNAR1_X231 + PDE8A_X116246 | C4 | 849 | 0.772 | [0,665 ; 0,879] | -4.12 | 76.3 | 80.5 | 71.4 | 75.8 | 76.7 | N= 76 [ 41 (1); 35 (2)] |
| 46 | IFNAR1_X227 + LYN_X85 + IFNAR1_X231 + PDE8A_X116246 | C4 | 545 | 0.782 | [0,678; 0,886] | -5.41 | 75.0 | 73.2 | 77.1 | 71.1 | 79.0 | N= 76 [ 41 (1); 35 (2)] |
| 47 | IFNAR1_X84 + LYN_X153 + IFNAR1_X231 + PDE8A_X116246 | C4 | 859 | 0.776 | [0,671 ; 0,881] | -2.93 | 75.0 | 78.1 | 71.4 | 73.5 | 76.2 | N= 76 [ 41 (1); 35 (2)] |
| 48 | IL17RA_X77 + IFNAR1_X227 + IFNAR1_X84 + IFNAR1_X231 + PDE8A_X116246 | C5 | 295 | 0.803 | [0,704 ; 0,903] | -6.86 | 77.6 | 75.6 | 80.0 | 73.7 | 81.6 | N= 76 [ 41 (1); 35 (2)] |
| 49 | IL17RA_X77 + IFNAR1_X227 + IFNAR1_X115 + IFNAR1_X231 + PDE8A_X116246 | C5 | 323 | 0.792 | [0,689 ; 0,896] | -6.82 | 77.6 | 78.1 | 77.1 | 75.0 | 80.0 | N= 76 [ 41 (1); 35 (2)] |
| 50 | IFNAR1_X227 + IFNAR1_X115 + IFNAR1_X231 + IL17RA_X55 + PDE8A_X116246 | C5 | 1463 | 0.794 | [0,691 ; 0,898] | -4.96 | 76.3 | 73.2 | 80.0 | 71.8 | 81.1 | N= 76 [ 41 (1); 35 (2)] |
| 51 | IL17RA X77 + IFNAR1_X227 + LYN_X85 + IFNAR1_X231 + PDE8A_X116246 | C5 | 259 | 0.790 | [0,687 ; 0,893] | -6.62 | 76.3 | 75.6 | 77.1 | 73.0 | 79.5 | N= 76 [ 41 (1); 35 (2)] |
| 52 | IFNAR1_X227 + LYN_X85 + IL17RA_X184 + IFNAR1_X231 + PDE8A_X116246 | C5 | 1304 | 0.788 | [0,685 ; 0,891] | -5.73 | 76.3 | 75.6 | 77.1 | 73.0 | 79.5 | N= 76 [ 41 (1); 35 (2)] |
| 53 | IFNAR1_X227 + IFNAR1_X84 + IL17RA_X184 + IFNAR1_X231 + PDE8A_X116246 | C5 | 1388 | 0.801 | [0,702 ; 0,899] | -6.10 | 75.0 | 70.7 | 80.0 | 70.0 | 80.6 | N= 76 [ 41 (1); 35 (2)] |
| 54 | IFNAR1_X227 + LYN_X85 + IFNAR1_X115 + IFNAR1_X231 + PDE8A_X116246 | C5 | 1268 | 0.796 | [0,696 ; 0,896] | -6.07 | 75.0 | 73.2 | 77.1 | 71.1 | 79.0 | N= 76 [ 41 (1); 35 (2)] |
| 55 | IFNAR1_X227 + IFNAR1_X84 + LYN_X153 + IFNAR1_X231 + PDE8A_X116246 | C5 | 1398 | 0.798 | [0,699 ; 0,897] | -4.70 | 73.7 | 68.3 | 80.0 | 68.3 | 80.0 | N= 76 [ 41 (1); 35 (2)] |
| 56 | IFNAR1_X227 + LYN_X85 + IFNAR1_X84 + IFNAR1_X231 + PDE8A X116246 | C5 | 1240 | 0.794 | [0,694 ; 0,894] | -6.17 | 73.7 | 75.6 | 71.4 | 71.4 | 75.6 | N= 76 [ 41 (1); 35 (2)] |

(continued)

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 57 | IFNAR1_X227 + IFNAR1_X84 + IL17RA_X78 + IFNAR1_X231 + PDE8A X116246 | C5 | 1373 | 0.792 | [0,691 ; 0,893] | -5.16 | 73.7 | 70.7 | 77.1 | 69.2 | 78.4 | N= 76 [41 (1); 35 (2)] |
| 58 | IFNAR1_X227 + IFNAR1_X84 + IFNAR1_X231 + IL17RA_X55 + PDE8A X116246 | C5 | 1407 | 0.790 | [0,688 ; 0,892] | -5.34 | 73.7 | 68.3 | 80.0 | 68.3 | 80.0 | N= 76 [41 (1); 35 (2)] |
| 59 | IL17RA X77 + LYN_X85 + IFNAR1_X84 + IFNAR1_X231 + PDE8A X116246 | C5 | 415 | 0.790 | [0,69 ; 0,89] | -6.12 | 73.7 | 73.2 | 74.3 | 70.3 | 76.9 | N= 76 [41 (1); 35 (2)] |
| 60 | IFNAR1_X227 + IFNAR1_X84 + IFNAR1_X231 + IFNAR1_X111 + PDE8A X116246 | C5 | 1410 | 0.790 | [0,688 ; 0,891] | -5.30 | 72.4 | 61.0 | 85.7 | 65.2 | 83.3 | N= 76 [41 (1); 35 (2)] |
| 61 | IFNAR1_X227 + IFNAR1_X84 + IFNAR1_X115 + IFNAR1_X231 + PDE8A X116246 | C5 | 1352 | 0.789 | [0,688 ; 0,89] | -5.43 | 72.4 | 61.0 | 85.7 | 65.2 | 83.3 | N= 76 [41 (1); 35 (2)] |
| 62 | IL17RA X77 + IFNAR1_X227 + LYN_X85 + IFNAR1_X115 + IFNAR1_X231 + PDE8A_ X116246 | C6 | 553 | 0.803 | [0,702 ; 0,903] | -7.26 | 80.3 | 92.7 | 65.7 | 88.5 | 76.0 | N= 76 [41 (1); 35 (2)] |
| 63 | IL17RA X77 + IFNAR1_X227 + LYN_X85 + IL17RA_X78 + IFNAR1_X231 + PDE8A_ X116246 | C6 | 574 | 0.794 | [0,692 ; 0,897] | -7.59 | 79.0 | 80.5 | 77.1 | 77.1 | 80.5 | N= 76 [41 (1); 35 (2)] |
| 64 | IL17RA X77 + IFNAR1_X227 + IFNAR1_X115 + IL17RA_X78 + IFNAR1_X231 + PDE8A_ X116246 | C6 | 714 | 0.797 | [0,694 ; 0,899] | -7.06 | 77.6 | 78.1 | 77.1 | 75.0 | 80.0 | N= 76 [41 (1); 35 (2)] |
| 65 | IL17RA X77 + IFNAR1_X162 + IFNAR1_X227 + IFNAR1_X84 + IFNAR1_X231 + PDE8A_ X116246 | C6 | 75 | 0.805 | [0,707 ; 0,903] | -6.63 | 76.3 | 73.2 | 80.0 | 71.8 | 81.1 | N= 76 [41 (1); 35 (2)] |
| 66 | IL17RA X77 + IFNAR1_X227 + IFNAR1_X115 + IFNAR1_X231 + IFNAR1_X111 + PDE8A_ X116246 | C6 | 751 | 0.803 | [0,702 ; 0,904] | -6.68 | 76.3 | 75.6 | 77.1 | 73.0 | 79.5 | N= 76 [41 (1); 35 (2)] |
| 67 | IL17RA X77 + IFNAR1_X227 + IFNAR1_X84 + IFNAR1_X231 + IFNAR1_X111 + PDE8A_ X116246 | C6 | 695 | 0.802 | [0,703 ; 0,902] | -6.61 | 76.3 | 78.1 | 74.3 | 74.3 | 78.1 | N= 76 [41 (1); 35 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 68 | IL17RA X77 + IFNAR1_X227 + IFNAR1_X84 + IL17RA_X78 + IFNAR1_X231 + PDE8A_ X116246 | C6 | 658 | 0.801 | [0,702 ; 0,901] | -7.18 | 76.3 | 75.6 | 77.1 | 73.0 | 79.5 | N= 76 [ 41 (1); 35 (2)] |
| 69 | IFNAR1_X227 + IFNAR1_X84 + IL17RA_X78 + IL17RA_X184 + IFNAR1_X231 + PDE8A_ X116246 | C6 | 2354 | 0.798 | [0,698 ; 0,897] | -6.44 | 76.3 | 70.7 | 82.9 | 70.7 | 82.9 | N= 76 [ 41 (1); 35 (2)] |
| 70 | IFNAR1_X227 + LYN_X85 + IFNAR1_X84 + IL17RA_X184 + IFNAR1_X231 + PDE8A_ X116246 | C6 | 2137 | 0.808 | [0,711 ; 0,904] | -6.43 | 75.0 | 82.9 | 65.7 | 76.7 | 73.9 | N= 76 [ 41 (1); 35 (2)] |
| 71 | IFNAR1_X227 + LYN_X85 + IFNAR1_X84 + IFNAR1_X231 + IFNAR1_X111 + PDE8A_ X116246 | C6 | 2159 | 0.803 | [0,706 ; 0,901] | -5.94 | 75.0 | 82.9 | 65.7 | 76.7 | 73.9 | N= 76 [ 41 (1); 35 (2)] |
| 72 | IL17RA_X77 + IFNAR1_X227 + IFNAR1_X84 + IFNAR1_X231 + LYN_X116_T + PDE8A_ X116246 | C6 | 694 | 0.803 | [0,705 ; 0,901] | -6.96 | 75.0 | 80.5 | 68.6 | 75.0 | 75.0 | N= 76 [ 41 (1); 35 (2)] |
| 73 | IFNAR1_X227 + LYN_X85 + IFNAR1_X115 + IFNAR1_X231 + IL17RA_X55 + PDE8A_ X116246 | C6 | 2212 | 0.802 | [0,702 ; 0,902] | -5.94 | 75.0 | 78.1 | 71.4 | 73.5 | 76.2 | N= 76 [ 41 (1); 35 (2)] |
| 74 | IFNAR1_X227 + IFNAR1_X84 + IFNAR1_X115 + IL17RA_X184 + IFNAR1_X231 + PDE8A_ X116246 | C6 | 2319 | 0.802 | [0,704 ; 0,901] | -6.11 | 75.0 | 70.7 | 80.0 | 70.0 | 80.6 | N= 76 [ 41 (1); 35 (2)] |
| 75 | IFNAR1_X227 + LYN_X85 + IFNAR1_X84 + LYN_X153 + IFNAR1_X231 + PDE8A_ X116246 | C6 | 2147 | 0.801 | [0,704 ; 0,899] | -5.36 | 75.0 | 87.8 | 60.0 | 80.8 | 72.0 | N= 76 [ 41 (1); 35 (2)] |
| 76 | IL17RA_X77 + IFNAR1_X227 + IFNAR1_X115 + IFNAR1_X231 + IL17RA_X55 + PDE8A_ X116246 | C6 | 748 | 0.801 | [0,701 ; 0,9] | -8.23 | 75.0 | 70.7 | 80.0 | 70.0 | 80.6 | N= 76 [ 41 (1); 35 (2)] |
| 77 | IFNAR1_X227 + IFNAR1_X84 + IL17RA_X184 + IFNAR1_X231 + IL17RA_X55 + PDE8A_ X116246 | C6 | 2393 | 0.801 | [0,702 ; 0,899] | -6.14 | 75.0 | 70.7 | 80.0 | 70.0 | 80.6 | N= 76 [ 41 (1); 35 (2)] |

(continued)

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 78 | IFNAR1_X227 + IFNAR1_X84 + LYN_X153 + IFNAR1_X231 + IL17RA_X55 + PDE8A_X116246 | C6 | 2403 | 0.799 | [ 0,7 ; 0,897 ] | -4.86 | 73.7 | 58.5 | 91.4 | 65.3 | 88.9 | N= 76 [ 41 (1); 35 (2)] |
| 79 | IFNAR1_X227 + IFNAR1_X84 + IL17RA_X78 + LYN_X153 + IFNAR1_X231 + PDE8A_X116246 | C6 | 2364 | 0.802 | [0,704 ; 0,9 ] | -4.86 | 72.4 | 56.1 | 91.4 | 64.0 | 88.5 | N= 76 [ 41 (1); 35 (2)] |
| 80 | IFNAR1_X227 + IFNAR1_X115 + IFNAR1_X84 + IFNAR1_X162 + IFNAR1_X231 + IFNAR1_X129 + IFNAR1_X111 + LYN_X153 + LYN_X284 + IFNAR1_X152 + IL17RA_X78 + LYN_X116_T + IL17RA X77 + LYN_X47 + IL17RA_X184 + LYN_X85 + LYN_X63 + LYN_X172 + LYN_X203 + IL17RA_X55 + LYN_X163 + PTPRC_X240 + IL17RA_X151 + LYN_X64 + LYN_X255 + LYN_X167 + LYN_X148 + PTPRC_X299 + PDE8A_X116246 | C29 | 14 | 0.977 | [0,948 ; 1 ] | 68.06 | 93.3 | 92.7 | 94.7 | 85.7 | 97.4 | N= 60 [ 41 (1); 19 (2)] |

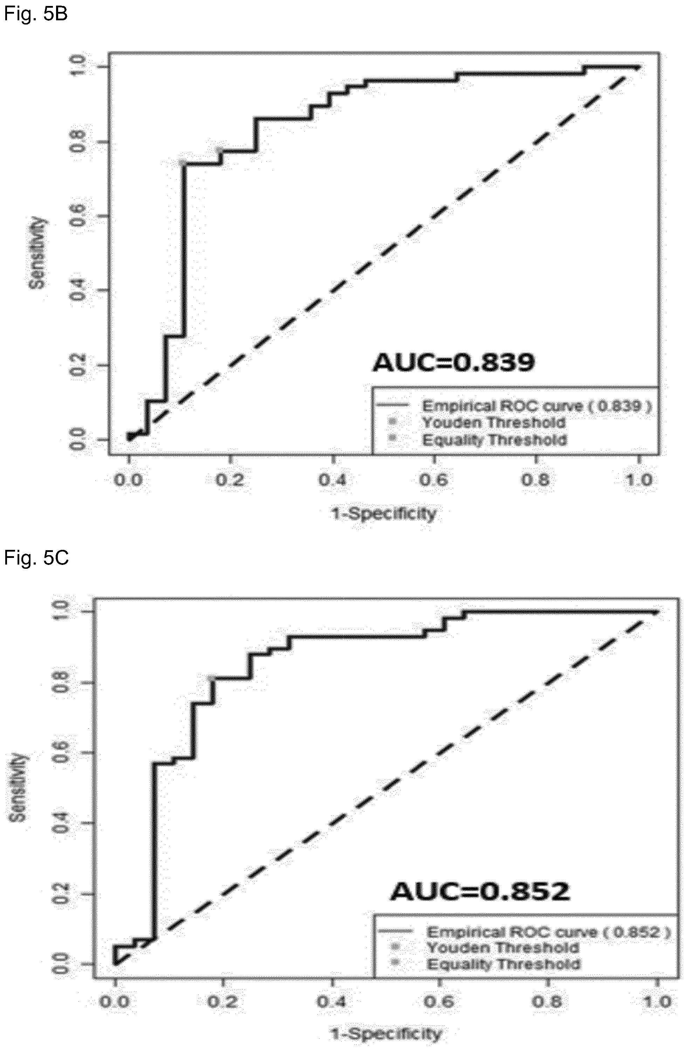

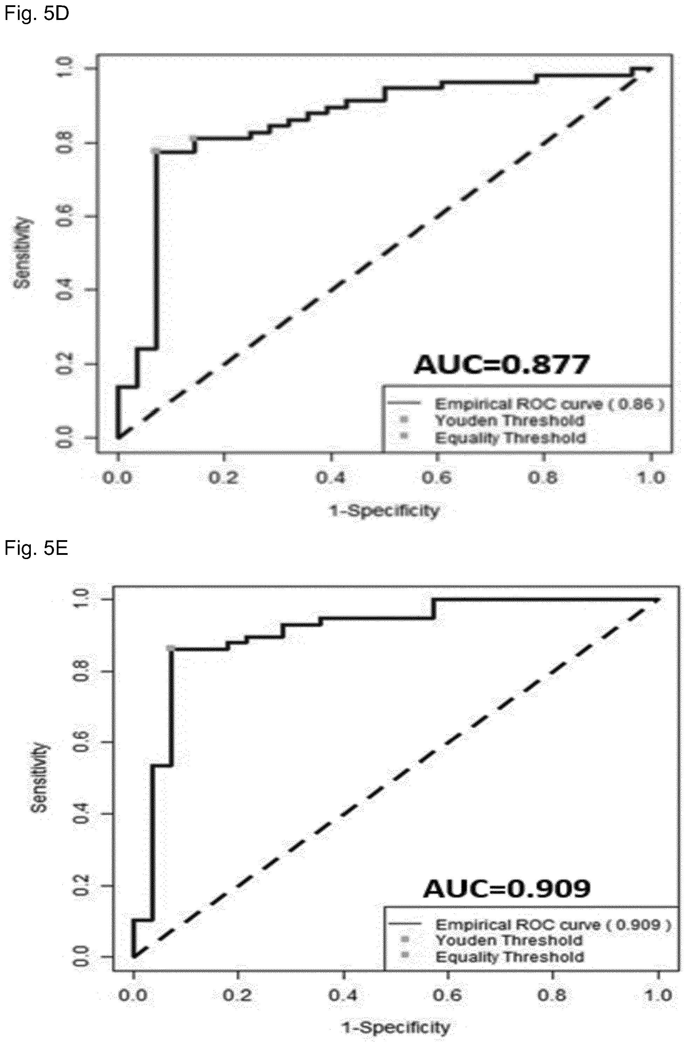

In the second cohort, using multiplexed sequencing, when only 2 sites, each for one BMK, were chosen, the AUC was 0.786 (Sp 78.6%; Se 81.0%, Figure 5A). In this cohort, increasing the number of biomarkers systematically increased diagnostic overall performance of the assay. When 3 sites in 3 BMKs were combined, the AUC increased to 0.839 (Sp 89.3%; Se 74.1%, Figure 5B). With 4 and 5 sites, the AUC rose to 0.852 and 0.860, with specificities of 82.1% and 92.9% and sensitivities of 81% and 77.6%, respectively (Figure 5C and D). When the combination was increased to include 19 sites, the AUC was above 0.9 (AUC 0.909, Sp 92.9%, Se 86.2%, figure 5E). Furthermore, we when we combined 48 differentially edited sites present in 9 different BMKs, we obtained an exceptional AUC of 0.993, with 100% specificity and 96.6% sensitivity (Figure 5F). for this combination the equation used is the following:

$Z=$ - ( 0.179462180023875 )* MDM2_Site207_W - ( -1.04404354492012 )* IFNAR1_Site101_nan - ( 2.64479093229565 )* GAB2_Site88 - ( 4.11125599566128 )* v1MDM2_Site76 - ( 1.61229526347695 )* MDM2_Site143 - ( 5.57184346092994 )* LYN_Site90_S - ( 5.25866173918605 )* PDE8A_Site116248_B - ( 18.995426153835 )* KCNJ15_Site134_D - ( -1.04699821617189 )* v1MDM2_Site86 - ( 0.480007114248953 )* CAMK1D_Site131 - ( 3.56662634744781 )* KCNJ15_Site130_A - ( 2.4602823172239 )* KCNJ15_Site129_B - ( - 9.43134276848121 )* KCNJ15_Site133_C - ( 1.42715048538683 )* IFNAR1_Site76_nan - ( 1.00606326866477 )* CAMK1D_Site49 - ( 0.0542132983688757 )* v1MDM2_Site87_E + ( 5.91151340839856 )* LYN_Site155_X - ( - 2.20437543623734 )* KCNJ15_Site131 - ( 1.30800292033623 )* PTPRC_Site169_E - ( 4.82052635778379 )* MDM2_Site277 - ( -10.1173014238565 )* KCNJ15_Site190 - ( -3.7454636873821 )* PTPRC_Site170_F - ( 1.03362539706716 )* PTPRC_Site259 - ( -4.87935759821363 )* PTPRC_Site163_D - ( 0.628308423459796 )* PRKCB_Site119_E - ( -0.901688238257015 )* KCNJ15_Site193_E - ( -0.425246156880022 )* MDM2_Site264 - ( - 0.533167398964742 )* IFNAR1_Site123_nan - ( -5.64307592868436 )* IFNAR1_Site131_nan - ( 47.981969789844 )* GAB2_Site131_H - ( 0.0402950393688472 )* MDM2_Site237_X - ( -3.33278344933062 )* MDM2_Site192 - ( 0.928449146700883 )* CAMK1D_Site64_D - ( -1.08069775385396 )* GAB2_Site126_A - ( -4.23184777312141 )* CAMK1D_Site113_G - ( 1.99562009099246 )* GAB2_Site52_B - ( 2.3505776086063 )* IFNAR1_Site142_nan - ( 3.97251790792544 )* PRKCB_Site103_C - ( 0.570977372558063 )* PRKCB_Site126 - ( -0.410508492870566 )* PRKCB_Site132_H - ( 0.975605316457069 )* IFNAR1_Site128_nan - ( -1.09288061572838 )* PDE8A_Site116278_C + ( 12.6078350770629 )* CAMK1D_Site125 - ( 1.48817555399057 )* PTPRC_Site253 - ( -0.716028094006562 )* PRKCB_Site99 - ( -0.722585574440859 )* IFNAR1_Site151_nan - ( 0.00161227142613181 )* IFNAR1_Site152_C - (-7.23169188321118)* PRKCB_Site183.

The list of all combination tested in the second prioritization cohort, all of which included the 86 patients, is presented in Table 5. All combinations were statistically significant between control and cases with a p value $<10^{-4}$.

**Table 5:** Diagnostic performances obtained with patients of the second prioritization cohort analyzed with multiplex sequencing

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | GAB2_X88 + KCNJ15_X133 | C2 | 44 | 0.786 | [0.667 ; 0.905 ] | -3.1179 | 80.2 | 78.6 | 81.0 | 88.7 | 66.7 | N= 86 [ 28 (1); 58 (2)] |
| 2 | GAB2_X88 + KCNJ15_X134 | C2 | 39 | 0.800 | [0.685 ; 0.915] | -2.4900 | 79.1 | 78.6 | 79.3 | 88.5 | 64.7 | N= 86 [ 28 (1); 58 (2)] |
| 3 | LYN_X90 + KCNJ15_X134 | C2 | 73 | 0.786 | [0.676 ; 0.896 ] | -1.9946 | 79.1 | 60.7 | 87.9 | 82.3 | 70.8 | N= 86 [ 28 (1); 58 (2)] |
| 4 | GAB2_X88 + KCNJ15_X130 | C2 | 42 | 0.772 | [0.651 ; 0.892 ] | -3.7777 | 79.1 | 71.4 | 82.8 | 85.7 | 66.7 | N= 86 [ 28 (1); 58 (2)] |
| 5 | GAB2_X88 + KCNJ15_X193 | C2 | 51 | 0.778 | [0.662 ; 0.895 ] | -2.5714 | 77.9 | 78.6 | 77.6 | 88.2 | 62.9 | N= 86 [28 (1); 58 (2)] |
| 6 | GAB2_X88 + KCNJ15_X129 | C2 | 43 | 0.782 | [0.663 ; 0.901 ] | -2.0607 | 76.7 | 75.0 | 77.6 | 86.5 | 61.8 | N= 86 [28 (1); 58 (2)] |
| 7 | GAB2_X88 + KCNJ15_X190 | C2 | 48 | 0.774 | [0.656 ; 0.892 ] | -1.3815 | 75.6 | 75.0 | 75.9 | 86.3 | 60.0 | N= 86 [28 (1); 58 (2)] |
| 8 | MDM2_X207 + CAMK1D_X125 | C2 | 31 | 0.779 | [0.676 ; 0.881 ] | -1.2096 | 74.4 | 78.6 | 72.4 | 87.5 | 57.9 | N= 86 [ 28 (1); 58 (2)] |
| 9 | LYN_X90 + KCNJ15_X129 | C2 | 77 | 0.770 | [0.655 ; 0.884 ] | -1.3684 | 74.4 | 71.4 | 75.9 | 84.6 | 58.8 | N= 86 [ 28 (1); 58 (2)] |
| 10 | MDM2_X207 + LYN_X90 | **C2** | 2 | 0.812 | [0.712 ; 0.911 ] | -2.5068 | 73.3 | 85.7 | 67.2 | 90.7 | 55.8 | N= 86 [ 28 (1); 58 (2)] |
| 11 | LYN_X90 + CAMK1D_X131 | C2 | 74 | 0.772 | [0.664 ; 0.879 ] | -0.8314 | 73.3 | 78.6 | 70.7 | 87.2 | 56.4 | N= 86 [28 (1); 58 (2)] |
| 12 | MDM2_X207 + GAB2_X88 | C2 | 1 | 0.785 | [0.685 ; 0.885 ] | -2.7339 | 72.1 | 82.1 | 67.2 | 88.6 | 54.8 | N= 86 [28 (1); 58 (2)] |
| 13 | MDM2_X207 + IFNAR1_X142 | C2 | 26 | 0.782 | [0.683 ; 0.881 ] | -2.2274 | 72.1 | 89.3 | 63.8 | 92.5 | 54.4 | N= 86 [28 (1); 58 (2)] |
| 14 | LYN_X90 + PDE8A_X116248 | C2 | 72 | 0.772 | [0.665 ; 0.878 ] | -7.3860 | 70.9 | 82.1 | 65.5 | 88.4 | 53.5 | N= 86 [28 (1); 58 (2)] |

(continued)

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 15 | GAB2_X88 + LYN_X90 + KCNJ15_X130 | C3 | 635 | 0.823 | [0.714 ; 0.933] | -3.9804 | 83.7 | 67.9 | 91.4 | 85.5 | 79.2 | N= 86 [28 (1); 58 (2)] |
| 16 | GAB2_X88 + LYN_X90 + KCNJ15_X190 | C3 | 641 | 0.816 | [0.708 ; 0.924] | -1.7582 | 82.6 | 71.4 | 87.9 | 86.4 | 74.1 | N= 86 [28 (1); 58 (2)] |
| 17 | GAB2_X88 + KCNJ15_X134 + CAMK1D_X125 | C3 | 724 | 0.817 | [0.709 ; 0.924] | -1.7720 | 82.6 | 71.4 | 87.9 | 86.4 | 74.1 | N= 86 [28 (1); 58 (2)] |
| 18 | GAB2_X88 + KCNJ15_X134 + MDM2_X191 | C3 | 699 | 0.804 | [0.69 ; 0.919] | -2.4499 | 82.6 | 75.0 | 86.2 | 87.7 | 72.4 | N= 86 [28 (1); 58 (2)] |
| 19 | GAB2_X88 + KCNJ15_X134 + KCNJ15_X193 | C3 | 709 | 0.803 | [0.688 ; 0.918] | -1.5841 | 81.4 | 85.7 | 79.3 | 92.0 | 66.7 | N= 86 [28 (1); 58 (2)] |
| 20 | GAB2_X88 + KCNJ15_X134 + KCNJ15_X190 | C3 | 706 | 0.808 | [0.692 ; 0.924] | -3.0436 | 81.4 | 82.1 | 81.0 | 90.4 | 67.7 | N= 86 [28 (1); 58 (2)] |
| 21 | GAB2_X88 + KCNJ15_X133 + IFNAR1_X131 | C3 | 856 | 0.789 | [0.672 ; 0.906] | -3.0585 | 81.4 | 78.6 | 82.8 | 88.9 | 68.8 | N= 86 [28 (1); 58 (2)] |
| 22 | GAB2_X88 + KCNJ15_X133 + GAB2_X52 | C3 | 863 | 0.789 | [0.67 ; 0.909] | -2.3545 | 81.4 | 78.6 | 82.8 | 88.9 | 68.8 | N= 86 [28 (1); 58 (2)] |
| 23 | GAB2_X88 + KCNJ15_X133 + GAB2_X126 | C3 | 861 | 0.787 | [0.668 ; 0.906] | -3.0734 | 81.4 | 78.6 | 82.8 | 88.9 | 68.8 | N= 86 [28 (1); 58 (2)] |
| 24 | GAB2_X88 + KCNJ15_X134 + MDM2_X237 | C3 | 713 | 0.808 | [0.696 ; 0.921] | -3.3208 | 80.2 | 82.1 | 79.3 | 90.2 | 65.7 | N= 86 [28 (1); 58 (2)] |
| 25 | GAB2_X88 + KCNJ15_X134 + CAMK1D_X64 | C3 | 715 | 0.797 | [0.681 ; 0.912] | -2.8067 | 80.2 | 82.1 | 79.3 | 90.2 | 65.7 | N= 86 [28 (1); 58 (2)] |
| 26 | GAB2_X88 + LYN_X90 + KCNJ15_X133 | C3 | 637 | 0.829 | [0.72 ; 0.938] | -3.0116 | 79.1 | 89.3 | 74.1 | 93.5 | 62.5 | N= 86 [28 (1); 58 (2)] |
| 27 | GAB2_X88 + LYN_X90 + KCNJ15_X134 | C3 | 632 | 0.839 | [0.733 ; 0.945] | -2.5595 | 79.1 | 89.3 | 74.1 | 93.5 | 62.5 | N= 86 [28 (1); 58 (2)] |
| 28 | GAB2_X88 + LYN_X90 + KCNJ15_X134 + MDM2_X191 | C4 | 7175 | 0.840 | [0.734 ; 0.945] | -2.6812 | 86.1 | 78.6 | 89.7 | 89.7 | 78.6 | N= 86 [28 (1); 58 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 29 | MDM2_X207 + GAB2_X88 + LYN_X90 + MDM2_X192 | C4 | 19 | 0.841 | [0.742 ; 0.939 ] | -2.9468 | 84.9 | 78.6 | 87.9 | 89.5 | 75.9 | N= 86 [28 (1); 58 (2)] |
| 30 | MDM2_X207 + GAB2_X88 + LYN_X90 + CAMK1D_X131 | C4 | 3 | 0.854 | [ 0.76 ; 0.948 ] | -3.3309 | 83.7 | 78.6 | 86.2 | 89.3 | 73.3 | N= 86 [28 (1); 58 (2)] |
| 31 | GAB2_X88 + KCNJ15_X134 + MDM2_X237 + GAB2_X126 | C4 | 8592 | 0.820 | [0.709 ; 0.932 ] | -2.6335 | 82.6 | 82.1 | 82.8 | 90.6 | 69.7 | N= 86 [28 (1); 58 (2)] |
| 32 | GAB2_X88 + KCNJ15_X134 + KCNJ15_X129 + KCNJ15_X190 | C4 | 8324 | 0.813 | [0.696 ; 0.93 ] | -3.7791 | 82.6 | 85.7 | 81.0 | 92.2 | 68.6 | N= 86 [ 28 (1); 58 (2)] |
| 33 | MDM2_X207 + KCNJ15_X134 + IFNAR1_X142 + IFNAR1_X128 | C4 | 2129 | 0.816 | [0.718 ; 0.914] | -3.0729 | 81.4 | 89.3 | 77.6 | 93.8 | 65.8 | N= 86 [ 28 (1); 58 (2)] |
| 34 | GAB2_X88 + LYN_X90 + KCNJ15_X134 + CAMK1D_X125 | C4 | 7200 | 0.852 | [0.752 ; 0.953 ] | -1.6869 | 81.4 | 82.1 | 81.0 | 90.4 | 67.7 | N= 86 [ 28 (1); 58 (2)] |
| 35 | MDM2_X207 + GAB2_X88 + LYN_X90 + KCNJ15_X134 + IFNAR1_X142 | C5 | 27 | 0.850 | [0.751 ; 0.949 ] | -3.8492 | 88.4 | 82.1 | 91.4 | 91.4 | 82.1 | N= 86 [ 28 (1); 58 (2)] |
| 36 | GAB2_X88 + LYN_X90 + KCNJ15_X134 + CAMK1D_X131 + PTPRC_X170 | C5 | 4005 | 0.854 | [0.753 ; 0.955 ] | -3.2924 | 87.2 | 71.4 | 94.8 | 87.3 | 87.0 | N= 86 [ 28 (1); 58 (2)] |
| 37 | GAB2_X88 + LYN_X90 + KCNJ15_X134 + PRKCB_X103 + PRKCB_X132 | C5 | 4097 | 0.853 | [0.751 ; 0.955 ] | -2.7727 | 86.1 | 78.6 | 89.7 | 89.7 | 78.6 | N= 86 [ 28 (1); 58 (2)] |
| 38 | GAB2_X88 + LYN_X90 + MDM2_X191 + KCNJ15_X129 + PRKCB_X132 | C5 | 4214 | 0.828 | [0.722 ; 0.935 ] | -2.1014 | 86.1 | 75.0 | 91.4 | 88.3 | 80.8 | N= 86 [ 28 (1); 58 (2)] |
| 39 | GAB2_X88 + LYN_X90 + CAMK1D_X131 + KCNJ15_X130 + CAMK1D_X49 | C5 | 4118 | 0.845 | [0.744 ; 0.945 ] | -3.6631 | 84.9 | 82.1 | 86.2 | 90.9 | 74.2 | N= 86 [ 28 (1); 58 (2)] |
| 40 | GAB2_X88 + LYN_X90 + KCNJ15_X134 + MDM2_X191 + KCNJ15_X190 | C5 | 4017 | 0.841 | [0.736 ; 0.946 ] | -3.3395 | 84.9 | 82.1 | 86.2 | 90.9 | 74.2 | N= 86 [ 28 (1); 58 (2)] |
| 41 | GAB2_X88 + LYN_X90 + PDE8A_X116248 + CAMK1D_X131 + KCNJ15_X129 | C5 | 3894 | 0.840 | [ 0.74 ; 0.94 ] | -5.7536 | 84.9 | 82.1 | 86.2 | 90.9 | 74.2 | N= 86 [ 28 (1); 58 (2)] |

EP 4 055 190 B1

32

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 42 | MDM2_X207 + LYN_X90 + KCNJ15_X134 + CAMK1D_X131 + KCNJ15_X133 | C5 | 940 | 0.860 | [0.77; 0.951 ] | -0.7551 | 82.6 | 92.9 | 77.6 | 95.7 | 66.7 | N= 86 [ 28 (1); 58 (2)] |
| 43 | MDM2_X207 + GAB2_X88 + LYN_X90 + KCNJ15_X134 + IFNAR1_X76 + IFNAR1_X142 | C6 | 185 | 0.849 | [0.75; 0.947] | -3.6239 | 88.4 | 85.7 | 89.7 | 92.9 | 80.0 | N= 86 [ 28 (1); 58 (2)] |
| 44 | GAB2_X88 + LYN_X90 + KCNJ15_X134 + MDM2_X191 + PRKCB_X103 + PRKCB_X132 | C6 | 12353 | 0.858 | [0.756 ; 0.959 ] | -2.4262 | 88.4 | 78.6 | 93.1 | 90.0 | 84.6 | N= 86 [ 28 (1); 58 (2)] |
| 45 | MDM2_X207 + GAB2_X88 + LYN_X90 + KCNJ15_X134 + IFNAR1_X142 + IFNAR1_X128 | C6 | 219 | 0.853 | [0.756 ; 0.95 ] | -3.9043 | 87.2 | 85.7 | 87.9 | 92.7 | 77.4 | N= 86 [ 28 (1); 58 (2)] |
| 46 | GAB2_X88 + LYN_X90 + CAMK1D_X131 + KCNJ15_X129 + CAMK1D_X49 + PTPRC_X170 | C6 | 12809 | 0.855 | [0.755 ; 0.954 ] | -3.3713 | 87.2 | 82.1 | 89.7 | 91.2 | 79.3 | N= 86 [ 28 (1); 58 (2)] |
| 47 | GAB2_X88 + LYN_X90 + KCNJ15_X134 + IFNAR1_X142 + PRKCB_X103 + PRKCB_X132 | C6 | 12635 | 0.854 | [0.754 ; 0.954 ] | -2.5817 | 87.2 | 82.1 | 89.7 | 91.2 | 79.3 | N= 86 [ 28 (1); 58 (2)] |
| 48 | GAB2_X88 + LYN_X90 + KCNJ15_X133 + PRKCB_X103 + PRKCB_X126 + PRKCB_X132 | C6 | 13795 | 0.840 | [0.734 ; 0.946 ] | -2.9446 | 87.2 | 82.1 | 89.7 | 91.2 | 79.3 | N= 86 [ 28 (1); 58 (2)] |
| 49 | MDM2_X207 + GAB2_X88 + LYN_X90 + KCNJ15_X134 + CAMK1D_X131 + KCNJ15_X190 | C6 | 128 | 0.877 | [0.786 ; 0.968 ] | -5.1449 | 86.1 | 85.7 | 86.2 | 92.6 | 75.0 | N= 86 [ 28 (1); 58 (2)] |
| 50 | MDM2_X207 + GAB2_X88 + LYN_X90 + PDE8A_X116248 + CAMK1D_X49 + IFNAR1_X142 | C6 | 88 | 0.861 | [0.773 ; 0.949 ] | -7.4108 | 86.1 | 85.7 | 86.2 | 92.6 | 75.0 | N= 86 [ 28 (1); 58 (2)] |
| 51 | MDM2_X207 + GAB2_X88 + LYN_X90 + PDE8A_X116248 + KCNJ15_X134 + IFNAR1_X142 | C6 | 11 | 0.858 | [0.766 ; 0.95 ] | -7.0457 | 86.1 | 85.7 | 86.2 | 92.6 | 75.0 | N= 86 [ 28 (1); 58 (2)] |

EP 4 055 190 B1

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 52 | MDM2_X207 + GAB2_X88 + LYN_X90 + KCNJ15_X134 + KCNJ15_X190 + IFNAR1_X142 | C6 | 206 | 0.852 | [0.755 ; 0.948 ] | -5.4371 | 86.1 | 85.7 | 86.2 | 92.6 | 75.0 | N= 86 [ 28 (1); 58 (2)] |
| 53 | MDM2_X207 + KCNJ15_X134 + CAMK1D_X131 + MDM2_X191 + IFNAR1_X142 + IFNAR1_X128 | C6 | 7332 | 0.819 | [0.724 ; 0.914] | -3.3357 | 83.7 | 89.3 | 81.0 | 94.0 | 69.4 | N= 86 [ 28 (1); 58 (2)] |
| 54 | MDM2_X207 + LYN_X90 + KCNJ15_X134 + CAMK1D_X131 + KCNJ15_X133 + CAMK1D_X49 | C6 | 3658 | 0.861 | [ 0.77 ; 0.951 ] | -0.8548 | 82.6 | 92.9 | 77.6 | 95.7 | 66.7 | N= 86 [ 28 (1); 58 (2)] |
| 55 | MDM2_X207 + GAB2_X88 + LYN_X90 + PDE8A_X116248 + KCNJ15_X134 + CAMK1D_X131 + MDM2_X191 + KCNJ15_X130 + KCNJ15_X129 + KCNJ15_X133 + IFNAR1_X76 + CAMK1D_X49 + MDM2_X87 + KCNJ15_X190 + IFNAR1_X142 + PRKCB_X103 + PRKCB_X126 + PRKCB_X132 + IFNAR1_X128 | C19 | 6 | 0.909 | [0.833 ; 0.985 ] | -6.0428 | 88.4 | 92.9 | 86.2 | 96.2 | 76.5 | N= 86 [ 28 (1); 58 (2)] |
| 56 | MDM2_X207 + GAB2_X88 + LYN_X90 + PDE8A_X116248 + KCNJ15_X134 + CAMK1D_X131 + MDM2_X191 + KCNJ15_X130 + KCNJ15_X129 + KCNJ15_X133 + IFNAR1_X76 + CAMK1D_X49 + MDM2_X87 + KCNJ15_X190 + PTPRC_X170 + PRKCB_X103 + PRKCB_X126 + PRKCB_X132 + IFNAR1_X128 | C19 | 5 | 0.908 | [0.829 ; 0.986 ] | -5.2338 | 88.4 | 89.3 | 87.9 | 94.4 | 78.1 | N= 86 [ 28 (1); 58 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 57 | MDM2_X207 + GAB2_X88 + LYN_X90 + PDE8A_X116248 + KCNJ15_X134 + CAMK1D_X131 + MDM2_X191 + KCNJ15_X130 + KCNJ15_X129 + KCNJ15_X133 + IFNAR1_X76 + CAMK1D_X49 + MDM2_X87 + KCNJ15_X190 + PTPRC_X170 + PRKCB_X119 + KCNJ15_X193 + IFNAR1_X123 + IFNAR1_X131 + GAB2_X131 + MDM2_X237 + MDM2_X192 + CAMK1D_X64 + GAB2_X126 + CAMK1D_X113 + GAB2_X52 + PRKCB_X103 + PRKCB_X126 + PRKCB_X132 + IFNAR1_X128 + CAMK1D_X125 + MDM2_X82 + PTPRC_X253 + PRKCB_X99 + IFNAR1_X151 + IFNAR1_X152 | C36 | 11 | 0.969 | [0.938 ; 0.999 ] | 36.3081 | 94.2 | 92.9 | 94.8 | 96.5 | 89.7 | N= 86 [ 28 (1); 58 (2)] |
| 58 | MDM2_X207 + GAB2_X88 + LYN_X90 + KCNJ15_X134 + CAMK1D_X131 + MDM2_X191 + KCNJ15_X130 + KCNJ15_X129 + KCNJ15_X133 + IFNAR1_X76 + CAMK1D_X49 + MDM2_X87 + KCNJ15_X190 + PTPRC_X170 + PRKCB_X119 + KCNJ15_X193 + IFNAR1_X123 + IFNAR1_X131 + GAB2_X131 + MDM2_X237 + MDM2_X192 + CAMK1D_X64 + GAB2_X126 + CAMK1D_X113 + GAB2_X52 + IFNAR1_X142 + PRKCB_X103 + PRKCB_X126 + PRKCB_X132 + IFNAR1_X128 + CAMK1D_X125 + MDM2_X82 + PTPRC_X253 + PRKCB_X99 + IFNAR1_X151 + IFNAR1_X152 | C36 | 34 | 0.963 | [0.925 ; 1 ] | 46.2509 | 94.2 | 96.4 | 93.1 | 98.2 | 87.1 | N= 86 [ 28 (1); 58 (2)] |

EP 4 055 190 B1

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 59 | MDM2_X207 + GAB2_X88 + LYN_X90 + PDE8A_X116248 + KCNJ15_X134 + CAMK1D_X131 + MDM2_X191 + KCNJ15_X130 + KCNJ15_X129 + KCNJ15_X133 + IFNAR1_X76 + CAMK1D_X49 + MDM2_X87 + KCNJ15_X190 + PTPRC_X170 + PRKCB_X119 + KCNJ15_X193 + IFNAR1_X123 + IFNAR1_X131 + GAB2_X131 + MDM2_X237 + MDM2_X192 + CAMK1D_X64 + GAB2_X126 + CAMK1D_X113 + GAB2_X52 + IFNAR1_X142 + PRKCB_ X103 + PRKCB_X126 + PRKCB_X132 + CAMK1D_X125 + MDM2_X82 + PTPRC_ X253 + PRKCB_X99 + IFNAR1_X151 + IFNAR1_X152 | C36 | 7 | 0.967 | [0.935 ; 0.999 ] | 15.6122 | 89.5 | 100.0 | 84.5 | 100.0 | 75.7 | N= 86 [ 28 (1); 58 (2)] |

EP 4 055 190 B1

(continued)

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_X207 + IFNAR1_X101 + GAB2_X88 + MDM2_X76 + MDM2_X143 + LYN_X90 + PDE8A_X116248 + KCNJ15_X134 + MDM2_X86 + CAMK1D_X131 + KCNJ15_X130 + KCNJ15_X129 + KCNJ15_X133 + IFNAR1_X76 + CAMK1D_X49 + MDM2_X87 + LYN_X155_X + KCNJ15_X131 + PTPRC_X169 + MDM2_X277 + KCNJ15_X190 + PTPRC_X170 + PTPRC_X259 + PTPRC_X163 + PRKCB_X119 + KCNJ15_X193 + MDM2_X264 + IFNAR1_X123 + IFNAR1_X131 + GAB2_X131 + MDM2_X237 + MDM2_X192 + CAMK1D_X64 + GAB2_X126 + CAMK1D_X113 + GAB2_X52 + IFNAR1_X142 + PRKCB_X103 + PRKCB_X126 + PRKCB_X132 + IFNAR1_X128 + PDE8A_X116278 + CAMK1D_X125 + PTPRC_X253 + PRKCB_X99 + IFNAR1_X151 + IFNAR1_X152 + PRKCB_X183 | C48 | 747 | 0.993 | [0.981 ; 1] | -84.5770 | 97.7 | 100.0 | 96.6 | 100.0 | 93.3 | N = 86 [28 (1); 58 (2)] |

Altogether, these data suggest that the different biomarkers in the panel of 14 genes selected in the prioritization cohorts could be used to sequentially diagnose DEP. Importantly, all the identified biomarkers included in this panel of 14 genes were included in at least one combination, suggesting that all those BMKs could be used to diagnose DEP with suitable diagnostic performances.

A panel of the 8 most interesting biomarkers was selected and sequenced in a multiplex format to further validate our results in a large cohort (validation cohort) of 411 subjects, of which 143 were healthy controls and 268 were DEP patients (Figure 19).

All BMKs were combined with each other to evaluate the potential increase in sensibility and specificity using RandomForest (RF), a machine learning approach. Analysis of the RNA editing variants of 8 BMKs (GAB2, IFNAR1, KCNJ15, LYN, MDM2, PRKCB, CAMK1D and PDE8A) gave an AUC ROC curve of 0.929 (CI 95%: [0.876 - 0.981]), with a sensitivity of 82.9% and a specificity of 87.1% (Figure 22A) on test dataset, for the model1 adjusted by age, sex, psychiatric treatment and addiction.

**Table 5.1 : RandomForest model adjusted by age, sex, psychiatric treatment and addictions (model1, see Fig. 22A)**

| List of target in RandomForest model1 | List of A-I RNA editing variants in RandomForest model1 |
|---|---|
| IFNAR1, GAB2, KCNJ15, MDM2, LYN, PRKCB, CAMK1D, | CAMK1D_E, CAMK1D_FS, IFNAR1.1_Site33355798, IFNAR1.1_Site33355809, IFNAR1.1_Site33355817, IFNAR1.1_Site33355838_P, IFNAR1.1_Site33355840_E, GAB2_AE, GAB2_BCDE, GAB2_CE, GAB2_D, GAB2_EJ, GAB2_Site78331030, GAB2_Site78331054, KCNJ15_C, KCNJ15_CE, KCNJ15_Site38287584, LYN_AC, LYN_Site56012495, MDM2_AIKTVW, MDM2_I, MDM2_SVW, MDM2_SW, PDE8A MDM2_SWX, MDM2_Site68821573, MDM2_Site68821584, MDM2_Site68821586_W, MDM2_Site68821602, MDM2_Site68821616_X, MDM2_Site68821643, MDM2_W, PDE8A_B, PDE8A_Site85096687_B, PRKCB_B, PRKCB_ DHIJKM, PRKCB_DIJ, PRKCB_DIJK, PRKCB_IJK, PRKCB_NoEdit, PRKCB_Site23920893_G, PRKCB_ Site23920902_I, PRKCB_Site23920904, PRKCB_ Site23920910_J, PRKCB_Site23920911, PRKCB_ Site23920919_K |

In addition, the randomForest model2 adjusted only by age and sex using the same 8 BMKs (CAMK1D, IFNAR1, GAB2, MDM2, KCNJ15, PRKCB, LYN and PDE8A) gave an AUC ROC curve of 0.911 (CI 95%: [0.854 - 0.968]), with a sensitivity of 80.0% and a specificity of 82.4% (Figure 22B) on test dataset.

Both models allow a clear separation of control from depressed patients

**Table 5.2 RandomForest model adjusted by age and sex (model2, see Fig. 22B)**

| List of target in randomForest model2 | List of A-I RNA editing variants in randomForest model2 |
|---|---|
| IFNAR1, GAB2, KCNJ15, MDM2, LYN, PRKCB, CAMK1D, PDE8A | CAMK1D_A, CAMK1D_AE, CAMK1D_E, CAMK1D_ Site12378440, IFNAR1.1_Site33355789 A, IFNAR1.1_Site33355793_G, IFNAR1.1_Site33355798, IFNAR1.1_Site33355809, IFNAR1.1_Site33355817, IFNAR1.1_Site33355838_P, IFNAR1.1_Site33355839_L, IFNAR1.1_Site33355840_E, GAB2_CE, GAB2_Site78331054, KCNJ15_C, KCNJ15_CE, |

(continued)

| List of target in randomForest model2 | List of A-I RNA editing variants in randomForest model2 |
|---|---|
| | KCNJ15_Site38287525_B, KCNJ15_Site38287529_C, KCNJ15_Site38287530_D, KCNJ15_Site38287586, KCNJ15_Site38287589_E, KCNJ15_Site38287590, KCNJ15_Site38287591, LYN_A, LYN_AC, MDM2_ASV, MDM2_KSX, MDM2_SVW, MDM2_SW, MDM2_SWX, MDM2_Site68821573, MDM2_Site68821586_W, MDM2_Site68821602, MDM2_Site68821616_X, MDM2_Site68821643, MDM2_T, MDM2_W, MDM2_WX, PDE8A_B, PDE8A_Site85096687_B, |
| | PRKCB_B, PRKCB_DIJ, PRKCB_DIJK, PRKCB_DJ, PRKCB_I, PRKCB_IJK, PRKCB_J, PRKCB_JK, PRKCB_Site23920884_D, PRKCB_Site23920893_G, PRKCB_Site23920902_I, PRKCB_Site23920904, PRKCB_Site23920910_J, PRKCB_Site23920911, PRKCB_Site23920919_K, PRKCB_Site23920921_L |

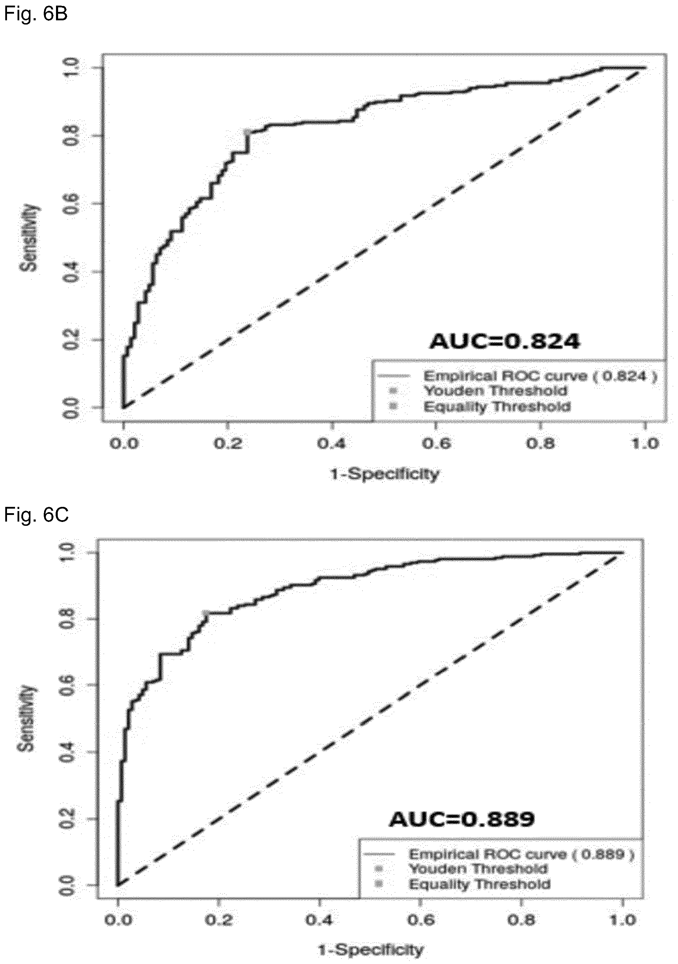

When we analyzed the editing sites of the whole population and combined 17 editing variants in 5 BMKs, we obtained an AUC of 0.751 (Sp 70.6%; Se 72.4%, Figure 6A). When the 8 biomarkers included in the multiplex were used and 53 differentially edited sites were combined, the AUC rose to 0.824, with a Specificity of 76.2% and Sensitivity of 81% (Figure 6B). When the number of editing sites in those 8 BMKs was further increased to reach 79 sites, all of which were sequenced in multiplex in a single experiment, the AUC further increased to 0.899 (Sp 82.5%; Se 81.7%, Figure 6C). In the latter case, the equation used is the following:

$Z$= - ( -0.181885362537332 )* MDM2_Site68821586_W - ( 0.45875321689627 )* MDM2_SWX + ( 0.932293378426399 )* PRKCB_Site23920910_J - ( 0.235744342326864 )* MDM2_W - ( 0.188045930057369 )* MDM2_Site68821643 + ( 0.129971821791191 )* PRKCB_DIJK - ( 0.112543222902224 )* MDM2_SVW + ( 0.0714766020787205 )* CAMK1D_E - ( -0.0566149561843161 )* MDM2_SW + ( 0.343458398647021 )* PRKCB_B - (0.0511754835571903)* GAB2_Site78331054 + ( 0.0777964634309061 )* PRKCB_Site23920911 + ( -0.309760257245042 )* PRKCB_Site23920919_K + ( 0.0892196021964463 )* MDM2_T + ( 0.0591702551145443 )* PRKCB_J - ( 0.163034043783367 )* KCNJ15_CE - ( - 0.3554615992579 )* MDM2_Site68821616_X + ( 0.194383441652219 )* CAMK1D_AE - ( -0.0357242710579534 )* KCNJ15_C + ( -0.137804039545265 )* PRKCB_JK - ( 0.073268779527063 )* GAB2_CE - ( 0.583431075372027 )* PDE8A_Site85096687_B - ( 0.083656284732413 )* KCNJ15_Site38287529_C + ( 0.241597825714507 )* PRKCB_Site23920902_I + ( 0.0897642626007787 )* PRKCB_DIJ + ( 0.311212124874424 )* PRKCB_Site23920893_G - ( - 0.0694365074403336 )* KCNJ15_Site38287586 - ( 0.356961148091174 )* KCNJ15_Site38287525_B - ( 0.0284155839590126 )* MDM2_Site68821573 + ( 0.161274457136425 )* IFNAR1.1_Site33355809 - ( -0.578010271214577 )* PDE8A_B + ( 0.00936832259402957 )* PRKCB_Site23920921_L - ( - 0.0220552696975651 )* MDM2_WX + ( 0.0416981587266934 )* IFNAR1.1_Site33355817 - ( 0.056383762969179 )* KCNJ15_Site38287589_E + ( 0.034447202518999 )* IFNAR1.1_Site33355838_P - ( 0.181855602464856 )* KCNJ15_Site38287590 + ( -0.232428154178395 )* PRKCB_Site23920884_D - ( 0.0566507858081719 )* KCNJ15_Site38287530_D + ( 0.00350968357773277 )* PRKCB_IJK - ( 0.0986696717892891 )* KCNJ15_Site38287591 + ( 0.0416357072635886 )* PRKCB_Site23920889_F + ( -0.510062501581064 )* PRKCB_Site23920844_B + ( -0.201713338082801 )* PRKCB_DJ - ( 0.219330533848547 )* LYN_AC + ( -0.0902753379195889 )* IFNAR1.1_Site33355798 + ( 0.0364611306918377 )* PRKCB_DHIJK + ( -0.0154244319603596 )* GAB2_BCDE + ( -0.0721590982562632 )* 0.130383083484889 )* LYN_AG + ( 0.136853965680293 )* GAB2_Site78331030 - ( 0.201599671305856 )* CAMK1D_Site12378440 + ( -0.0494625908197476 )* IFNAR1.1_Site33355793_G - ( 0.112438468962481 )* MDM2_KSX + ( 0.129569099073175 )* IFNAR1.1_Site33355839_L - ( 0.12561875043809 )* GAB2_ABDE - ( 0.127875837752046 )* PRKCB_DJM - ( -0.421356734328941 )* KCNJ15_ACE - ( 0.125572361520418 )* MDM2_ASV - ( -0.0421188089161209 )* MDM2_Site68821576_V + ( 0.333082982874752 )* LYN_A + (0.205974310972437 )* IFNAR1.1_Site33355762_F - ( 0.26063881758559 )* MDM2_Site68821602 - ( 0.16091158013623 )* LYN_Site56012491 - ( 0.585856554487505 )* PRKCB_Site23920927_M - ( -0.328951375049536 )* KCNJ15_Site38287526_A + ( 0.0681422708577013 )* GAB2_EJ + ( 0.0265646151191535 )* PRKCB_I + ( - 0.114728592530574 )* LYN_Site56012490 + ( 0.0423564487829415 )* CAMK1D_Site12378387 + ( -0.270495232628549 )* IFNAR1.1_Site33355807_B - ( 0.202529212838335 )* PRKCB_Site23920952 - ( -0.0881700257476326 )* KCNJ15_ACD + ( 0.0213312643325165 )* GAB2_DE + ( -0.00540846808767115 )* MDM2_STX + ( 0.18510874500411 )* GAB2_Site78331008_D - ( 0.060213548631588 )* MDM2_Site68821567 - ( -0.123438884158084 )* MDM2_Site68821612_A

The list of combinations tested on the whole population in the validation cohort (n=411), is presented in Table 6. All combinations were statistically significant between control and cases with a p value $<10^{-4}$.

EP 4 055 190 B1

**Table 6:** Diagnostic performances obtained with patients of the whole population of the validation cohort (n=411) analyzed by multiplex sequencing of 8 targets

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | MDM2_Site68821586_W MDM2_SWX PRKCB_Site23920910_J MDM2_W MDM2_Site68821643 PRKCB_DIJK MDM2_SVW CAMK1D_E MDM2_SW PRKCB_B GAB2_Site78331054 PRKCB_Site23920911 PRKCB_Site23920919_K MDM2_T PRKCB_J KCNJ15_CE MDM2_Site68821616_X CAMK1D_AE KCNJ15_C PRKCB_JK GAB2_CE PDE8A_Site85096687_B KCNJ15_Site38287529_C PRKCB_Site23920902_I PRKCB_DIJ PRKCB_Site23920893_G KCNJ15_Site38287586 KCNJ15_Site38287525_B MDM2_Site68821573 IFNAR1.1_Site33355809 PDE8A_B PRKCB_Site23920921_L MDM2_WX IFNAR1.1_Site33355817 KCNJ15_Site38287589_E IFNAR1.1_Site33355838_P KCNJ15_Site38287590 PRKCB_Site23920884_D KCNJ15_Site38287530_D PRKCB_IJK KCNJ15_Site38287591 PRKCB_Site23920889_F PRKCB_Site23920844_B PRKCB_DJ LYN_AC IFNAR1.1_Site33355798 PRKCB_DHIJK GAB2_BCDE IFNAR1.1_Site33355801_N CAMK1D_A LYN_AG GAB2_Site78331030 CAMK1D_Site12378440 IFNAR1.1_Site33355793_G MDM2_KSX IFNAR1.1_Site33355839_L IFNAR1.1_Site33355789_A GAB2_ABDE PRKCB_DJM KCNJ15_ACE MDM2_ASV MDM2_Site68821576_V LYN_A IFNAR1.1_Site33355762_F MDM2_Site68821602 LYN_Site56012491 PRKCB_Site23920927_M KCNJ15_Site38287526_A GAB2_EJ PRKCB_I LYN_Site56012490 CAMK1D_Site12378387 IFNAR1.1_Site33355807_B PRKCB_Site23920952 KCNJ15_ACD GAB2_DE MDM2_STX GAB2_Site78331008_D MDM2_Site68821567 MDM2_Site68821612_A | C80 | 49 | 0.889 | [ 0.858 ; 0.92 ] | -0.1901 | 81.02 | 82.52 | 80.22 | 89.58 | 69.01 | N= 411 [ 143 (1); 268(2)] |

41

| | | | | | | | | | | | |
|---|---|---|---|---|---|---|---|---|---|---|---|
| 2 | MDM2_Site68821586_W MDM2_SWX PRKCB_Site23920910_J MDM2_W MDM2_Site68821643 PRKCB_DIJK MDM2_SVW CAMK1D_E MDM2_SW PRKCB_B GAB2_Site78331054 PRKCB_Site23920911 PRKCB_Site23920919_K MDM2_T PRKCB_J KCNJ15_CE MDM2_Site68821616_X CAMK1D_AE KCNJ15_C PRKCB_JK GAB2_CE PDE8A_Site85096687_B KCNJ15_Site38287529_C PRKCB_Site23920902_I PRKCB_DIJ PRKCB_Site23920893_G KCNJ15_Site38287586 KCNJ15_Site38287525_B MDM2_Site68821573 IFNAR1.1_Site33355809 PDE8A_B PRKCB_Site23920921_L IFNAR1.1_Site33355840_E MDM2_WX IFNAR1.1_Site33355817 KCNJ15_Site38287589_E IFNAR1.1_Site33355838_P KCNJ15_Site38287590 PRKCB_Site23920884_D KCNJ15_Site38287530_D PRKCB_IJK KCNJ15_Site38287591 PRKCB_Site23920889_F PRKCB_Site23920844_B PRKCB_DJ LYN_AC IFNAR1.1_Site33355798 PRKCB_DHIJK GAB2_BCDE IFNAR1.1_Site33355801_N CAMK1D_A LYN_AG GAB2_Site78331030 CAMK1D_Site12378440 IFNAR1.1_Site33355793_G MDM2_KSX IFNAR1.1_Site33355839_L IFNAR1.1_Site33355789_A GAB2_ABDE PRKCB_DJM KCNJ15_ACE MDM2_ASV MDM2_Site68821576_V LYN_A IFNAR1.1_Site33355762_F MDM2_Site68821602 LYN_Site56012491 PRKCB_Site23920927_M KCNJ15_Site38287526_A GAB2_EJ PRKCB_I LYN_Site56012490 CAMK1D_Site12378387 PRKCB_Site23920952 KCNJ15_ACD GAB2_DE MDM2_STX GAB2_Site78331008_D MDM2_Site68821567 MDM2_Site68821612_A | C80 | 8 | 0.889 | [ 0.858 ; 0.92 ] | -0.2879 | 82.97 | 79.02 | 83.21 | 88.14 | 71.52 | N= 411 [ 143 (1); 268 (2)] |

| 3 | MDM2_Site68821586_W MDM2_SWX PRKCB_Site23920910_J MDM2_W MDM2_Site68821643 PRKCB_DIJK MDM2_SVW CAMK1D_E MDM2_SW PRKCB_B GAB2_Site78331054 PRKCB_Site23920911 PRKCB_Site23920919_K MDM2_T PRKCB_J KCNJ15_CE MDM2_Site68821616_X CAMK1D_AE KCNJ15_C PRKCB_JK GAB2_CE PDE8A_Site85096687_B KCNJ15_Site38287529_C PRKCB_Site23920902_I PRKCB_DIJ PRKCB_Site23920893_G KCNJ15_Site38287586 KCNJ15_Site38287525_B MDM2_Site68821573 IFNAR1.1_Site33355809 PDE8A_B PRKCB_Site23920921_L IFNAR1.1_Site33355840_E MDM2_WX IFNAR1.1_Site33355817 KCNJ15_Site38287589_E IFNAR1.1_Site33355838_P KCNJ15_Site38287590 PRKCB_Site23920884_D KCNJ15_Site38287530_D PRKCB_IJK KCNJ15_Site38287591 PRKCB_Site23920889_F PRKCB_Site23920844_B PRKCB_DJ LYN_AC IFNAR1.1_Site33355798 PRKCB_DHIJK GAB2_BCDE IFNAR1.1_Site33355801_N CAMK1D_A LYN_AG GAB2_Site78331030 CAMK1D_Site12378440 IFNAR1.1_Site33355793_G MDM2_KSX IFNAR1.1_Site33355839_L IFNAR1.1_Site33355789_A GAB2_ABDE PRKCB_DJM KCNJ15_ACE MDM2_ASV MDM2_Site68821576_V LYN_A IFNAR1.1_Site33355762_F LYN_Site56012491 PRKCB_Site23920927_M KCNJ15_Site38287526_A GAB2_EJ PRKCB_I LYN_Site56012490 CAMK1D_Site12378387 IFNAR1.1_Site33355807_B PRKCB_Site23920952 KCNJ15_ACD GAB2_DE MDM2_STX GAB2_Site78331008_D MDM2_Site68821567 MDM2_Site68821612_A | C80 | 16 | **0.889** | [ 0.858 ; 0.92 ] | -0.2398 | 81.02 | **81.82** | **80.6** | 89.26 | 69.23 | N= 411 [ 143 (1); 268 (2)] |

| 4 | MDM2_Site68821586_W MDM2_SWX PRKCB_Site23920910_J MDM2_W MDM2_Site68821643 PRKCB_DIJK MDM2_SVW CAMK1D_E MDM2_SW PRKCB_B GAB2_Site78331054 PRKCB_Site23920911 PRKCB_Site23920919_K MDM2_T PRKCB_J KCNJ15_CE MDM2_Site68821616_X CAMK1D_AE KCNJ15_C PRKCB_JK GAB2_CE PDE8A_Site85096687_B KCNJ15_Site38287529_C PRKCB_Site23920902_I PRKCB_DIJ PRKCB_Site23920893_G KCNJ15_Site38287586 KCNJ15_Site38287525_B MDM2_Site68821573 IFNAR1.1_Site33355809 PDE8A_B PRKCB_Site23920921_L IFNAR1.1_Site33355840_E MDM2_WX IFNAR1.1_Site33355817 KCNJ15_Site38287589_E IFNAR1.1_Site33355838_P KCNJ15_Site38287590 PRKCB_Site23920884_D KCNJ15_Site38287530_D PRKCB_IJK KCNJ15_Site38287591 PRKCB_Site23920889_F PRKCB_Site23920844_B PRKCB_DJ LYN_AC IFNAR1.1_Site33355798 PRKCB_DHIJK GAB2_BCDE IFNAR1.1_Site33355801_N CAMK1D_A LYN_AG GAB2_Site78331030 CAMK1D_Site12378440 IFNAR1.1_Site33355793_G MDM2_KSX IFNAR1.1_Site33355839_L IFNAR1.1_Site33355789_A GAB2_ABDE PRKCB_DJM KCNJ15_ACE MDM2_ASV MDM2_Site68821576_V LYN_A IFNAR1.1_Site33355762_F LYN_Site56012491 PRKCB_Site23920927_M GAB2_EJ PRKCB_I LYN_Site56012490 CAMK1D_Site12378387 IFNAR1.1_Site33355807_B PRKCB_Site23920952 KCNJ15_ACD GAB2_DE MDM2_STX GAB2_Site78331008_D MDM2_Site68821567 MDM2_Site68821612_A | C79 | 118 | 0.889 | [ 0.857 ; 0.92 ] | -0.0579 | 81.27 | 87.41 | 77.99 | 92.07 | 67.93 | N= 411 [ 143 (1); 268 (2)] |

| | | | | | | | | | | | | |
|---|---|---|---|---|---|---|---|---|---|---|---|---|
| 5 | MDM2_Site68821586_W MDM2_SWX PRKCB_Site23920910_J MDM2_W MDM2_Site68821643 PRKCB_DIJK MDM2_SVW CAMK1D_E MDM2_SW PRKCB_B GAB2_Site78331054 PRKCB_Site23920911 PRKCB_Site23920919_K MDM2_T PRKCB_J KCNJ15_CE MDM2_Site68821616_X CAMK1D_AE KCNJ15_C PRKCB_JK GAB2_CE PDE8A_Site85096687_B KCNJ15_Site38287529_C PRKCB_Site23920902_I PRKCB_DIJ PRKCB_Site23920893_G KCNJ15_Site38287586 KCNJ15_Site38287525_B MDM2_Site68821573 IFNAR1.1_Site33355809 PDE8A_B PRKCB_Site23920921_L IFNAR1.1_Site33355840_E MDM2_WX IFNAR1.1_Site33355817 KCNJ15_Site38287589_E IFNAR1.1_Site33355838_P KCNJ15_Site38287590 PRKCB_Site23920884_D KCNJ15_Site38287530_D PRKCB_IJK KCNJ15_Site38287591 PRKCB_Site23920889_F PRKCB_Site23920844_B PRKCB_DJ LYN_AC IFNAR1.1_Site33355798 PRKCB_DHIJK GAB2_BCDE IFNAR1.1_Site33355801_N CAMK1D_A LYN_AG GAB2_Site78331030 CAMK1D_Site12378440 IFNAR1.1_Site33355793_G MDM2_KSX IFNAR1.1_Site33355839_L IFNAR1.1_Site33355789_A GAB2_ABDE KCNJ15_ACE MDM2_ASV MDM2_Site68821576_V LYN_A IFNAR1.1_Site33355762_F MDM2_Site68821602 LYN_Site56012491 PRKCB_Site23920927_M KCNJ15_Site38287526_A GAB2_EJ PRKCB_I LYN_Site56012490 CAMK1D_Site12378387 PRKCB_Site23920952 KCNJ15_ACD GAB2_DE MDM2_STX GAB2_Site78331008_D MDM2_Site68821567 MDM2_Site68821612_A | C79 | 218 | 0.887 | [ 0.855 ; 0.918 ] | -0.3527 | 83.7 | 78.32 | 86.57 | 88.21 | 75.68 | N= 411 [ 143 (1); 268 (2)] |
| 6 | MDM2_Site68821586_W MDM2_SWX PRKCB_Site23920910_J MDM2_W MDM2_Site68821643 PRKCB_DIJK MDM2_SVW CAMK1D_E MDM2_SW PRKCB_B GAB2_Site78331054 PRKCB_Site23920911 PRKCB_Site23920919_K MDM2_T PRKCB_J KCNJ15_CE MDM2_Site68821616_X CAMK1D_AE KCNJ15_C PRKCB_JK GAB2_CE PDE8A_Site85096687_B KCNJ15_Site38287529_C | C79 | 1152 | 0.889 | [ 0.858 ; 0.92 ] | -0.2397 | 82 | 82.52 | 81.72 | 89.75 | 70.66 | N= 411 [ 143 (1); 268 (2)] |

| 7 | | | C53 | #### | 0.825 | [ 0.783 ; 0.867 ] | -0.1737 | 80.29 | 76.92 | 76.87 | 86.19 | 63.95 | N= 411 [143 (1); 268 (2)] |

Feature set (column 1):

PRKCB_Site23920902_I PRKCB_DIJ
PRKCB_Site23920893_G KCNJ15_Site38287586
KCNJ15_Site38287525_B MDM2_Site68821573
IFNAR1.1_Site33355809 PDE8A_B
PRKCB_Site23920921_L MDM2_WX
IFNAR1.1_Site33355817 KCNJ15_Site38287589_E
IFNAR1.1_Site33355838_P KCNJ15_Site38287590
PRKCB_Site23920884_D KCNJ15_Site38287530_D
PRKCB_IJK KCNJ15_Site38287591
PRKCB_Site23920889_F PRKCB_Site23920844_B
PRKCB_DJ LYN_AC IFNAR1.1_Site33355798
PRKCB_DHIJK GAB2_BCDE
IFNAR1.1_Site33355801_N CAMK1D_A LYN_AG
GAB2_Site78331030 CAMK1D_Site12378440
IFNAR1.1_Site33355793_G MDM2_KSX
IFNAR1.1_Site33355839_L GAB2_ABDE PRKCB_DJM
KCNJ15_ACE MDM2_ASV MDM2_Site68821576_V
LYN_A IFNAR1.1_Site33355762_F
MDM2_Site68821602 LYN_Site56012491
PRKCB_Site23920927_M KCNJ15_Site38287526_A
GAB2_EJ PRKCB_I LYN_Site56012490
CAMK1D_Site12378387 IFNAR1.1_Site33355807_B
PRKCB_Site23920952 KCNJ15_ACD GAB2_DE
MDM2_STX GAB2_Site78331008_D
MDM2_Site68821567 MDM2_Site68821612_A

Feature set (column 2):

MDM2_Site68821586_W MDM2_SWX
PRKCB_Site23920910_J MDM2_W
MDM2_Site68821643 PRKCB_DIJK MDM2_SVW
MDM2_SW PRKCB_B GAB2_Site78331054
PRKCB_Site23920911 PRKCB_Site23920919_K
MDM2_T PRKCB_J KCNJ15_CE
MDM2_Site68821616_X CAMK1D_AE KCNJ15_C
PRKCB_JK PDE8A_Site85096687_B
KCNJ15_Site38287529_C PRKCB_Site23920902_I
PRKCB_DIJ KCNJ15_Site38287586
KCNJ15_Site38287525_B MDM2_Site68821573
IFNAR1.1_Site33355809 PDE8A_B
PRKCB_Site23920921_L IFNAR1.1_Site33355840_E
MDM2_WX IFNAR1.1_Site33355817
KCNJ15_Site38287589_E IFNAR1.1_Site33355838_P
KCNJ15_Site38287590 PRKCB_Site23920884_D
KCNJ15_Site38287530_D PRKCB_IJK
KCNJ15_Site38287591 PRKCB_Site23920889_F

| # | Sequence | | | | | | | | | | |
|---|---|---|---|---|---|---|---|---|---|---|---|
|  | PRKCB_Site23920844_B PRKCB_DJ LYN_AC IFNAR1.1_Site33355798 PRKCB_DHIJK GAB2_BCDE IFNAR1.1_Site33355801_N CAMK1D_A LYN_AG GAB2_Site78331030 CAMK1D_Site12378440 IFNAR1.1_Site33355793_G MDM2_KSX | | | | | | | | | | |
| 8 | MDM2_Site68821586_W MDM2_SWX PRKCB_Site23920910_J MDM2_W MDM2_Site68821643 PRKCB_DIJK MDM2_SVW CAMK1D_E MDM2_SW PRKCB_B GAB2_Site78331054 PRKCB_Site23920911 PRKCB_Site23920919_K MDM2_T PRKCB_J KCNJ15_CE MDM2_Site68821616_X CAMK1D_AE KCNJ15_C PRKCB_JK PDE8A_Site85096687_B KCNJ15_Site38287529_C PRKCB_Site23920902_I PRKCB_DIJ KCNJ15_Site38287586 KCNJ15_Site38287525_B MDM2_Site68821573 IFNAR1.1_Site33355809 PDE8A_B PRKCB_Site23920921_L IFNAR1.1_Site33355840_E MDM2_WX IFNAR1.1_Site33355817 KCNJ15_Site38287589_E IFNAR1.1_Site33355838_P KCNJ15_Site38287590 PRKCB_Site23920884_D KCNJ15_Site38287530_D PRKCB_IJK PRKCB_Site23920889_F PRKCB_Site23920844_B PRKCB_DJ LYN_AC IFNAR1.1_Site33355798 PRKCB_DHIJK GAB2_BCDE IFNAR1.1_Site33355801_N CAMK1D_A LYN_AG GAB2_Site78331030 CAMK1D_Site12378440 IFNAR1.1_Site33355793_G MDM2_KSX | C53 | 6995 | 0.824 | [ 0.782 ; 0.865 ] | -0.2023 | 79.32 | 76.22 | 80.97 | 86.45 | 68.12 | N= 411 [ 143 (1); 268 (2)] |
| 9 | MDM2_SWX PRKCB_Site23920910_J MDM2_W PRKCB_DIJK MDM2_SVW CAMK1D_E MDM2_SW PRKCB_B GAB2_Site78331054 PRKCB_Site23920911 PRKCB_Site23920919_K MDM2_T PRKCB_J KCNJ15_CE MDM2_Site68821616_X CAMK1D_AE KCNJ15_C PRKCB_JK | C18 | 187 | 0.752 | [ 0.704 ; 0.8 ] | -0.0589 | 71.1 | 71.3 | 70.9 | 82.3 | 56.7 | N= 411 [ 143 (1); 268 (2)] |
| 10 | MDM2_Site68821586_W MDM2_SWX PRKCB_Site23920910_J MDM2_Site68821643 PRKCB_DIJK MDM2_SVW CAMK1D_E MDM2_SW GAB2_Site78331054 PRKCB_Site23920911 PRKCB_Site23920919_K MDM2_T PRKCB_J KCNJ15_CE MDM2_Site68821616_X CAMK1D_AE KCNJ15_C | C17 | 606 | 0.751 | [ 0.702 ; 0.799 ] | -0.0981 | 71.78 | 70.63 | 72.39 | 82.2 | 57.71 | N= 411 [ 143 (1); 268 (2)] |

The panel of biomarkers analyzed above thus gave excellent diagnostic performances on to diagnose DEP the whole population of the validation cohort (411 subjects), suggesting that our panel of biomarkers could indeed have clinical applications in the diagnosis of DEP.

We next sought to investigate whether the diagnostic performances of our assay could be further increased by taking into account sex-specific differences. Indeed, the existence of sex-specific transcriptional signatures has been suggested in DEP. It has previously been shown that the brain transcriptional profile of DEP differs greatly by sex, suggesting that the molecular mechanisms underlying DEP could differ between women and men[42]. Modifications in RNA editing were thus analyzed separately in the male and the female population of the validation cohort. When sex-specificity was introduced in the analysis, a significant improvement of diagnostic performances was observed. Noteworthy, the female validation cohort (n=277) included more subjects than the male validation cohort (n=134), as this is reflecting the proportion of males and females suffering DEP in the general population worldwide. Indeed, in the female population of the validation cohort (n=277, 90 healthy controls and 187 DEP patients), when we combined 11 RNA editing variants, representing 4 different BMKS, were combined, the AUC was 0.784 (Sp 71.1%; Se 78.6%, Figure 7A). The equation used in this case is the following:

Z= - ( -0.000533736120198011 )* MDM2_Site68821586_W- (0.211359639047036)* MDM2_W - ( 0.146264452332436 )* MDM2_SWX - ( 0.160086235452429 )* GAB2_Site78331054 - ( 0.090792634422732 )* MDM2_SW + ( 0.117672779615208 )* MDM2_T + ( 0.209398709260439 )* CAMK1D_AE + ( 0.115253600910406 )* PRKCB_J - ( 0.193553830436994 )* CAMK1D_BC + ( 0.0404496406797322 )* CAMK1D_E + ( 0.0771184775348071 )* PRKCB_Site23920910_J

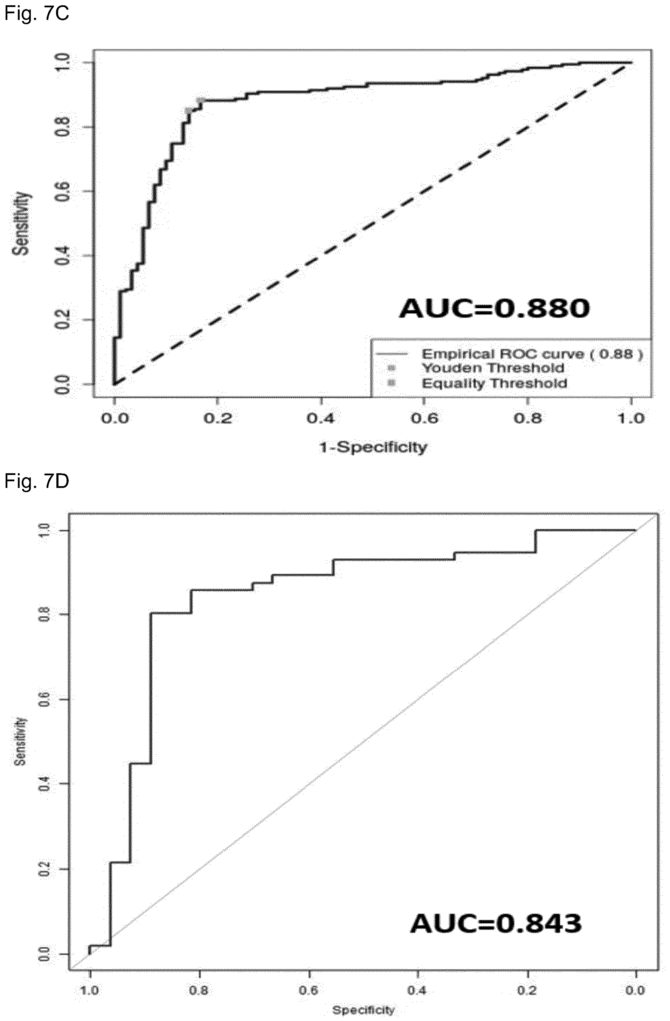

When the number of biomarkers was increased to 6 different targets and included 30 RNA editing variants, the AUC rose to 0.835 (Sp 80.0%; Se 83.4%, Figure 7B). Moreover, when we used the 8 biomarkers included in the multiplex and combined 49 differentially edited sites, the AUC reached to 0.880, with both specificity and sensitivity above 80% (Sp 83.3%; Se 88.2%, Figure 7C).

Using machine-learning approach with both mROC and RandomForest (see material and methods), we randomly separated the sex-specific populations into a train- and a test set. When randomly splitting the female population into a train set (75% of the whole population) and a test set (25% of the whole population) and applying the above-mentioned methods, we obtained an AUC of 0.843 in the test set with the mROC method (Sp 88.9%; Se 80.4%, Figure 7D) and an AUC of 0.895 with the MultDS RandomForest method (Sp 86%; Se 80%, Figure 7E). Altogether, the data obtained by two different machine-learning methods on the female validation cohort confirmed the excellent diagnostic performances of our panel of biomarkers.

The list of combinations tested in the female validation cohort (n=277), is presented in Table 16. All combinations were statistically significant between control and cases with a p value <$10^{-4}$.

**Table 16:** Diagnostic performances obtained with patients of the female population of the validation cohort analyzed by multiplex sequencing of 8 targets.

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | MDM2_Site68821586_W MDM2_W MDM2 _SWX GAB2_Site78331054 MDM2_SW MDM2_T CAMK1D_AE PRKCB_J CAMK1D_BC CAMK1D_E MDM2_Site68821612_A MDM2Site68821576_V MDM2_Site68821643 PRKCB_ Site23920910_J MDM2_Site68821573 PRKCB_ DJ MDM2_Site68821616_X CAMK1D_AF PRKCB_DHIJK MDM2_Site68821570 MDM2_Site68821602 MDM2_WX MDM2_SVW MDM2_SVWX CAMK1D_ Site12378375_G PRKCB_I CAMK1D_A PDE8A_ Site85096687_B CAMK1D_Site12378321_C PRKCB_Site23920952 MDM2_X LYN_A CAMK1D_ABG PRKCB_JK CAMK1D_FS GAB2_Site78331030 MDM2_K KCNJ15_C GAB2_IJ PRKCB_ Site23920921_L PRKCB_IJK IFNAR1.1_Site33355809 MDM2_Site68821572_T GAB2_BDEHJ GAB2_H | C50 | 443 | **0.883** | [ 0.841 ; 0.925] | -0.2642 | 85.6 | 84.4 | 86.1 | 92.0 | 74.5 | N= 277 [90 (1); 187 (2)] |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | PRKCB_AD PDE8A_B KCNJ15_CD GAB2_BCDJ IFNAR1.1_Site33355840_E | | | | | | | | | | | |
| 2 | MDM2_Site68821586_W MDM2_W MDM2_SWX GAB2_Site78331054 MDM2_SW MDM2_T CAMK1D_AE PRKCB_J CAMK1D_BC CAMK1D_E MDM2_Site68821612_A MDM2 _Site68821576_V MDM2_Site68821643 PRKCB_ Site23920910_J MDM2_Site68821573 PRKCB_ DJ MDM2_Site68821616_X CAMK1D_AF PRKCB DHIJK MDM2_Site68821570 MDM2_Site68821602 MDM2_WX MDM2_SVW MDM2_SVWX CAMK1D_ Site12378375_G PRKCB_I CAMK1D_A PDE8A_ Site85096687_B CAMK1D_Site12378321_C PRKCB_Site23920952 MDM2_X LYN_A CAMK1D _ABG PRKCB_JK CAMK1D_FS GAB2_Site78331030 MDM2_K KCNJ15_C GAB2_IJ PRKCB_ Site23920921_L | C50 | 440 | 0.880 | [ 0.837 ; 0.923 ] | -0.2692 | 85.2 | 85.6 | 85.0 | 92.4 | 73.3 | N= 277 [90 (1); 187 (2)] |

EP 4 055 190 B1

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
|  | PRKCB_IJK IFNAR1.1Site33355809 MDM2_Site68821572_T GAB2_BEJ GAB2_BDEHJ GAB2_H PDE8A_B KCNJ15_CD GAB2_BCDJ IFNAR1.1_Site33355840_E |  |  |  |  |  |  |  |  |  |  |  |
| 3 | MDM2_Site68821586_W MDM2_W MDM2_SW MDM2_T CAMK1D _AE PRKCB_J CAMK1D_BC CAMK1D_E MDM2_Site68821612_A MDM2_Site68821576_V PRKCB_Site23920910_J MDM2_Site68821573 PRKCB_DJ MDM2_Site68821616_X CAMK1D_AF PRKCB _ DHIJK MDM2_Site68821570 MDM2_Site68821602 PRKCB _ DIJK MDM2_WX MDM2_SVW MDM2_SVWX CAMK1D_Site12378375_G PRKCB_I CAMK1D_A PDE8A_Site85096687_B CAMK1D_Site12378321_C PRKCB_Site23920952 MDM2_X LYN_A CAMK1D_ABG PRKCB_JK | C49 | 19592 | 0.880 | [ 0.834 ; 0.925 ] | -0.3719 | 87.0 | 80.0 | 90.4 | 90.4 | 80.0 | N= 277 [90 (1); 187 (2)] |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | CAMK1D_FS GAB2_Site78331030 MDM2_K KCNJ15_C GAB2_IJ PRKCB_ Site23920921_L PRKCB IJK IFNAR1.1_Site33355809 MDM2_Site68821572_T GAB2_BEJ GAB2_BDEHJ GAB2_H PRKCB_AD PDE8A_B KCNJ15_CD GAB2_BCDJ IFNAR1.1 Site33355840 E | | | | | | | | | | | |
| 4 | MDM2_Site68821586_W MDM2_W MDM2_SWX MDM2_SW MDM2_T CAMK1D_ AE PRKCB_J CAMK1D_BC CAMK1D_E MDM2_Site68821612_A MDM2 _Site68821576_V MDM2_Site68821643 PRKCB_ Site23920910_J MDM2_Site68821573 MDM2_Site68821616_X CAMK1D_AF PRKCB_DHIJK MDM2_Site68821570 MDM2_Site68821602 PRKCB DIJK MDM2_WX MDM2_SVW MDM2_SVWX CAMK1D_ Site12378375_G PRKCB_I CAMK1D_A PDE8A_ Site85096687_B | C49 | 17939 | 0.880 | [ 0.835 ; 0.924 ] | -0.2916 | 86.6 | 83.3 | 88.2 | 91.7 | 77.3 | N= 277 [90 (1); 187 (2)] |

EP 4 055 190 B1

52

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | CAMK1D_Site12378321_C PRKCB_Site23920952 MDM2_X LYN_A CAMK1D _ABG PRKCB_JK CAMK1D_FS GAB2_Site78331030 MDM2_K KCNJ15_C PRKCB_ Site23920921_L PRKCB_IJK IFNAR 1.1_Site33355809 MDM2_Site68821572_T GAB2_BEJ GAB2_BDEHJ GAB2_H PRKCB_AD - PDE8A_B KCNJ15_CD GAB2_BCDJ IFNAR1.1 Site33355840 E | | | | | | | | | | | |
| | MDM2_Site68821586_W MDM2_W MDM2_SWX MDM2_SW MDM2_T CAMK1D_ AE PRKCB_J CAMK1D_BC MDM2_Site68821612_A MDM2_Site68821643 PRKCB_ Site23920910_J MDM2_Site68821573 PRKCB_ DJ MDM2_Site68821616_X CAMK1D_AF PRKCB DHIJK MDM2 _Site68821570 MDM2_Site68821602 PRKCB DIJK MDM2_WX MDM2_SVW MDM2_SVWX | | | | | | | | | | | |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 5 | CAMK1DG PRKCB_I CAMK1D_A<br>_Site12378375_ PDE8A_<br>Site85096687_B<br>CAMK1D_Site12378321_C<br>PRKCB_Site23920952<br>MDM2_X LYN_A CAMK1D<br>_ABG PRKCB_JK<br>CAMK1D_FS<br>GAB2_Site78331030 MDM2_K<br>KCNJ15_C GAB2_IJ PRKCB_<br>Site23920921_L<br>PRKCB IJK<br>IFNAR1.1Site33355809<br>MDM2_Site68821572_T<br>GAB2_BEJ<br>GAB2_BDEHJ GAB2_H<br>PRKCB_AD PDE8A_B<br>KCNJ15_CD GAB2_BCDJ<br>IFNAR1.1 Site33355840 E | C49 | 18198 | 0.879 | [ 0.835 ; 0.924 ] | -0.3089 | 86.6 | 82.2 | 88.8 | 91.2 | 77.9 | N= 277 [90 (1); 187 (2)] |
|  | MDM2_Site68821586_W<br>MDM2_SWX<br>GAB2_Site78331054<br>MDM2_SW MDM2_T<br>CAMK1D_AE PRKCB_J<br>CAMK1D_BC CAMK1D_E<br>MDM2_Site68821612_A<br>MDM2Site68821576_V<br>MDM2_Site68821643 PRKCB_<br>Site23920910_J<br>MDM2_Site68821573 PRKCB_<br>DJ |  |  |  |  |  |  |  |  |  |  |  |

EP 4 055 190 B1

54

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 6 | MDM2_Site68821616_X CAMK1D_AF PRKCB _ DHIJK MDM2 _Site68821570 MDM2_Site68821602 PRKCB _ DIJK MDM2_WX MDM2_SVW PRKCB_I CAMK1D_A PDE8A_Site85096687_B CAMK1D_Site12378321_C PRKCB_Site23920952 MDM2_X LYN_A | C31 | 4531 | **0.835** | [ 0.782 ; 0.887 ] | -0.2749 | 82.3 | 80.0 | 83.4 | 89.7 | 69.9 | N= 277 [90 (1); 187 (2)] |
| 7 | MDM2_Site68821586_W MDM2_SWX GAB2_Site78331054 MDM2_SW MDM2_T CAMK1D_AE PRKCB_J CAMK1D_BC CAMK1D_E MDM2_Site68821612_A MDM2 _Site68821576_V MDM2_Site68821643 PRKCB_ Site23920910_J MDM2_Site68821573 PRKCB_ DJ MDM2_Site68821616_X CAMK1D_AF PRKCB DHIJK MDM2 _Site68821570 PRKCB DIJK MDM2_WX MDM2_SVW CAMK1D Site12378375 G PRKCB I CAMK1D A PDE8A_Site85096687_B | C31 | 4570 | 0.834 | [ 0.781 ; 0.886 ] | -0.279 | 82.3 | 80.0 | 83.4 | 89.7 | 69.9 | N= 277 [90 (1); 187 (2)] |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
|  | CAMK1D_Site12378321_C PRKCB_Site23920952 MDM2_X LYN_A |  |  |  |  |  |  |  |  |  |  |  |
| 8 | MDM2_Site68821586_W MDM2_W MDM2_SWX GAB2_Site78331054 MDM2_SW MDM2_T CAMK1D_AE PRKCB_J CAMK1D_BC CAMK1D_E MDM2_Site68821612_A MDM2_Site68821576_V PRKCB_Site23920910_J MDM2_Site68821573 PRKCB DJ MDM2 Site68821616 X PRKCB_DHIJK MDM2_Site68821570 MDM2_Site68821602 PRKCB -DIJK MDM2_WX MDM2_SVW CAMK1D_Site12378375_G PRKCB_I CAMK1D_A PDE8A_Site85096687_B CAMK1D_Site12378321_C PRKCB_Site23920952 MDM2_X LYN_A | C31 | 1254 | 0.833 | [ 0.78 ; 0.886 ] | -0.1426 | 80.9 | 81.1 | 80.8 | 89.9 | 67.0 | N= 277 [90 (1); 187 (2)] |

EP 4 055 190 B1

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 9 | MDM2 _Site68821586 _W MDM2_W GAB2_Site78331054 MDM2_T CAMK1D_AE PRKCB_J CAMK1D_BC CAMK1D_E MDM2_Site68821612_A MDM2Site68821576_V MDM2 Site68821643 | C11 | 202 | 0.781 | [ 0.723 ; 0.838 ] | -0.0475 | 74.7 | 78.9 | 72.7 | 87.7 | 58.2 | N= 277 [90 (1); 187 (2)] |
| 10 | MDM2_W MDM2_SWX GAB2_Site78331054 MDM2_T CAMK1D_AE PRKCB_J D_BC CAMK1 CAMK1D_E MDM2_Site68821612_A MDM2_Site68821643 PRKCB _Site23920910_J | C11 | 325 | 0.787 | [ 0.731 ; 0.842 ] | -0.0183 | 73.7 | 80.0 | 70.6 | 88.0 | 56.7 | N= 277 [90 (1); 187 (2)] |
| 11 | MDM2_Site68821586 _W MDM2_W MDM2_SWX GAB2_Site78331054 MDM2_SW MDM2_T CAMK1D_AE PRKCB_J CAMK1D_BC CAMK1D_E PRKCB_Site23920910_J | C11 | 4 | 0.784 | [ 0.729 ; 0.84 ] | -0.1626 | 76.2 | 71.1 | 78.6 | 85.0 | 61.5 | N= 277 [90 (1); 187 (2)] |

The performances of the male population were even better. Indeed, when we analyzed separately the male population of the validation cohort (n=134, 53 healthy controls and 81 DEP patients) and included 8 editing variants in 6 BMKs, we obtained an AUC of 0.831 (Sp 83.0%; Se 75.3%, Figure 8A). In this case, the equation used is the following:

$$Z = - ( 0.238400918804649 )* GAB2\_CH - ( 0.230156347297484 )* CAMK1D\_DS - ( 0.19154950723228 )* KCNJ15\_Site38287525\_B - ( 0.21914392389275 )* GAB2\_CE - ( 0.155783908162178 )* LYN\_Site56012491 - ( 0.268738279594405 )* MDM2\_SVW - ( 0.107036976834934 )* MDM2\_Site68821643 + ( 0.400819265369961 )* PRKCB\_Site23920893\_G.$$

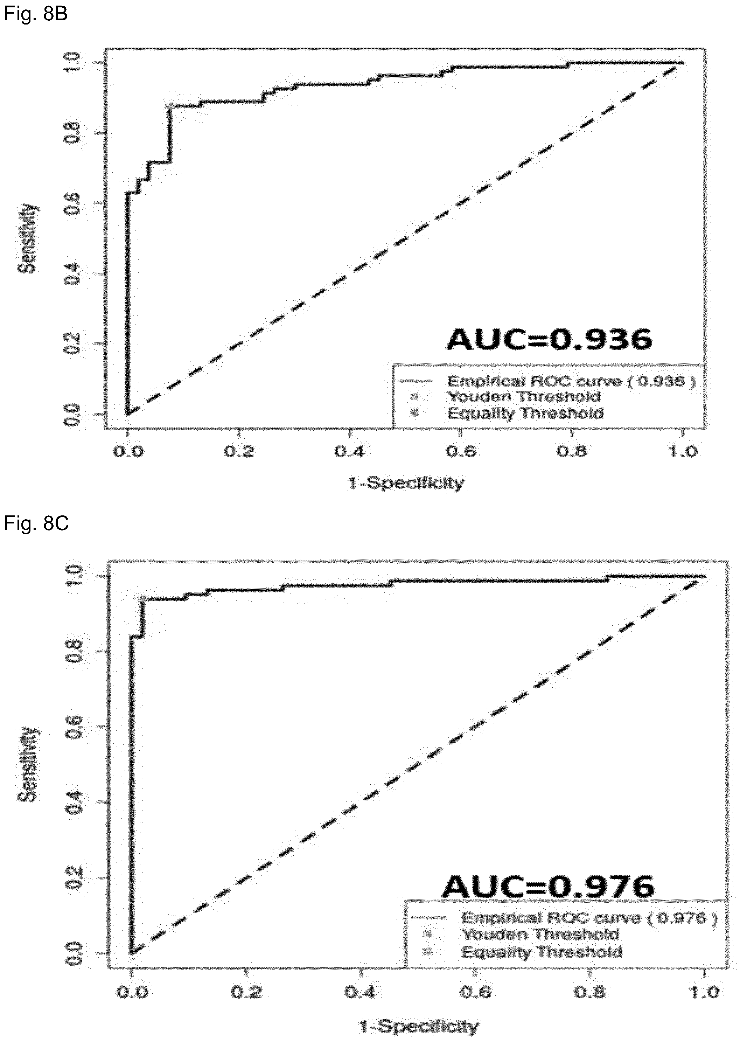

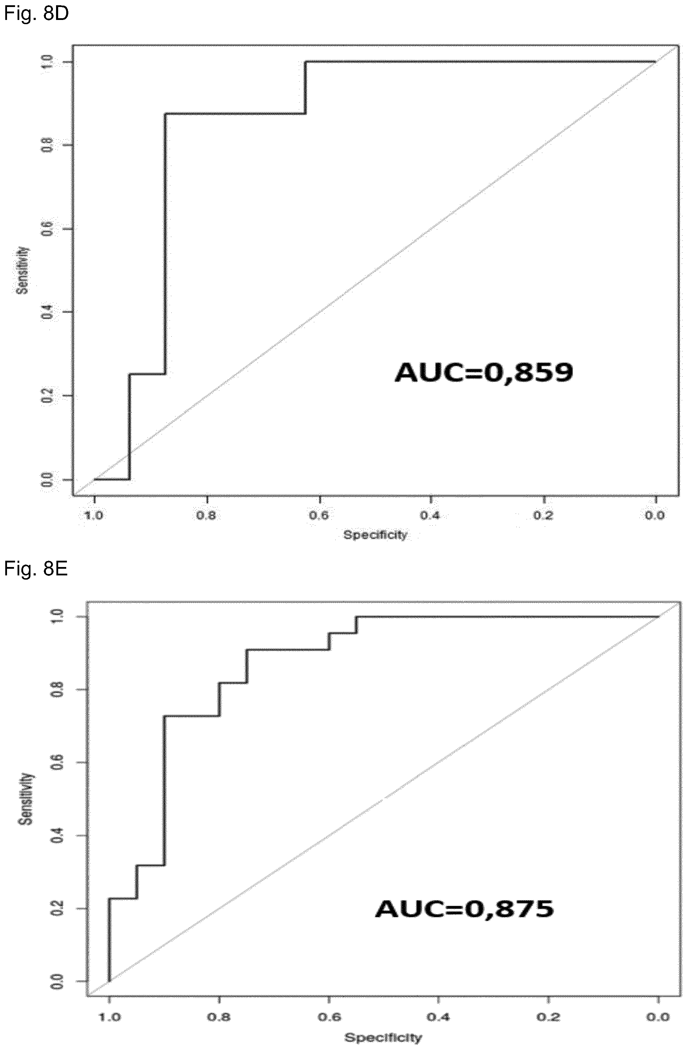

With 7 biomarkers, including 39 RNA editing variants, the AUC was above 0.9 (AUC 0.936, Spe 92.5%, Se 87.7%, figure 8B). When the 8 biomarkers included in the panel were combined, with 68 editing variants in these BMKS, we obtained an outstanding AUC of 0.976 (Spe 98.1 %, Se 93.8%, Figure 8C). Using machine-learning, we randomly split the male population into a training (70% of the whole population) and a test set (30% of the whole population) and obtained an AUC with mROC was 0.859 (Sp 87.5%, Se 87.5%, Figure 8D), while the RandomForest method led to an AUC of 0.875 (Sp 82%, Se 80%, Figure 8E).

The list of combinations tested in the male validation cohort (n=134), is presented in Table 8. All combinations were statistically significant between control and cases with a p value $<10^{-4}$.

**Table 8:** Diagnostic performances obtained with patients of the male population (n=134) of the validation cohort analyzed by multiplex sequencing of 8 targets.

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | GAB2_CH CAMK1D_DS KCNJ15_Site38287525_B GAB2_CE LYN_Site56012491 LYN_AC KCNJ15_Site38287530_D MDM2_SVW MDM2_Site68821643 KCNJ15_ACE PRKCB_Site23920893_G KCNJ15_Site38287529_C CAMK1D_BF KCNJ15_CE PDE8A_E KCNJ15_ACD MDM2_KST GAB2_JK PRKCB_JM PRKCB_ADJK CAMK1D_D GAB2_Site78330939_H KCNJ15_Site38287526 _A CAMK1D_R GAB2_ABDE KCNJ15_C KCNJ15_Site38287531 CAMK1D_AF GAB2_H GAB2_DH GAB2_Site78331054 LYN_Site56012495 MDM2_KSX LYN_Site56012468_C MDM2_BG LYN_Site56012521 LYN_Site56012556_F KCNJ15_AC PRKCB_DIJK GAB2 _Site78331011_C KCNJ15_ABC PRKCB_DJM PDE8A_Site85096729_F CAMK1D_F GAB2_DEHJ | C68 | 1992 | 0.976 | [ 0.952 ; 1 ] | -0.7316 | 95.5 | 98.1 | 93.8 | 98.7 | 91.2 | N= 134 [53 (1); 81 (2)] |

(continued)

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | KCNJ15_Site38287591 LYN_Site56012567 PRKCB_Site23920911 LYN_Site56012553_A MDM2_SWX KCNJ15_Site38287586 CAMK1D_FS GAB2_DK KCNJ15_AB PRKCB_Site23920889_F PRKCB_AJ PRKCB_Site23920919_K LYN_Site56012469_D PRKCB_Site23920910_J IFNAR1.1_AC LYN_Site56012446 PRKCB_Site23920884_D IFNAR1.1_C GAB2_BCDE LYN_Site56012568 PRKCB_IJK IFNAR1.1_AB PDE8A_B | | | | | | | | | | | |
| | GAB2_CH CAMK1D_DS KCNJ15_Site38287525_B LYN_Site56012491 LYN_AC LYN_AF KCNJ15_Site38287530_D MDM2_SVW MDM2_Site68821643 KCNJ15_ACE PRKCB_Site23920893_G KCNJ15_Site38287529_C CAMK1D_BF KCNJ15_CE PDE8A_E KCNJ15_ACD MDM2_KST | | | | | | | | | | | |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 2 | GAB2_JK PRKCB_JM PRKCB _ADJK CAMK1D_D GAB2_Site78330939_H KCNJ15_Site38287526_A CAMK1D_R GAB2_ABDE KCNJ15_C KCNJ15_Site38287531 CAMK1D_AF GAB2_H GAB2_DH LYN_Site56012495 MDM2_KSX LYN_Site56012468 _C MDM2_BG LYN_Site56012521 LYN_ Site56012556_F KCNJ15_AC PRKCB DIJK GAB2 _Site78331011 C KCNJ15_ABC PRKCB_DJM PDE8A_Site85096729_F CAMK1 D_F GAB2_DEHJ KCNJ15_Site38287591 LYN_ Site56012567 PRKCB_Site23920911 LYN_ Site56012553_A MDM2_SWX KCNJ15_Site38287586 CAMK1D_FS GAB2_DK KCNJ15_AB PRKCB_Site23920889_F PRKCB_AJ PRKCB_ Site23920919_K LYN_Site56012469_D PRKCB_ Site23920910_J IFNAR1.1_AC LYN_ Site56012446 | C67 | 46501 | 0.975 | [ 0.953 ; 0.997 ] | -0.679 | 95.5 | 100.0 | 92.6 | 100.0 | 89.8 | N= 134 [53 (1); 81 (2)] |

(continued)

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | PRKCB_Site23920884_D IFNAR1.1_C GAB2_BCDE LYN_ Site56012568 PRKCB_IJK IFNAR1.1 AB PDE8A B | | | | | | | | | | | |
| 3 | GAB2_CH CAMK1D_DS KCNJ15_Site38287525_B GAB2_CE LYN_Site56012491 LYN_AC KCNJ15_Site38287530_D MDM2_SVW MDM2_Site68821643 KCNJ15_ACE PRKCB_Site23920893_G CAMK1D_BF KCNJ15_CE PDE8A_E KCNJ15_ACD MDM2_KST GAB2_JK PRKCB_JM PRKCB _ADJK CAMK1D_D GAB2_Site78330939_H KCNJ15 _Site38287526 A CAMK1D R GAB2_ABDE KCNJ15_C KCNJ15_Site38287531 CAMK1D_AF GAB2_H GAB2_DH GAB2_Site78331054 LYN_Site56012495 MDM2_KSX LYN_Site56012468_C MDM2_BG LYN_Site56012521 LYN_ Site56012556_F | C67 | 41346 | 0.977 | [ 0.952 ; 1 ] | -0.7686 | 95.5 | 98.1 | 93.8 | 98.7 | 91.2 | N= 134 [53 (1); 81 (2)] |

(continued)

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | KCNJ15_AC PRKCB _DIJK | | | | | | | | | | | |
| | GAB2_Site78331011_C | | | | | | | | | | | |
| | KCNJ15 ABC PRKCB DJM | | | | | | | | | | | |
| | PDE8A_Site85096729_F | | | | | | | | | | | |
| | CAMK1D_F GAB2_DEHJ | | | | | | | | | | | |
| | KCNJ15_Site38287591 LYN_ Site56012567 | | | | | | | | | | | |
| | PRKCB_Site23920911 LYN_ Site56012553_A | | | | | | | | | | | |
| | MDM2_SWX | | | | | | | | | | | |
| | KCNJ15_Site38287586 | | | | | | | | | | | |
| | CAMK1D_FS | | | | | | | | | | | |
| | GAB2_DK KCNJ15_AB | | | | | | | | | | | |
| | PRKCB_Site23920889_F | | | | | | | | | | | |
| | PRKCB_AJ PRKCB_ Site23920919_K | | | | | | | | | | | |
| | LYN_Site56012469_D PRKCB_ Site23920910_J | | | | | | | | | | | |
| | IFNAR1.1_AC LYN_ Site56012446 | | | | | | | | | | | |
| | PRKCB_Site23920884_D IFNAR1.1_C | | | | | | | | | | | |
| | GAB2_BCDE LYN_ Site56012568 PRKCB_IJK | | | | | | | | | | | |
| | IFNAR1.1 AB PDE8A B | | | | | | | | | | | |
| | GAB2_CH CAMK1D_DS KCNJ15_Site38287525_B | | | | | | | | | | | |
| | GAB2_CE LYN_Site56012491 LYN_AC LYN_AF | | | | | | | | | | | |
| | KCNJ15_Site38287530_D MDM2_SVW | | | | | | | | | | | |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 4 | MDM2_Site68821643 KCNJ15_ACE<br><br>PRKCB_Site23920893_G KCNJ15_Site38287529_C CAMK1 D_BF KCNJ15_CE PDE8A_E KCNJ15_ACD MDM2_KST GAB2_JK PRKCB_JM PRKCB _ADJK CAMK1D_D GAB2_Site78330939_H KCNJ15_Site38287526 _A CAMK1D_R GAB2_ABDE KCNJ15_C KCNJ15_Site38287531 CAMK1D_AF GAB2_H GAB2_DH PRKCB_B GAB2_Site78331054 LYN_Site56012495 MDM2_KSX LYN_Site56012468_C MDM2_BG LYN_Site56012556_F KCNJ15_AC PRKCB_DIJK GAB2_Site78331011_C KCNJ15_ABC PRKCB_DJM PDE8A_ Site85096729_F CAMK1D_F GAB2_DEHJ KCNJ15_Site38287591 LYN_Site56012567 PRKCB_ Site23920911 LYN_Site56012553_A MDM2_SWX | C67 | 5058 | 0.980 | [ 0.958 ; 1 ] | -1.2498 | 95.5 | 96.2 | 95.1 | 97.5 | 92.7 | N= 134 [53 (1); 81 (2)] |

EP 4 055 190 B1

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
|  | KCNJ15_Site38287586 CAMK1D_FS GAB2_DK PRKCB_Site23920889_F PRKCB_AJ PRKCB_Site23920919_K LYN_Site56012469_D PRKCB_Site23920910_J IFNAR1.1_AC LYN_Site56012446 IFNAR1.1_C GAB2_BCDE LYN_Site56012568 PRKCB_IJK IFNAR1.1_AB PDE8A B |  |  |  |  |  |  |  |  |  |  |  |
| 5 | GAB2_CH CAMK1D_DS KCNJ15_Site38287525_B GAB2_CE LYN_Site56012491 LYN_AC LYN_AF KCNJ15_Site38287530_D MDM2_SVW MDM2_Site68821643 KCNJ15_ACE PRKCB_Site23920893_G KCNJ15_Site38287529_C CAMK1 D_BF KCNJ15_CE PDE8A_E KCNJ15_ACD GAB2_JK PRKCB_ADJK CAMK1D_D GAB2_Site78330939_H KCNJ15_Site38287526_A CAMK1D R GAB2_ABDE KCNJ15_C | C39 | 2261 | 0.936 | [ 0.898 ; 0.975 ] | -0.0614 | 88.1 | 100.0 | 80.3 | 100.0 | 76.8 | N= 134 [53 (1); 81 (2)] |

EP 4 055 190 B1

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
|  | KCNJ15_Site38287531 CAMK1D_AF GAB2_H GAB2_DH PRKCB_B GAB2_Site78331054 LYN_Site56012495 MDM2_KSX LYN_Site56012468_C LYN_Site56012521 KCNJ15_AC KCNJ15_ABC PRKCB_DJM PDE8A Site85096729 F |  |  |  |  |  |  |  |  |  |  |  |
| 6 | GAB2_CH CAMK1D_DS KCNJ15_Site38287525_B GAB2_CE LYN_Site56012491 LYN_AC LYN_AF KCNJ15_Site38287530_D MDM2_SVW MDM2_Site68821643 KCNJ15_ACE PRKCB_Site23920893_G KCNJ15_Site38287529_C KCNJ15_CE PDE8A_E KCNJ15_ACD MDM2_KST GAB2_JK<br><br>PRKCB_ADJK CAMK1 D_D<br><br>GAB2_Site78330939_H KCNJ15_Site38287526_A CAMK1D_R GAB2_ABDE KCNJ15_C KCNJ15_Site38287531 CAMK1D_AF GAB2_H | C39 | 3516 | 0.936 | [ 0.896 ; 0.975 ] | -0.3892 | 89.6 | 92.5 | 87.7 | 94.7 | 83.1 | N= 134 [53 (1); 81 (2)] |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | GAB2_DH PRKCB_B GAB2_Site78331054 MDM2_KSX LYN_Site56012468_C MDM2_BG LYN_Site56012521 KCNJ15_AC KCNJ15_ABC PRKCB DJM PDE8A Site85096729 F | | | | | | | | | | | |
| 7 | GAB2_CH CAMK1D_DS KCNJ15_Site38287525_B GAB2_CE LYN_Site56012491 LYN_AC LYN_AF KCNJ15_Site38287530_D MDM2_SVW MDM2_Site68821643 KCNJ15_ACE PRKCB_Site23920893_G KCNJ15_Site38287529_C KCNJ15_CE PDE8A_E KCNJ15_ACD MDM2_KST GAB2_JK PRKCB_JM PRKCB_ADJK CAMK1 D_D GAB2_Site78330939_H KCNJ15_Site38287526_A CAMK1D_R GAB2_ABDE KCNJ15_C KCNJ15_Site38287531 CAMK1D_AF GAB2_H GAB2 DH PRKCB B GAB2 Site78331054 LYN_Site56012495 MDM2_KSX | C39 | 3287 | 0.937 | [ 0.898 ; 0.975 ] | -0.4206 | 89.6 | 88.7 | 90.1 | 92.4 | 85.5 | N= 134 [53 (1); 81 (2)] |

(continued)

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
|  | LYN_Site56012468_C LYN_Site56012521 KCNJ15_AC KCNJ15_ABC PRKCB_DJM |  |  |  |  |  |  |  |  |  |  |  |
| 8 | GAB2_CH CAMK1D_DS KCNJ15_Site38287525_B GAB2_CE LYN_Site56012491 LYN_AC LYN_AF KCNJ15_Site38287530_D MDM2_SVW MDM2_Site68821643 KCNJ15_ACE PRKCB_Site23920893_G KCNJ15_Site38287529_C KCNJ15_CE PDE8A_E KCNJ15_ACD GAB2_JK PRKCB_JM MDM2_KST PRKCB_ADJKCAMK1D_D GAB2_Site78330939_H KCNJ15_Site38287526_A CAMK1D_R GAB2_ABDE KCNJ15_C CAMK1D_AF GAB2_H GAB2_DH PRKCB_B GAB2_Site78331054 LYN_Site56012495 MDM2_KSX MDM2_BG LYN_Site56012521 KCNJ15_AC KCNJ15_ABC PRKCB_DJM PDE8A Site85096729 F | C39 | 3374 | 0.943 | [0.908 ; 0.979] | -0.2029 | 88.1 | 96.2 | 82.7 | 97.1 | 78.5 | N= 134 [53 (1); 81 (2)] |

68

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 9 | GAB2_CH CAMK1D_DS KCNJ15_Site38287525_B GAB2_CE LYN_Site56012491 LYN_AC KCNJ15_Site38287530_D MDM2_SVW PRKCB Site23920893 G | C9 | 8 | 0.833 | [ 0.764 ; 0.901 ] | -0.1683 | 78.4 | 79.3 | 77.8 | 85.1 | 70.0 | N= 134 [53 (1); 81 (2)] |
| 10 | GAB2_CH CAMK1 D_DS GAB2_CE LYN_Site56012491 LYN_AC LYN_AF MDM2 Site68821643 PRKCB Site23920893 G | C8 | 62 | 0.806 | [ 0.731 ; 0.881 ] | -0.2557 | 79.9 | 71.7 | 85.2 | 82.1 | 76.0 | N= 134 [53 (1); 81 (2)] |
| 11 | GAB2_CH CAMK1D_DS KCNJ15_Site38287525_B GAB2_CE LYN_Site56012491 MDM2_SVW MDM2 Site68821643 PRKCB Site23920893 G | C8 | 20 | 0.831 | [ 0.763 ; 0.899 ] | -0.1107 | 78.4 | 83.0 | 75.3 | 87.1 | 68.8 | N= 134 [53 (1); 81 (2)] |

The data obtained with both methods thus gave excellent diagnostic performances for sex-specific diagnosis of DEP, with AUCs above 0.850, specificities and sensitivities above 80% for both females and males, indicative of a diagnostic assay that could be used into the clinic.

As we have identified RNA editing biomarkers for the diagnosis of DEP, we sought to investigate whether the non-edited isoforms of the validated targets could be useful to discriminate the control and patient populations and might be used in combination with RNA editing biomarkers. The AUC ROC for each non-edited target was then calculated, along with the significance for each non-edited isoform. Analysis of the non-edited isoforms of PRKCB, MDM2, IFNAR1 and PDE8A on the validation cohort (n=411 subjects) gave AUCs above 0.5; the non -edited isoforms of both PRKCB and MDM2 were significantly different between control and DEP (p<0.05, Table 9).

Table 9: Diagnostic performances obtained with non-edited isoforms on the validation cohort analyzed with multiplex sequencing;

| Target | p-value | AUC ROC | % Non-edited | Population |
|---|---|---|---|---|
| PRKCB non-edited | 0.0001 | 0.622 | 43.6 | N= 411 [ 143 (0) ; 268 (1)] |
| MDM2 non-edited | 0.0132 | 0.573 | 33.7 | N= 411 [ 143 (0) ; 268 (1)] |
| IFNAR1 non-edited | 0.0785 | 0.553 | 74.4 | N= 411 [ 143 (0) ; 268 (1)] |
| PDE8A non-edited | 0.0915 | 0.551 | 78.1 | N= 411 [ 143 (0) ; 268 (1)] |

In addition, when the non-edited isoforms were combined with one isoform of the same targets, we observed significant differences between healthy control and DEP patients (Table 19), suggesting that the non-edited isoforms could also be used in the diagnostic assay;

Table 19: Diagnostic performances obtained with non-edited isoforms combined with edited targets on the validation cohort analyzed with multiplex sequencing;

| Target | Ratio | p-value | AUC ROC | Population |
|---|---|---|---|---|
| **CAMK1D** | BGNS / non-edited | 0.0002 | 0.615 | N= 411 [ 143 (0) ; 268 (1)] |
| **CAMK1D** | ABCDEFRS / non-edited | 0.0002 | 0.613 | N= 411 [ 143 (0) ; 268 (1)] |
| **CAMK1D** | ABCEFGR / non-edited | 0.0007 | 0.599 | N= 411 [ 143 (0) ; 268 (1)] |
| **CAMK1D** | BCM / non-edited | 0.0008 | 0.599 | N= 411 [ 143 (0) ; 268 (1)] |
| **CAMK1D** | BEIRS / non-edited | 0.0009 | 0.599 | N= 411 [ 143 (0) ; 268 (1)] |
| **CAMK1D** | ABDG / non-edited | 0.0026 | 0.592 | N= 411 [ 143 (0) ; 268 (1)] |
| **GAB2** | AEFHI / non-edited | 0.0005 | 0.603 | N= 411 [ 143 (0) ; 268 (1)] |
| **IFNAR1** | ABCEFGILNP / non-edited | 0.001 | 0.601 | N= 411 [ 143 (0) ; 268 (1)] |
| **MDM2** | KOSX / non-edited | 0.0007 | 0.602 | N= 411 [ 143 (0) ; 268 (1)] |
| **MDM2** | AJSWX / non-edited | 0.0003 | 0.609 | N= 411 [ 143 (0) ; 268 (1)] |
| **MDM2** | OP / non-edited | 0.0007 | 0.602 | N= 411 [ 143 (0) ; 268 (1)] |
| **PRKCB** | BDFIJK / non-edited | 0.0006 | 0.601 | N= 411 [ 143 (0) ; 268 (1)] |
| **PRKCB** | DJP / non-edited | 0.0008 | 0.600 | N= 411 [ 143 (0) ; 268 (1)] |
| **PRKCB** | BN / non-edited | 0.0009 | 0.600 | N= 411 [ 143 (0) ; 268 (1)] |
| **PRKCB** | CDFIJK / non-edited | 0.0013 | 0.593 | N= 411 [ 143 (0) ; 268 (1)] |
| **PRKCB** | BJKM / non-edited | 0.0024 | 0.591 | N= 411 [ 143 (0) ; 268 (1)] |
| **PRKCB** | AGHIJKLM / non-edited | 0.0031 | 0.594 | N= 411 [ 143 (0) ; 268 (1)] |
| **PRKCB** | DJ / non-edited | 0.0003 | 0.612 | N= 411 [ 143 (0) ; 268 (1)] |

**Conclusion**

To our knowledge, this research is the first study to investigate RNA editing modifications to be used as blood biomarkers to diagnose mood disorders. We performed an editome analysis of DEP in patients recruited from the outpatients of the Department of Emergency Psychiatry and Post-Acute Care (CHRU of Montpellier). The presence of psychiatric diagnoses was confirmed by trained psychiatrists, who managed the Montgomery-Åsberg Depression Rating Scale (MADRS) in french and the 30-item Inventory of Depressive Symptomatology, Clinician Rated (IDS-C30) to score depression. We sought association with depression in the editome of patients following RNA-Seq study in a discovery cohort of 64 subjects. We identified 646 A-to-I editing variants, representing 366 genes, which were differentially edited between depressed patients and healthy controls. This gene set showed a strong enrichment for biological processes including multiple immune categories, thus confirming a strong immune component in the physiopathology of DEP[43]. The list of variants was then narrowed down to a subset of 14 genes (17 variants) by using much stringent criteria, and validated these biomarkers individually in a prioritization cohort of 86 individuals. These genes also showed a strong enrichment in genes related to regulation of immune system process. In addition, some were strongly related to pathways involved in neuronal system and neurotransmitter receptor-related pathways. Finally, we identified different combinations of a panel of 8 markers and validated this combination in a large cohort of 411 patients. Moreover, when sex specificity was introduced, and a machine-learning approach was used, the performances of the diagnostic assay were substantially increased. Our findings identified combinations of biomarkers with high diagnostic performances, suitable to be clinically useful to objectively diagnose depressive disorders from blood samples.

We previously identified alterations of the serotonin receptor 5HT2CR and PDE8A mRNA editing in brain samples of suicide decedents by capillary electrophoresis single-strand conformation 5 polymorphism (CE-SSCP)[5,44]. We recently demonstrated altered RNA editing in psychiatric patients with chronic hepatitis C virus (HCV) undergoing antiviral combination therapy with IFN-a and ribavirin, in whom treatment-emergent depression is a common complication[22]. In the latter study, measurements of editing levels were based on targeted next-generation sequencing (NGS), which provides both high throughput and sufficient per base depth to allow reliable quantification for all sites studied.

**References**

1. American Psychiatric Association. Diagnostic and Statistical Manual of Mental Disorders. 5th ed. Washington, DC. 2013.

2. Dunlop K, Talishinsky A, Liston C. Intrinsic Brain Network Biomarkers of Antidepressant Response: a Review. Current psychiatry reports. Aug 13 2019;21(9):87.

3. Jentsch MC, Van Buel EM, Bosker FJ, et al. Biomarker approaches in major depressive disorder evaluated in the context of current hypotheses. Biomarkers in medicine. 2015;9(3):277-297.

4. Smith KM, Renshaw PF, Bilello J. The diagnosis of depression: current and emerging methods. Comprehensive psychiatry. Jan 2013;54(1):1-6.

5. Chimienti F, Cavarec L, Vincent L, et al. Brain region-specific alterations of RNA editing in PDE8A mRNA in suicide decedents. Translational psychiatry. Feb 15 2019;9(1):91.

6. Dobin A, Gingeras TR. Optimizing RNA-Seq Mapping with STAR. Methods in molecular biology. 2016;1415:245-262.

7. DePristo MA, Banks E, Poplin R, et al. A framework for variation discovery and genotyping using next-generation DNA sequencing data. Nature genetics. May 2011;43(5):491-498.

8. John D, Weirick T, Dimmeler S, Uchida S. RNAEditor: easy detection of RNA editing events and the introduction of editing islands. Briefings in bioinformatics. Nov 1 2017;18(6):993-1001.

9. Yang H, Wang K. Genomic variant annotation and prioritization with ANNOVAR and wANNOVAR. Nature protocols. Oct 2015;10(10):1556-1566.

10. Tempel S. Using and understanding RepeatMasker. Methods in molecular biology. 2012;859:29-51.

11. Kasprzyk A, Keefe D, Smedley D, et al. EnsMart: a generic system for fast and flexible access to biological data. Genome research. Jan 2004; 14(1): 160-169.

12. Langmead B, Salzberg SL. Fast gapped-read alignment with Bowtie 2. Nature methods. Mar 4 2012;9(4):357-359.

13. Li H, Handsaker B, Wysoker A, et al. The Sequence Alignment/Map format and SAMtools. Bioinformatics. Aug 15 2009;25(16):2078-2079.

14. Li H. A statistical framework for SNP calling, mutation discovery, association mapping and population genetical parameter estimation from sequencing data. Bioinformatics. Nov 1 2011;27(21):2987-2993.

15. Gentleman RC, Carey VJ, Bates DM, et al. Bioconductor: open software development for computational biology and bioinformatics. Genome biology. 2004;5(10):R80.

16. Linden A. Measuring diagnostic and predictive accuracy in disease management: an introduction to receiver operating characteristic (ROC) analysis. Journal of evaluation in clinical practice. Apr 2006; 12(2): 132-139.

17. Kramar A, Faraggi D, Fortune A, Reiser B. mROC: a computer program for combining tumour markers in predicting disease states. Computer methods and programs in biomedicine. Sep 2001;66(2-3):199-207.

18. Breiman L. Random Forests. Machine Learning. Kluwer Academic Publishers. 2001;45:5-32.

19. Consortium GO. Gene Ontology Consortium: going forward. Nucleic acids research. Jan 2015;43(Database issue):D1049-1056.

20. Raudvere U, Kolberg L, Kuzmin I, et al. g:Profiler: a web server for functional enrichment analysis and conversions of gene lists (2019 update). Nucleic acids research. Jul 2 2019;47(W1):W191-W198.

21. Pomaznoy M, Ha B, Peters B. GOnet: a tool for interactive Gene Ontology analysis. BMC bioinformatics. Dec 7 2018;19(1):470.

22. Salvetat N, Van der Laan S, Vire B, et al. RNA editing blood biomarkers for predicting mood alterations in HCV patients. Journal of neurovirology. Jul 22 2019.

23. Szklarczyk D, Gable AL, Lyon D, et al. STRING v11: protein-protein association networks with increased coverage, supporting functional discovery in genome-wide experimental datasets. Nucleic acids research. Jan 8 2019;47(D1):D607-D613.

24. Shelton RC. The molecular neurobiology of depression. The Psychiatric clinics of North America. Mar 2007;30(1):1-11.

25. Costas J, Gratacos M, Escaramis G, et al. Association study of 44 candidate genes with depressive and anxiety symptoms in post-partum women. Journal of psychiatric research. Aug 2010;44(11):717-724.

26. Colledge M, Snyder EM, Crozier RA, et al. Ubiquitination regulates PSD-95 degradation and AMPA receptor surface expression. Neuron. Oct 30 2003;40(3):595-607.

27. Liu B, Mink S, Wong KA, et al. PIAS1 selectively inhibits interferon-inducible genes and is important in innate immunity. Nature immunology. Sep 2004;5(9):891-898.

28. Murakami Y, Ishibashi T, Tomita E, et al. Depressive symptoms as a side effect of Interferon-alpha therapy induced by induction of indoleamine 2,3-dioxygenase 1. Scientific reports. Jul 20 2016;6:29920.

29. Brodie EJ, Infantino S, Low MSY, Tarlinton DM. Lyn, Lupus, and (B) Lymphocytes, a Lesson on the Critical Balance of Kinase Signaling in Immunity. Frontiers in immunology. 2018;9:401.

30. Hayashi T, Umemori H, Mishina M, Yamamoto T. The AMPA receptor interacts with and signals through the protein tyrosine kinase Lyn. Nature. Jan 7 1999;397(6714):72-76.

31. Moura-Alves P, Fae K, Houthuys E, et al. AhR sensing of bacterial pigments regulates antibacterial defence. Nature. Aug 28 2014;512(7515):387-392.

32. Song MS, Song SJ, Kim SY, Oh HJ, Lim DS. The tumour suppressor RASSF1A promotes MDM2 self-ubiquitination by disrupting the MDM2-DAXX-HAUSP complex. The EMBO journal. Jul 9 2008;27(13):1863-1874.

33. Wang H, Nestor CE, Benson M, Zhang H. GAB2 regulates type 2 T helper cell differentiation in humans. Cytokine. Aug 2017;96:234-237.

34. Verploegen S, Lammers JW, Koenderman L, Coffer PJ. Identification and characterization of CKLiK, a novel granulocyte Ca(++)/calmodulin-dependent kinase. Blood. Nov 1 2000;96(9):3215-3223.

35. Lin E, Kuo PH, Liu YL, Yu YW, Yang AC, Tsai SJ. A Deep Learning Approach for Predicting Antidepressant Response in Major Depression Using Clinical and Genetic Biomarkers. Frontiers in psychiatry. 2018;9:290.

36. Zhou X, Chen Y, Mok KY, et al. Identification of genetic risk factors in the Chinese population implicates a role of immune system in Alzheimer's disease pathogenesis. Proceedings of the National Academy of Sciences of the United States of America. Feb 20 2018; 115(8): 1697-1706.

37. Beurel E, Lowell JA. Th17 cells in depression. Brain, behavior, and immunity. Mar 2018;69:28-34.

38. Fischer EH, Charbonneau H, Tonks NK. Protein tyrosine phosphatases: a diverse family of intracellular and transmembrane enzymes. Science. Jul 26 1991;253(5018):401-406.

39. Irie-Sasaki J, Sasaki T, Matsumoto W, et al. CD45 is a JAK phosphatase and negatively regulates cytokine receptor signalling. Nature. Jan 18 2001;409(6818):349-354.

40. Basterzi AD, Yazici K, Buturak V, et al. Effects of venlafaxine and fluoxetine on lymphocyte subsets in patients with major depressive disorder: a flow cytometric analysis. Progress in neuro-psychopharmacology & biological psychiatry. Feb 1 2010;34(1):70-75.

41. Aston C, Jiang L, Sokolov BP. Transcriptional profiling reveals evidence for signaling and oligodendroglial abnormalities in the temporal cortex from patients with major depressive disorder. Molecular psychiatry. Mar 2005;10(3):309-322.

42. Seney ML, Huo Z, Cahill K, et al. Opposite Molecular Signatures of Depression in Men and Women. Biological psychiatry. Jul 1 2018;84(1):18-27.

43. Wohleb ES, Franklin T, Iwata M, Duman RS. Integrating neuroimmune systems in the neurobiology of depression.

Nature reviews. Neuroscience. Aug 2016; 17(8):497-511.
44. Weissmann D, van der Laan S, Underwood MD, et al. Region-specific alterations of A-to-I RNA editing of serotonin 2c receptor in the cortex of suicides with major depression. Translational psychiatry. Aug 30 2016;6(8):e878.

**Claims**

1. An in vitro method for diagnosing depression disorders, in a human patient from a biological sample of said patient, said method comprising:

   a) determining for a combination of at least two A to I editing RNA biomarkers:

   - for each selected biomarker, the relative proportion of RNA editing at a given editing site for at least one or a combination of sites which can be edited on the RNA transcript of said at least two biomarkers, and/or
   - the relative percentage of an isoform or of a combination of isoforms of the RNA transcript of each of said two biomarkers;

   wherein said at least two A to I editing RNA biomarkers are selected from the group consisting of PRKCB, MDM2, PIAS1, IFNAR1, IFNAR2, LYN, AHR, RASSF1, GAB2, CAMK1D, KCNJ15, IL17RA, PTPRC and PDE8A biomarkers,
   b) determining a result value obtained for each of said at least two biomarkers and a final result value based on an algorithm or equation that includes the result value obtained for each selected biomarker;
   c) determining the final result value or depression score obtained for the patient and a control result value obtained for a healthy control subject, wherein the control result value was determined in a manner comparable to that of the final result value; and
   d) if said final result or depression score value is greater than a threshold/cut-off, classifying said patient as being positive or having depression disorders, or, if said final result value is not greater than said threshold, classifying said patient as being negative or not having depression disorders.

2. The in vitro method for diagnosing depression according to claim 1, wherein in step b), said at least two A to I editing RNA biomarkers are selected from the group consisting of PRKCB, MDM2, IFNAR1, KCNJ15, LYN, CAMK1D, GAB2 and PDE8A.

3. The in vitro method for diagnosing depression disorders, preferably DEP, according to one of claims 1 and 2, wherein in step b), the result value or depression score is calculated by an algorithm implementing a multivariate method including:

   - mROC program, particularly to identify the linear combination, which maximizes the AUC (Area Under the Curve) ROC and wherein the equation for the respective combination is provided and can be used as a new virtual marker Z, as follows:

$$Z = a.(\text{Biomarker } 1) + b.(\text{Biomarker } 2) + \ldots i.(\text{Biomarker } i) + \ldots n.(\text{Biomarker } n)$$

   where i are calculated coefficients and (Biomarker i) are the level of the considered biomarker; and/or
   - a Random Forest (RF) approach applied to assess the RNA editing site(s) and/or isoforms combinations, particularly to rank the importance of the RNA editing site(s) and/or isoform(s), and to combine the best RNA editing site(s) and/or isoform(s), and/or optionally
   - a multivariate analysis applied to assess the RNA editing site(s) and/or isoforms combinations for the diagnostic, said multivariate analysis being selecting for example from the group consisting of:
   - logistic regression model applied for univariate and multivariate analysis to estimate the relative risk of patient at different level of RNA editing site or isoforms values; and/or
   - CART (Classification And Regression Trees) approach applied to assess RNA editing site(s) and/or isoforms combinations; and/or
   - Support Vector Machine (SVM) approach;
   - Artificial Neural Network (ANN) approach;
   - Bayesian network approach;

- WKNN (weighted k-nearest neighbours) approach;
- Partial Least Square - Discriminant Analysis (PLS-DA);
- Linear and Quadratic Discriminant Analysis (LDA / QDA), and
- Any other mathematical method that combines biomarkers.

4. The method of one of claims 1 to 3, wherein said depression disorder is DEP.

5. The method of one of claims 1 to 4, wherein said biological sample is whole blood, serum, plasma, urine, cerebrospinal fluid or saliva, preferred is the whole blood sample.

6. The method of one of claims 1 to 5, wherein for each selected biomarker, said result value is statistically/specifically different from said control result value at $p < 0.05$.

7. The method of one of claims 1 to 6, wherein the following criteria has to be satisfied for the biomarker or the combination of biomarkers selected tin step a):

   - the coverage >30;
   - AUC>0.8;
   - 0.95 >FoldChange >1.05, and
   - $p < 0.05$.

8. The method of one of claims 1 to 7, wherein said selected A to I editing RNA biomarker(s) comprised in step a) is selected from the group consisting of:
   a combination comprising at least 3, 4, 5, 6, 7 or 8 of the following biomarkers: PRKCB, MDM2, IFNAR1, KCNJ15, LYN, CAMK1D, GAB2 and PDE8A, preferably the combination of the cited 8 biomarkers.

9. The method of one of claims 1 to 8, wherein said selected A to I editing RNA biomarker(s) comprised in step a) the combination of the following 8 biomarkers GAB2, IFNAR1, KCNJ15, LYN, MDM2, PRKCB, CAMK1D and PDE8A when the model is adjusted by age, sex, psychiatric treatment and addiction, or the when the model is adjusted by age and sex, and using a Random Forest (RF) algorithm applied to assess the RNA editing site(s) and/or isoforms combinations, particularly to rank the importance of the RNA editing site(s) and/or isoform(s), and to combine the best RNA editing site(s) and/or isoform(s), and preferably, wherein the calculation of the relative percentage of at least one of the RNA edition site or isoform is based on the RNA edition site or isoform listed in Tables 5.1 and 5.2.

10. The method of one of claims 1 to 9, wherein said combination of at least 2, 3, 4, 5, 6, 7 or 8 selected biomarkers comprises the calculation of the relative percentage of at least one of the RNA edition site or isoform listed in Table 2A (edition sites), 2B (isoforms) or 3 (edition sites) for each of the selected biomarker.

11. The method of one of claims 2 to 10, wherein said combination of at least 2, 3, 4, 5, 6, 7 or 8 selected biomarkers is selected from the biomarkers combinations given in Tables 4 and 5, preferably the combinations comprising the following 8 biomarkers PRKCB, MDM2, IFNAR1, KCNJ15, LYN, CAMK1D, GAB2 and PDE8A.

12. The method of one of claims 1 to 11, wherein the algorithm or equation allowing the calculation of the result value or depression score Z are selected from the Z equations listed in the examples and the figures, preferably the equation implemented a combination of the following 8 biomarkers PRKCB, MDM2, IFNAR1, KCNJ15, LYN, CAMK1D, GAB2 and PDE8A.

13. An in vitro method for diagnosing depression disorders, preferably MDD, in a human patient according to one of claims 1 to 12, wherein if the patient to be tested is a female, in step a) the biomarkers of said combination of at least two one A to I editing RNA biomarker are selected from the group of biomarkers consisting of PRKCB, MDM2, PIAS1, IFNAR1, IFNAR2, LYN, AHR, RASSF1, GAB2, CAMK1D, KCNJ15, IL17RA, PTPRC and PDE8A biomarkers, preferably the PRKCB, MDM2, IFNAR1, KCNJ15, LYN, CAMK1D, GAB2 and PDE8A biomarkers, preferably the combination of the 8 preferred biomarkers.

14. An in vitro method for diagnosing depression disorders, preferably MDD, in a human patient according to one of claims 1 to 12, wherein if the patient to be tested is a male, in step a) the biomarkers of said combination of at least two one A to I editing RNA biomarker are selected from the group of biomarkers consisting of PRKCB, MDM2, PIAS1, IFNAR1, IFNAR2, LYN, AHR, RASSF1, GAB2, CAMK1D, KCNJ15, IL17RA, PTPRC and PDE8A biomarkers, preferably the PRKCB, MDM2, IFNAR1, KCNJ15, LYN, CAMK1D, GAB2 and PDE8A biomarkers, preferably the combination of the 8 preferred biomarkers.

15. An in vitro method for diagnosing depression disorders, preferably DEP, in a human patient wherein said method comprises the method for diagnosing depression for a human patient according to one of claims 1 to 14, wherein the combination of biomarkers and/or the Z equation associated with said combination used for the diagnosing of depression disorders is different whether the patient to be tested is a female or a male.

16. The method of one of claims 1 to 15, wherein in step a) the relative proportion of RNA editing at a given editing and/or the percentage of an isoform are measuring by NGS in said biological sample.

17. The method of one of claims 1 to 17, wherein in step a), the amplicon/nucleic sequence used for the detection of the RNA editing sites and/or the isoforms of the RNA transcript of said biomarker is obtained or obtainable with:

- the set of primers listed in Table 1 for each of the selected biomarkers (SEQ ID NO. 1 to 36), or
- a set or a combination of sets of primers allowing to obtain amplicon(s) including, or identical to, the amplicon(s) obtainable by the set of primers listed in Table 1 (SEQ ID No.1 to SEQ ID No.36).

18. A method for monitoring treatment for depression disorders, preferably DEP, in a human subject, said method comprising:

A) diagnosing depression disorders in said human by the method according to one of claims 1 to 17 before the beginning of the treatment which is desired to be monitored.
B) repeating steps (a) to c) of the method of one of claims 1 to 17, after a period of time during which said patient received treatment for said depression disorders to obtain a post-treatment depression score;
C) comparing the post-treatment depression score from step (c) to:

- the depression score obtained before the period of time during which said patient receives treatment for said depression disorders, and
- to the depression score (control final value) for normal/healthy subjects, and classifying said treatment as being effective if the post-treatment depression score obtained in step B) is closer than the depression score obtained before the period of time during which said patient receives treatment for the depression disorders score obtained for normal/healthy subjects.

19. Kit for determining whether a patient present a depression disorder, preferably MDD, said kit comprising:

1) optionally instructions to apply the method according to one of claims 1 to 17, in order to obtain the result value or depression score the analysis of which determining whether said patient present a depression disorder, preferably DEP; and
2)

i) a combination of at least two set of primers from the group of pairs of primers selecting from the SEQ ID No.1 to SEQ ID No.36, the selection of which being dependent of the biomarkers combination used for depression disorders, preferably DEP, diagnostic;
or
ii) a combination of at least two sets of primers allowing to obtain amplicons identical to the amplicons obtained by the at least two set of primers selected in i).

**Patentansprüche**

1. In-vitro-Verfahren zum Diagnostizieren von Depressionsstörungen bei einem menschlichen Patienten aus einer biologischen Probe des Patienten, wobei das Verfahren folgendes umfasst:

a) Bestimmen einer Kombination von mindestens zwei A- bis I-editierenden RNA-Biomarkern:

- für jeden ausgewählten Biomarker den relativen Anteil der RNA-Editierung an einer gegebenen Editierstelle für mindestens eine oder eine Kombination von Stellen, die auf dem RNA-Transkript der mindestens zwei Biomarker editiert werden können, und/oder

- den relativen Prozentsatz einer Isoform oder einer Kombination von Isoformen des RNA-Transkripts von jedem der beiden Biomarker;

wobei die mindestens zwei A- bis I-editierenden RNA-Biomarker ausgewählt werden aus der Gruppe bestehend aus PRKCB, MDM2, PIAS1, IFNAR1, IFNAR2, LYN, AHR, RASSF1, GAB2, CAMK1D, KCNJ15, IL17RA, PTPRC und PDE8A Biomarkern,

b) Bestimmen eines für jeden der mindestens zwei Biomarker erhaltenen Ergebniswertes und eines Endergebniswertes auf der Grundlage eines Algorithmus oder einer Gleichung, der/die den für jeden ausgewählten Biomarker erhaltenen Ergebniswert einschließt;

c) Bestimmen des für den Patienten erhaltenen Endergebniswertes oder Depressions-Scores und eines für ein gesundes Kontrollsubjekt erhaltenen Kontrollergebniswertes, wobei der Kontrollergebniswert auf eine mit dem Endergebniswert vergleichbare Weise bestimmt wurde; und

d) wenn der Wert des Endergebnisses oder der Wert des Depressions-Scores größer ist als ein Schwellenwert/Grenzwert, Klassifizieren des Patienten als positiv oder mit Depressionsstörungen, oder, wenn der Endergebniswert nicht größer ist als der Schwellenwert, Klassifizieren des Patienten als negativ oder nicht mit Depressionsstörungen.

2. In-vitro-Verfahren zur Diagnostizierung von Depressionen nach Anspruch 1, wobei in Schritt b) die mindestens zwei A- bis I-editierenden RNA-Biomarker aus der Gruppe bestehend aus PRKCB, MDM2, IFNAR1, KCNJ15, LYN, CAMK1D, GAB2 und PDE8A ausgewählt werden.

3. In-vitro-Verfahren zur Diagnostizierung von Depressionsstörungen, vorzugsweise DEP, nach einem der Ansprüche 1 und 2, wobei in Schritt b) der Ergebniswert oder Depressions-Score durch einen Algorithmus berechnet wird, der ein multivariates Verfahren implementiert, einschließlich:

- ein mROC-Programm, insbesondere zur Identifizierung der linearen Kombination, die die AUC (Area Under the Curve) ROC maximiert und wobei die Gleichung für die jeweilige Kombination bereitgestellt wird und als neuer virtueller Marker Z verwendet werden kann, wie folgt:

$$Z = a.(\text{Biomarker 1}) + b.(\text{Biomarker2}) + ...i.(\text{Biomarker i}) +....n .(\text{Biomarker n})$$

wobei i die berechneten Koeffizienten und (Biomarker i) das Niveau des jeweiligen Biomarkers sind; und/oder

- einen Random Forest-Ansatz (RF-Ansatz), der angewandt wird, um die Kombinationen von RNA-Editierstellen und/oder Isoformen zu bewerten, insbesondere um eine Rangliste der Wichtigkeit der RNA-Editierstelle(n) und/oder Isoform(en) zu erstellen und die beste(n) RNA-Editierstelle(n) und/oder Isoform(en) zu kombinieren, und/oder optional

- eine multivariate Analyse, die angewandt wird, um die Kombinationen von RNA-Editierstellen und/oder Isoformen für die Diagnose zu bewerten, wobei die multivariate Analyse beispielsweise ausgewählt wird aus der Gruppe bestehend aus:

- einem logistischen Regressionsmodell, das für die univariate und multivariate Analyse angewandt wird, um das relative Risiko des Patienten bei unterschiedlichen Niveaus der Werte der RNA-Editierstelle oder Isoformen zu schätzen; und/oder

- ein Ansatz von CART (Classification And Regression Trees) wird angewandt, um die Kombination von RNA-Editierstellen und/oder Isoformen zu bewerten; und/oder

- Ansatz der unterstützenden Vektormaschine (Support Vector Machine, SVM);

- Ansatz des künstlichen neuronalen Netzes (Artificial Neural Network, ANN);

- Ansatz des Bayes'schen Netzwerks;

- Ansatz des WKNN (weighted k-nearest neighbours);

- Partielle Kleinste-Quadrate-Diskriminanzanalyse (Partial Least Square - Discriminant Analysis, PLS-DA);

- Lineare und quadratische Diskriminanzanalyse (Linear and Quadratic Discriminant Analysis, LDA/ QDA), und

- jedes andere mathematische Verfahren, das Biomarker kombiniert.

4. Verfahren nach einem der Ansprüche 1 bis 3, wobei die Depressionsstörung DEP ist.

5. Verfahren nach einem der Ansprüche 1 bis 4, wobei die biologische Probe Vollblut, Serum, Plasma, Urin, Liquor oder Speichel ist, bevorzugt die Vollblutprobe ist.

**6.** Verfahren nach einem der Ansprüche 1 bis 5, wobei für jeden ausgewählten Biomarker der Ergebniswert statistisch/spezifisch von dem Kontrollergebniswert bei p<0,05 verschieden ist.

**7.** Verfahren nach einem der Ansprüche 1 bis 6, wobei die folgenden Kriterien für den in Schritt a) ausgewählten Biomarker oder die Kombination von Biomarkern erfüllt sein müssen:

- die Abdeckung >30;
- AUC>0,8;
- 0,95 >FoldChange >1,05, und
- p<0,05.

**8.** Verfahren nach einem der Ansprüche 1 bis 7, wobei der/die ausgewählte(n) A- bis I-editierende(n) RNA-Biomarker, umfasst/umfassend in Schritt a), ausgewählt wird/werden aus der Gruppe bestehend aus:
einer Kombination, die mindestens 3, 4, 5, 6, 7 oder 8 der folgenden Biomarker umfasst: PRKCB, MDM2, IFNAR1, KCNJ15, LYN, CAMK1D, GAB2 und PDE8A, vorzugsweise die Kombination der genannten 8 Biomarker.

**9.** Verfahren nach einem der Ansprüche 1 bis 8, wobei der/die ausgewählte(n) A- bis I-editierende(n) RNA-Biomarker in Schritt a) die Kombination der folgenden 8 Biomarker umfasst/umfassen: GAB2, IFNAR1, KCNJ15, LYN, MDM2, PRKCB, CAMK1D und PDE8A, wenn das Modell nach Alter, Geschlecht, psychiatrischer Behandlung und Sucht angepasst wird, oder wenn das Modell nach Alter und Geschlecht angepasst wird, und unter Verwendung eines Random-Forest-Algorithmus (RF-Algorithmus), der angewandt wird, um die Kombinationen von RNA-Editierstellen und/oder Isoformen zu bewerten, insbesondere um die Wichtigkeit der RNA-Editierstelle(n) und/oder Isoform(en) in eine Rangliste zu bringen und die beste(n) RNA-Editierstelle(n) und/oder Isoform(en) zu kombinieren, und vorzugsweise, wobei die Berechnung des relativen Prozentsatzes von mindestens einer der RNA-Editionsstelle oder Isoform auf der RNA-Editionsstelle oder Isoform basiert, die in den Tabellen 5.1 und 5.2. aufgeführt sind.

**10.** Verfahren nach einem der Ansprüche 1 bis 9, wobei die Kombination von mindestens 2, 3, 4, 5, 6, 7 oder 8 ausgewählten Biomarkern die Berechnung des relativen Prozentsatzes von mindestens einer der in Tabelle 2A (Editionsstellen), 2B (Isoformen) oder 3 (Editionsstellen) aufgeführten RNA-Editionsstellen oder Isoformen für jeden der ausgewählten Biomarker umfasst.

**11.** Verfahren nach einem der Ansprüche 2 bis 10, wobei die Kombination von mindestens 2, 3, 4, 5, 6, 7 oder 8 ausgewählten Biomarkern aus den in den Tabellen 4 und 5 angegebenen Kombinationen von Biomarkern ausgewählt wird, wobei die Kombinationen vorzugsweise die folgenden 8 Biomarker umfassen: PRKCB, MDM2, IFNAR1, KCNJ15, LYN, CAMK1D, GAB2 und PDE8A.

**12.** Verfahren nach einem der Ansprüche 1 bis 11, wobei der Algorithmus oder die Gleichung, die die Berechnung des Ergebniswertes oder des Depressions-Scores Z ermöglichen, aus den in den Beispielen und Figuren aufgeführten Z-Gleichungen ausgewählt werden, wobei die Gleichung vorzugsweise eine Kombination der folgenden 8 Biomarker PRKCB, MDM2, IFNAR1, KCNJ15, LYN, CAMK1D, GAB2 und PDE8A implementiert.

**13.** In-vitro-Verfahren zur Diagnostizierung von Depressionsstörungen, vorzugsweise MDD, bei einem menschlichen Patienten nach einem der Ansprüche 1 bis 12, wobei, wenn der zu testende Patient weiblich ist, in Schritt a) die Biomarker der Kombination von mindestens zwei ein A- bis I-editierenden RNA-Biomarkern ausgewählt werden aus der Gruppe von Biomarkern bestehend aus PRKCB, MDM2, PIAS1, IFNAR1, IFNAR2, LYN, AHR, RASSF1, GAB2, CAMK1D, KCNJ15, IL17RA, PTPRC und PDE8A Biomarker, vorzugsweise die PRKCB, MDM2, IFNAR1, KCNJ15, LYN, CAMK1D, GAB2 und PDE8A Biomarker, vorzugsweise die Kombination der 8 bevorzugten Biomarker.

**14.** In-vitro-Verfahren zur Diagnostizierung von Depressionsstörungen, vorzugsweise MDD, bei einem menschlichen Patienten nach einem der Ansprüche 1 bis 12, wobei, wenn der zu testende Patient männlich ist, in Schritt a) die Biomarker der Kombination von mindestens zwei ein A- bis I-editierenden RNA-Biomarkern ausgewählt werden aus der Gruppe von Biomarkern bestehend aus PRKCB, MDM2, PIAS1, IFNAR1, IFNAR2, LYN, AHR, RASSF1, GAB2, CAMK1D, KCNJ15, IL17RA, PTPRC und PDE8A Biomarker, vorzugsweise die PRKCB, MDM2, IFNAR1, KCNJ15, LYN, CAMK1D, GAB2 und PDE8A Biomarker, vorzugsweise die Kombination der 8 bevorzugten Biomarker.

**15.** In-vitro-Verfahren zur Diagnostizierung von Depressionsstörungen, vorzugsweise DEP, bei einem menschlichen Patienten, wobei das besagte Verfahren das Verfahren zur Diagnostizierung von Depressionen bei einem menschlichen Patienten nach einem der Ansprüche 1 bis 14 umfasst, wobei die Kombination von Biomarkern und/oder die mit dieser Kombination verbundene Z-Gleichung, die zur Diagnostizierung von Depressionsstörungen verwendet wird, unterschiedlich ist, je nachdem, ob es sich bei dem zu testenden Patienten um eine Frau oder einen Mann handelt.

16. Verfahren nach einem der Ansprüche 1 bis 15, wobei in Schritt a) der relative Anteil der RNA-Editierung bei einer gegebenen Editierung und/oder der Prozentsatz einer Isoform durch NGS in der biologischen Probe gemessen wird.

17. Verfahren nach einem der Ansprüche 1 bis 17, wobei in Schritt a) das Amplikon/die Nukleinsequenz, das/die für die Erkennung der RNA-Editierstellen und/oder der Isoformen des RNA-Transkripts des Biomarkers verwendet wird, mit folgendem erhalten wird oder erhalten werden kann:

- dem in Tabelle 1 aufgeführten Satz von Primern für jeden der ausgewählten Biomarker (SEQ ID Nr. 1 bis 36), oder
- einem Satz oder einer Kombination von Sätzen von Primern, die es ermöglichen, Amplikon(e) zu erhalten, das/die Amplikon(e) einschließt/einschließen oder mit diesen identisch sind, die durch den in Tabelle 1 aufgeführten Satz von Primern (SEQ ID Nr. 1 bis SEQ ID Nr. 36) erhalten werden können.

18. Verfahren zur Überwachung der Behandlung von Depressionsstörungen, vorzugsweise DEP, bei einem menschlichen Subjekt, wobei das Verfahren folgendes umfasst:

A) Diagnostizieren von Depressionsstörungen bei dem Menschen mit dem Verfahren nach einem der Ansprüche 1 bis 17 vor Beginn der Behandlung, die überwacht werden soll.
B) Wiederholen der Schritte (a) bis c) des Verfahrens nach einem der Ansprüche 1 bis 17 nach einem Zeitraum, in dem der Patient eine Behandlung für die Depressionsstörungen empfangen hat, um einen Depressions-Score nach der Behandlung zu erhalten;
C) Vergleichen des Depressions-Scores nach der Behandlung aus Schritt (c) mit:

- dem Depressions-Score, der vor dem Zeitraum erhalten wurde, in dem der Patient eine Behandlung für die Depressionsstörungen empfängt, und
- mit dem Depressions-Score (Kontrollendwert) für normale/gesunde Subjekte und Klassifizieren der Behandlung als wirksam, wenn der in Schritt B) erhaltene Depressions-Score nach der Behandlung näher an dem Depressions-Score liegt, der vor dem Zeitraum erhalten wurde, in dem der Patient eine Behandlung für die Depressionsstörungen empfängt, die für normale/gesunde Subjekte erhalten wurde.

19. Kit zur Bestimmung, ob ein Patient eine Depressionsstörung, vorzugsweise MDD, aufweist, wobei das Kit folgendes umfasst:

1) gegebenenfalls Anweisungen zur Anwendung des Verfahrens nach einem der Ansprüche 1 bis 17, um den Ergebniswert oder Depressions-Score zu erhalten, dessen Analyse bestimmt, ob der Patient eine Depressionsstörung, vorzugsweise DEP, aufweist; und
2)

i) eine Kombination aus mindestens zwei Primersätzen aus der Gruppe der Primerpaare, die aus den SEQ ID Nr. 1 bis SEQ ID Nr. 36 ausgewählt werden, wobei die Auswahl von der Biomarkerkombination abhängt, die für die Diagnose von Depressionsstörungen, vorzugsweise DEP, verwendet wird; oder
ii) eine Kombination aus mindestens zwei Primersätzen, die es ermöglicht, Amplikons zu erhalten, die mit den Amplikons identisch sind, die durch die mindestens zwei in i) ausgewählten Primersätzen erhalten wurden.

## Revendications

1. Procédé in vitro permettant de diagnostiquer des troubles dépressifs, chez un patient humain à partir d'un échantillon biologique dudit patient, ledit procédé comprenant :

a) la détermination pour une combinaison d'au moins deux biomarqueurs d'ARN d'édition A à I :

- pour chaque biomarqueur sélectionné, de la proportion relative d'édition d'ARN au niveau d'un site d'édition donné pour au moins l'un ou une combinaison de sites qui peuvent être édités sur la transcription d'ARN desdits au moins deux biomarqueurs, et/ou
- du pourcentage relatif d'un isoforme ou d'une combinaison d'isoformes de la transcription d'ARN de chacun desdits deux biomarqueurs ;

dans lequel lesdits au moins deux biomarqueurs d'ARN d'édition A à I sont sélectionnés dans le groupe constitué par les biomarqueurs PRKCB, MDM2, PIAS1, IFNAR1, IFNAR2, LYN, AHR, RASSF1, GAB2, CAMK1D, KCNJ15, IL17RA, PTPRC et PDE8A,

b) la détermination d'une valeur de résultat obtenue pour chacun desdits au moins deux biomarqueurs et d'une valeur de résultat final en fonction d'un algorithme ou d'une équation qui comporte la valeur de résultat obtenue pour chaque biomarqueur sélectionné ;

c) la détermination de la valeur de résultat final ou d'un score de dépression obtenu pour le patient et d'une valeur de résultat témoin obtenue pour un sujet témoin en bonne santé, dans lequel la valeur de résultat témoin a été déterminée d'une manière comparable à celle de la valeur de résultat final ; et

d) si ladite valeur de résultat final ou de score de dépression est supérieure à un seuil/une limite, la classification dudit patient comme étant positif ou présentant des troubles dépressifs, ou, si ladite valeur de résultat final n'est pas supérieure audit seuil, la classification dudit patient comme étant négatif ou ne présentant pas de troubles dépressifs.

2. Procédé in vitro permettant de diagnostiquer une dépression selon la revendication 1, dans lequel à l'étape b), lesdits au moins deux biomarqueurs d'ARN d'édition A à I sont sélectionnés dans le groupe constitué par PRKCB, MDM2, IFNAR1, KCNJ15, LYN, CAMK1D, GAB2 et PDE8A.

3. Procédé in vitro permettant de diagnostiquer des troubles dépressifs, de préférence un DEP, selon l'une des revendications 1 et 2, dans lequel à l'étape b), la valeur de résultat ou le score de dépression est calculé par un algorithme implémentant un procédé à variables multiples comportant :

- le programme mROC, en particulier pour identifier la combinaison linéaire, qui maximise l'AUC (aire sous la courbe) ROC et dans lequel l'équation pour la combinaison respective est fournie et peut être utilisée en tant que nouveau marqueur virtuel Z, comme suit :

Z = a.(Biomarqueur 1) +b.Biomarqueur 2) + ...i.(Biomarqueur i) +...n.(Biomarqueur n) où les i sont des coefficients calculés et les (Biomarqueur i) sont le niveau du biomarqueur considéré ; et/ou
- une méthode d'Arborescence aléatoire (RF) appliquée pour évaluer le(s) site(s) d'édition d'ARN et/ou des combinaisons d'isoformes, en particulier pour classer l'importance du ou des site(s) d'édition d'ARN et/ou isoforme(s), et pour combiner le(s) meilleur(s) site(s) d'édition d'ARN et/ou isoforme(s), et/ou facultativement
- une analyse à variables multiples appliquée pour évaluer le(s) site(s) d'édition d'ARN et/ou combinaisons d'isoformes pour le diagnostic, ladite analyse à variables multiples étant sélectionnée par exemple dans le groupe constitué par :
- un modèle de régression logistique appliqué pour une analyse à une seule variable et à variables multiples pour estimer le risque relatif du patient à un niveau différent de site d'édition d'ARN ou de valeurs d'isoformes ; et/ou
- une méthode CART (Classification et arbres de régression) appliquée pour évaluer un ou des site(s) d'édition d'ARN et/ou des combinaisons d'isoformes ; et/ou
- une méthode par Machine à vecteurs de support (SVM) ;
- une méthode par réseau neuronal artificiel (ANN) ;
- une méthode par réseau Bayésien ;
- une méthode WKNN (k plus proches voisins pondérés) ;
- une analyse discriminante par moindres carrés partiels (PLS-DA) ;
- une analyse discriminante linéaire et quadratique (LDA/ QDA), et
- n'importe quel autre procédé mathématique qui combine des biomarqueurs.

4. Procédé selon l'une des revendications 1 à 3, dans lequel ledit trouble dépressif est un DEP.

**5.** Procédé selon l'une des revendications 1 à 4, dans lequel ledit échantillon biologique est du sang total, du sérum, du plasma, de l'urine, du liquide céphalo-rachidien ou de la salive, on préfère l'échantillon de sang total.

**6.** Procédé selon l'une des revendications 1 à 5, dans lequel pour chaque biomarqueur sélectionné, ladite valeur de résultat est statistiquement/spécifiquement différente de ladite valeur de résultat témoin à p<0,05.

**7.** Procédé selon l'une des revendications 1 à 6, dans lequel les critères suivants doivent être respectés pour le biomarqueur ou la combinaison de biomarqueurs sélectionné à l'étape a) :

- la couverture >30 ;
- AUC>0,8 ;
- 0,95 >FoldChange >1,05, et
- p<0,05.

**8.** Procédé selon l'une des revendications 1 à 7, dans lequel ledit ou lesdits biomarqueur(s) d'ARN d'édition A à I sélectionnés compris à l'étape a) est/sont sélectionné(s) dans le groupe constitué par :
une combinaison comprenant au moins 3, 4, 5, 6, 7 ou 8 des biomarqueurs suivants : PRKCB, MDM2, IFNAR1, KCNJ15, LYN, CAMK1D, GAB2 et PDE8A, de préférence la combinaison des 8 biomarqueurs cités.

**9.** Procédé selon l'une des revendications 1 à 8, dans lequel ledit ou lesdits biomarqueur(s) d'ARN d'édition A à I sélectionnés comprenaient à l'étape a) la combinaison des 8 biomarqueurs suivants GAB2, IFNAR1, KCNJ15, LYN, MDM2, PRKCB, CAMK1D et PDE8A lorsque le modèle est ajusté par âge, sexe, traitement psychiatrique et addiction, ou lorsque le modèle est ajusté par âge et sexe, et que l'on utilise un algorithme d'arborescence aléatoire (RF) appliqué pour évaluer le(s) site(s) d'édition d'ARN et/ou combinaisons d'isoformes, en particulier pour classer l'importance du ou des site(s) d'édition d'ARN et/ou isoforme(s), et pour combiner le(s) meilleur(s) site(s) d'édition d'ARN et/ou isoforme(s), et de préférence, dans lequel le calcul du pourcentage relatif d'au moins l'un parmi le site d'édition d'ARN ou l'isoforme est en fonction du site d'édition d'ARN ou de l'isoforme listé dans les Tableaux 5.1 et 5.2.

**10.** Procédé selon l'une des revendications 1 à 9, dans lequel ladite combinaison d'au moins 2, 3, 4, 5, 6, 7 ou 8 biomarqueurs sélectionnés comprend le calcul du pourcentage relatif d'au moins l'un parmi le site d'édition d'ARN ou l'isoforme listé dans le Tableau 2A (sites d'édition), 2B (isoformes) ou 3 (sites d'édition) pour chacun des biomarqueurs sélectionnés.

**11.** Procédé selon l'une des revendications 2 à 10, dans lequel ladite combinaison d'au moins 2, 3, 4, 5, 6, 7 ou 8 biomarqueurs sélectionnés est sélectionnée parmi les combinaisons de biomarqueurs données dans les Tableaux 4 et 5, de préférence les combinaisons comprenant les 8 biomarqueurs suivants PRKCB, MDM2, IFNAR1, KCNJ15, LYN, CAMK1D, GAB2 et PDE8A.

**12.** Procédé selon l'une des revendications 1 à 11, dans lequel l'algorithme ou l'équation permettant le calcul de la valeur de résultat ou du score de dépression Z sont sélectionnés parmi les équations Z listées dans les exemples et les figures, de préférence l'équation a implémenté une combinaison des 8 biomarqueurs suivants PRKCB, MDM2, IFNAR1, KCNJ15, LYN, CAMK1D, GAB2 et PDE8A.

**13.** Procédé in vitro permettant de diagnostiquer des troubles dépressifs, de préférence un MDD, chez un patient humain selon l'une des revendications 1 à 12, dans lequel si le patient à tester est une femme, à l'étape a) les biomarqueurs de ladite combinaison d'au moins deux biomarqueurs d'ARN d'édition A à I sont sélectionnés dans le groupe de biomarqueurs constitués par les biomarqueurs PRKCB, MDM2, PIAS1, IFNAR1, IFNAR2, LYN, AHR, RASSF1, GAB2, CAMK1D, KCNJ15, IL17RA, PTPRC et PDE8A, de préférence les biomarqueurs PRKCB, MDM2, IFNAR1, KCNJ15, LYN, CAMK1D, GAB2 et PDE8A, de préférence la combinaison des 8 biomarqueurs préférés.

**14.** Procédé in vitro permettant de diagnostiquer des troubles dépressifs, de préférence un MDD, chez un patient humain selon l'une des revendications 1 à 12, dans lequel si le patient à tester est un homme, à l'étape a) les biomarqueurs de ladite combinaison d'au moins deux biomarqueurs d'ARN d'édition A à I sont sélectionnés dans le groupe de biomarqueurs constitués par les biomarqueurs PRKCB, MDM2, PIAS1, IFNAR1, IFNAR2, LYN, AHR, RASSF1, GAB2, CAMK1D, KCNJ15, IL17RA, PTPRC et PDE8A, de préférence les biomarqueurs PRKCB, MDM2, IFNAR1, KCNJ15, LYN, CAMK1D, GAB2 et PDE8A, de préférence la combinaison des 8 biomarqueurs préférés.

**15.** Procédé in vitro permettant de diagnostiquer des troubles dépressifs, de préférence un DEP, chez un patient humain dans lequel ledit procédé comprend le procédé permettant de diagnostiquer une dépression pour un patient humain selon l'une des revendications 1 à 14, dans lequel la combinaison de biomarqueurs et/ou l'équation Z associée à ladite combinaison utilisée pour le diagnostic de troubles dépressifs sont différentes si le patient à tester est un homme ou une femme.

**16.** Procédé selon l'une des revendications 1 à 15, dans lequel à l'étape a) la proportion relative d'édition d'ARN au niveau d'une édition donnée et/ou le pourcentage d'un isoforme sont mesurés par NGS dans ledit échantillon biologique.

**17.** Procédé selon l'une des revendications 1 à 17, dans lequel à l'étape a), l'amplicon/séquence nucléique utilisé pour la détection des sites d'édition d'ARN et/ou des isoformes de la transcription d'ARN dudit biomarqueur est obtenu ou peut être obtenu avec :

- l'ensemble d'amorces listées dans le Tableau 1 pour chacun des biomarqueurs sélectionnés (SEQ ID No. 1 à 36), ou
- un ensemble ou une combinaison d'ensembles d'amorces permettant d'obtenir un ou des amplicon(s) comportant les, ou identique(s) aux, amplicon(s) pouvant être obtenus par l'ensemble d'amorces listées dans le Tableau 1 (SEQ ID No.1 à SEQ ID No.36).

**18.** Procédé permettant de surveiller un traitement pour des troubles dépressifs, de préférence un DEP, chez un sujet humain, ledit procédé comprenant :

A) le diagnostic de troubles dépressifs chez ledit humain par le procédé selon l'une des revendications 1 à 17 avant le début du traitement que l'on souhaite surveiller.
B) la répétition des étapes (a) à c) du procédé selon l'une des revendications 1 à 17, après un laps de temps pendant lequel ledit patient a reçu un traitement pour lesdits troubles dépressifs pour obtenir un score de dépression post-traitement ;
C) la comparaison du score de dépression post-traitement provenant de l'étape (c) :

- au score de dépression obtenu avant le laps de temps pendant lequel ledit patient reçoit un traitement pour lesdits troubles dépressifs, et
- au score de dépression (valeur finale témoin) pour des sujets normaux/en bonne santé, et la classification dudit traitement comme étant efficace si le score de dépression post-traitement obtenu à l'étape B) est plus proche que le score de dépression obtenu avant le laps de temps pendant lequel ledit patient reçoit un traitement pour les troubles dépressifs, du score obtenu pour des sujets normaux/en bonne santé.

**19.** Trousse permettant de déterminer si un patient présente un trouble dépressif, de préférence un MDD, ladite trousse comprenant :

1) facultativement des instructions pour appliquer le procédé selon l'une des revendications 1 à 17, afin d'obtenir la valeur de résultat ou le score de dépression dont l'analyse détermine si ledit patient présente un trouble dépressif, de préférence un DEP ; et
2)

i) une combinaison d'au moins deux ensembles d'amorces parmi le groupe de paires d'amorces sélectionnées parmi SEQ ID No.1 à SEQ ID No.36, dont la sélection dépend de la combinaison de biomarqueurs utilisée pour un diagnostic de troubles dépressifs, de préférence un DEP ;
ou
ii) une combinaison d'au moins deux ensembles d'amorces permettant d'obtenir des amplicons identiques aux amplicons obtenus par les au moins deux ensembles d'amorces sélectionnés en i).

Fig. 1A

Fig. 1B

Fig. 1C

Fig. 1D

Fig. 2

Fig. 3

Fig. 4A

Fig. 4B

Fig. 4C

Fig. 4D

Fig. 5A

Fig. 5B

Fig. 5C

Fig. 5D

Fig. 5E

Fig. 5F

AUC=0.993

Empirical ROC curve ( 0.993 )
Youden Threshold
Equality Threshold

1-Specificity

Fig. 6A

AUC=0.751

Empirical ROC curve ( 0.751 )
Youden Threshold
Equality Threshold

1-Specificity

Fig. 6B

Fig. 6C

Fig. 7A

Fig. 7B

Fig. 7C

Fig. 7D

Fig. 7E

Fig. 8A

Fig. 8B

AUC=0.936

Empirical ROC curve ( 0.936 )
Youden Threshold
Equality Threshold

Fig. 8C

AUC=0.976

Empirical ROC curve ( 0.976 )
Youden Threshold
Equality Threshold

Fig. 8D

Fig. 8E

Fig. 9

Fig. 10A

Fig. 10B

Fig. 11

| | Total sample | CTRL | DEP |
|---|---|---|---|
| **Demographics** | | | |
| | n | n | n |
| Number | 64 | 32 | 32 |
| | | | |
| **Age** (min-max) | 24-61 | 24-61 | 26-61 |
| Age (mean±SD) | 42.5 (±10.1) | 42.5 (±10.1) | 42.6 (±10.3) |
| p value (*vs* Ctrl) | | | 0.46 |
| | | | |
| **Gender** | | | |
| Male (n (%)) | 32 (50%) | 16 (50%) | 16 (50%) |
| Female (n (%)) | 32 (50%) | 16 (50%) | 16 (50%) |
| | | | |
| **BMI (kg/m$^2$)** | | 25.6 | 23.9 |
| p value (*vs* Ctrl) | | | 0.15 |
| | | | |
| **Clinical characteristics** | | | |
| **Average MADRS score** (mean±SEM) | - | 1.06 (±0.3) | 21.3 (±2.5) |
| p value (*vs* Ctrl) | - | | 5.93E-09 |
| | | | |
| **Average IDSC30 score** (mean±SEM) | | 2.8 (±0.5) | 27.4 (± 2.9) |
| p value (*vs* Ctrl) | | | 2.59E-09 |

Data are the mean ± SEM. p-values of main characteristics are displayed with the Student's t-test.

CTRL: Healthy controls; DEP Major Depressive Episode; BMI: Body Mass Index

Fig. 12

| Category | GO ID | GO term | Ratio | p(FDR) |
|---|---|---|---|---|
| Biological Process | GO:0002682 | regulation of immune system process | 66/1391 | 2.37E-12 |
| Biological Process | GO:0050776 | regulation of immune response | 47/873 | 5.37E-10 |
| Biological Process | GO:0002376 | immune system process | 83/2370 | 1.09E-09 |
| Biological Process | GO:0050778 | positive regulation of immune response | 36/589 | 7.68E-09 |
| Biological Process | GO:0002768 | immune response-regulating cell surface receptor signaling pathway | 24/266 | 1.62E-08 |
| Biological Process | GO:0002757 | immune response-activating signal transduction | 26/332 | 3.53E-08 |
| Biological Process | GO:0002252 | immune effector process | 44/927 | 3.83E-08 |
| Biological Process | GO:0002253 | activation of immune response | 28/393 | 3.83E-08 |
| Biological Process | GO:0002429 | immune response-activating cell surface receptor signaling pathway | 22/234 | 3.83E-08 |
| Biological Process | GO:0002684 | positive regulation of immune system process | 43/882 | 3.83E-08 |
| Biological Process | GO:0002764 | immune response-regulating signaling pathway | 27/365 | 3.83E-08 |
| Biological Process | GO:0045321 | leukocyte activation | 42/894 | 1.13E-07 |
| Biological Process | GO:0001775 | cell activation | 45/1024 | 1.67E-07 |
| Biological Process | GO:0080090 | regulation of primary metabolic process | 144/5982 | 1.67E-07 |
| Biological Process | GO:0044093 | positive regulation of molecular function | 61/1713 | 2.90E-07 |
| Biological Process | GO:0006464 | cellular protein modification process | 88/2999 | 3.36E-07 |
| Biological Process | GO:0060255 | regulation of macromolecule metabolic process | 144/6072 | 3.63E-07 |
| Biological Process | GO:0048584 | positive regulation of response to stimulus | 68/2054 | 3.89E-07 |
| Biological Process | GO:0051171 | regulation of nitrogen compound metabolic process | 139/5827 | 5.79E-07 |
| Biological Process | GO:0006955 | immune response | 56/1560 | 8.88E-07 |

Fig. 13

| Pathway ID | Pathway name | Event hierarchy | Entities ratio | p(FDR) |
|---|---|---|---|---|
| R-HSA-202427 | Phosphorylation of CD3 and TCR zeta chains | Adaptative Immune System | 16/45 | 8.99E-10 |
| R-HSA-202433 | Generation of second messenger molecules | Adaptative Immune System | 16/58 | 1.79E-08 |
| R-HSA-202430 | Translocation of ZAP-70 to Immunological synapse | Adaptative Immune System | 14/42 | 1.79E-08 |
| R-HSA-389948 | PD-1 signaling | Adaptative Immune System | 14/45 | 3.27E-08 |
| R-HSA-202403 | TCR signaling | Adaptative Immune System | 21/146 | 8.91E-07 |
| R-HSA-388841 | Costimulation by the CD28 family | Adaptative Immune System | 19/97 | 1.30E-06 |
| R-HSA-202424 | Downstream TCR signaling | Adaptative Immune System | 17/124 | 3.53E-05 |
| R-HSA-877300 | Interferon gamma signaling | Cytokine Signaling in Immune System | 21/250 | 0.00273264 |
| R-HSA-2132295 | MHC class II antigen presentation | Adaptative Immune System | 15/148 | 0.00452889 |
| R-HSA-9616222 | Transcriptional regulation of granulopoiesis | Developmental biology | 9/71 | 0.02720614 |

Fig. 14

| Gene ID | GeneName | Region | Chr | Position (GRCh38) | Coverage | Fold Change | p value | AUC |
|---|---|---|---|---|---|---|---|---|
| ENSG00000177663 | IL17RA | 3UTR | 22 | 17110591 | 107 | 0.74 | 5.53E-03 | 0.929 |
| ENSG00000183049 | CAMK1D | intron | 10 | 12387673 | 34 | 0.66 | 6.32E-03 | 0.912 |
| ENSG00000142166 | IFNAR1 | 3UTR | 21 | 33355807 | 32 | 1.42 | 1.31E-02 | 0.910 |
| ENSG00000177663 | IL17RA | intron | 22 | 17089708 | 48 | 1.25 | 6.94E-03 | 0.906 |
| ENSG00000033327 | GAB2 | intron | 11 | 78330944 | 37 | 1.43 | 3.71E-03 | 0.896 |
| ENSG00000166501 | LYN | intron | 8 | 55890893 | 130 | 1.20 | 4.20E-02 | 0.880 |
| ENSG00000106546 | AHR | 3UTR | 7 | 17345159 | 61 | 1.36 | 1.47E-02 | 0.859 |
| ENSG00000033800 | PIAS1 | intron | 15 | 68138039 | 47 | 1.32 | 1.52E-02 | 0.857 |
| ENSG00000166501 | PRKCB | intron | 16 | 23920896 | 78 | 0.74 | 3.43E-03 | 0.853 |
| ENSG00000135679 | MDM2 | intron | 12 | 68821612 | 38 | 1.31 | 4.45E-02 | 0.850 |
| ENSG00000081237 | PTPRC | intron | 1 | 198680332 | 74 | 0.77 | 4.31E-03 | 0.846 |
| ENSG00000166501 | LYN | 3UTR | 8 | 56012553 | 44 | 1.48 | 2.11E-03 | 0.846 |
| ENSG00000159110 | IFNAR2 | intron | 21 | 33231766 | 37 | 0.73 | 6.82E-03 | 0.840 |
| ENSG00000068028 | RASSF1 | intron | 3 | 50332838 | 43 | 0.84 | 3.76E-02 | 0.825 |
| ENSG00000157551 | KCNJ15 | intron | 21 | 38288302 | 37 | 1.47 | 1.69E-02 | 0.819 |

Fig. 15

| Category | TermID | Term description | p(FDR) | matching proteins |
|---|---|---|---|---|
| Biological process | GO:0035556 | intracellular signal transduction | 7.64E-06 | AHR,CAMK1D,GAB2,IFNAR1,IFNAR2,LYN,MDM2,PIAS1,PRKCB,RASSF1 |
| Biological process | GO:0007259 | receptor signaling pathway via JAK-STAT | 1.41E-05 | IFNAR1,IFNAR2,LYN,PIAS1 |
| Biological process | GO:0007165 | signal transduction | 3.19E-05 | AHR,CAMK1D,GAB2,IFNAR1,IFNAR2,IL17RA,LYN,MDM2,PDE8A,PIAS1,PRKCB,PTPRC,RASSF1 |
| Biological process | GO:0050857 | positive regulation of antigen receptor-mediated signaling pathway | 9.82E-05 | LYN,PRKCB,PTPRC |
| Biological process | GO:0002682 | regulation of immune system process | 0.0002 | AHR,CAMK1D,GAB2,IFNAR2,LYN,PIAS1,PRKCB,PTPRC |
| Biological process | GO:0050853 | B cell receptor signaling pathway | 0.00029 | LYN,PRKCB,PTPRC |
| Biological process | GO:0071310 | cellular response to organic substance | 0.00039 | AHR,GAB2,IFNAR1,IFNAR2,IL17RA,LYN,MDM2,PDE8A,PIAS1 |
| Biological process | GO:0030888 | regulation of B cell proliferation | 0.0011 | AHR,LYN,PTPRC |
| Biological process | GO:0050776 | regulation of immune response | 0.0014 | GAB2,IFNAR2,LYN,PIAS1,PRKCB,PTPRC |
| Biological process | GO:0050861 | positive regulation of B cell receptor signaling pathway | 0.0014 | LYN,PRKCB |
| Biological process | GO:0002768 | immune response-regulating cell surface receptor signaling pathway | 0.0021 | GAB2,LYN,PRKCB,PTPRC |
| Biological process | GO:0007166 | cell surface receptor signaling pathway | 0.0025 | GAB2,IFNAR1,IFNAR2,IL17RA,LYN,PIAS1,PRKCB,PTPRC |
| Biological process | GO:0048522 | positive regulation of cellular process | 0.0025 | AHR,CAMK1D,GAB2,IL17RA,LYN,MDM2,PDE8A,PIAS1,PRKCB,PTPRC,RASSF1 |
| Biological process | GO:0048583 | regulation of response to stimulus | 0.0025 | CAMK1D,GAB2,IFNAR2,IL17RA,LYN,MDM2,PDE8A,PIAS1,PRKCB,PTPRC |
| Biological process | GO:0006950 | response to stress | 0.0041 | CAMK1D,IFNAR1,IFNAR2,IL17RA,LYN,MDM2,PRKCB,PTPRC,RASSF1 |
| Biological process | GO:0009893 | positive regulation of metabolic process | 0.0041 | AHR,CAMK1D,LYN,MDM2,PDE8A,PIAS1,PRKCB,PTPRC,RASSF1 |
| Biological process | GO:0051049 | regulation of transport | 0.0041 | CAMK1D,GAB2,IL17RA,KCNJ15,LYN,MDM2,PRKCB |
| Biological process | GO:0032879 | regulation of localization | 0.0046 | CAMK1D,GAB2,IL17RA,KCNJ15,LYN,MDM2,PRKCB,PTPRC |
| Biological process | GO:0006952 | defense response | 0.0047 | CAMK1D,IFNAR1,IFNAR2,IL17RA,LYN,PTPRC |
| Biological process | GO:0007049 | cell cycle | 0.0048 | AHR,MDM2,PIAS1,PRKCB,PTPRC,RASSF1 |

Fig. 16

| Matching entities | p(FDR) | Pathway name | Pathway ID |
|---|---|---|---|
| PRKCB | 0.0123504 | RUNX1 regulates transcription of genes involved in differentiation of myeloid cells | R-HSA-8939236 |
| MDM2;PIAS1 | 0.0202305 | SUMOylation of transcription factors | R-HSA-3232118 |
| IFNAR2;IFNAR1 | 0.0252798 | Regulation of IFNA signaling | R-HSA-912694 |
| PRKCB;MDM2 | 0.0252798 | Trafficking of AMPA receptors | R-HSA-399719 |
| PRKCB;MDM2 | 0.0252798 | Glutamate binding, activation of AMPA receptors and synaptic plasticity | R-HSA-399721 |
| MDM2 | 0.0252798 | Regulation of TP53 Degradation | R-HSA-6804757 |
| MDM2;PIAS1 | 0.0252798 | SUMOylation of ubiquitinylation proteins | R-HSA-3232142 |
| MDM2 | 0.0252798 | Regulation of TP53 Expression and Degradation | R-HSA-6806003 |
| LYN;GAB2 | 0.0252798 | Interleukin-3, Interleukin-5 and GM-CSF signaling | R-HSA-512988 |
| LYN;GAB2 | 0.0252798 | Signaling by SCF-KIT | R-HSA-1433557 |
| LYN;GAB2 | 0.0252798 | GAB1 signalosome | |
| LYN;IFNAR2;GAB2;IFNAR1;PI3 ASt;IFNAR1 | 0.0204054 | Cytokine Signaling in immune system | R-HSA-1280215 |
| PRKCB;MDM2;KCNS1 | 0.0288316 | Neurotransmitter receptors and postsynaptic signal transmission | R-HSA-112315 |

Fig. 17

| | Total Sample | CTRL | DEP |
|---|---|---|---|
| **Demographics** | | | |
| | n | n | n |
| Number | 76 | 41 | 35 |
| | | | |
| **Age** (min-max) | 19-61 | 21-61 | 19-60 |
| Age (mean±SD) | 43.1 (±11.7) | 43.5 (±12.7) | 42.5 (±10,6) |
| p value (*vs* Ctrl) | | | 0.36 |
| | | | |
| **Gender** | | | |
| Male (n (%)) | 39 (51.32%) | 21 (51.22%) | 16 (50%) |
| Female (n (%)) | 37 (48.68%) | 20 (48.78%) | 16 (50%) |
| | | | |
| **BMI** $(kg/m^2)$ | | 25.2 | 23.2 |
| p value (*vs* Ctrl) | | | 0.50 |
| | | | |
| **Clinical characteristics** | | | |
| **Average MADRS** score (mean±SEM) | | 0.76 (±0.2) | 24.3 (±1.8) |
| p value (*vs* Ctrl) | | | 7.16E-27 |
| | | | |
| **Average IDSC30** score (mean±SEM) | | 2.6 (±0.4) | 30.6(± 2.1) |
| p value (*vs* Ctrl) | | | 4.25E-26 |

Fig. 18

|  | Total sample | CTRL | DEP |
|---|---|---|---|
| **Demographics** | | | |
|  | **n** | **n** | **n** |
| Number | 86 | 28 | 58 |
| Age (min-max) | 18-64 | 21-64 | 18-62 |
| Age (mean±SD) | 43.2 (±12.3) | 45.8 (±14.4) | 41.4 (±11.2) |
| p value (*vs* Ctrl) | | | 0,16 |
| **BMI (kg/m²)** | | 24.2 | 23.5 |
| p value (*vs* Ctrl) | | | 0.44 |
| **Gender** | | | |
| Male (n (%)) | 40 (46.5%) | 13 (46.5%) | 27 (46.6%) |
| Female (n (%)) | 46 (53.5%) | 15 (53.5%) | 31 (53.4%) |
| **Clinical characteristics** | | | |
| **Average MADRS score (mean±SEM)** | | 0.5 (±1.1) | 33.1 (±0.9) |
| p value (*vs* Ctrl) | | | <0.00001 |
| **Average IDSC30 score (mean±SEM)** | | 2 (±1.9) | 38.6 (±1.1) |
| p value (*vs* Ctrl) | | | <0.00001 |

Fig. 19

|  | Total sample | CTRL | DEP |
|---|---|---|---|
| **Demographics** |  |  |  |
|  | **n** | **n** | **n** |
| **Number** | 411 | 143 | 268 |
|  |  |  |  |
| **Age** (min-max) | 18-67 | 18-65 | 18-67 |
| Age (mean±SD) | 39.4 (±13.4) | 39.4 (±14.1) | 39.4 (±13.1) |
| p value (*vs* Ctrl) |  |  | 0,99 |
|  |  |  |  |
| **Gender** |  |  |  |
| Male (n (%)) | 134 (32.6 %) | 53 (37.1%) | 81 (30.2%) |
| Female (n (%)) | 277 (67.4 %) | 90 (62.9%) | 187 (69.8%) |
|  |  |  |  |
| **BMI** (kg/m$^2$) |  | 24.6 | 23.8 |
| p value (*vs* Ctrl) |  |  | 0.12 |
|  |  |  |  |
| **Clinical characteristics** |  |  |  |
| **Average MADRS** score (mean±SEM) | - | 0.8 (±0.1) | 29.7 (±0.5) |
| p value (*vs* Ctrl) | - |  | <0.00001 |
|  |  |  |  |
| **Average IDSC30** score (mean±SEM) | - | 1.97 (±0.2) | 36.5 (± 0.5) |
| p value (*vs* Ctrl) | - |  | <0.00001 |

Fig. 20

| Category | GO ID | GO term | FDR |
|---|---|---|---|
| Biological Process | GO:0002376 | immune system process | 1.77E-10 |
| Biological Process | GO:0002682 | regulation of immune system process | 7.45E-10 |
| Biological Process | GO:0002252 | immune effector process | 1.52E-08 |
| Biological Process | GO:0002253 | activation of immune response | 7.50E-08 |
| Biological Process | GO:0050776 | regulation of immune response | 1.19E-07 |
| Biological Process | GO:0006955 | immune response | 1.51E-07 |
| Biological Process | GO:0002757 | immune response-activating signal transduction | 1.72E-07 |
| Biological Process | GO:0002764 | immune response-regulating signaling pathway | 1.74E-07 |
| Biological Process | GO:0050778 | positive regulation of immune response | 2.22E-07 |
| Biological Process | GO:0002684 | positive regulation of immune system process | 4.15E-07 |
| Biological Process | GO:0048584 | positive regulation of response to stimulus | 6.90E-07 |
| Biological Process | GO:0002444 | myeloid leukocyte mediated immunity | 1.0526E-06 |
| Biological Process | GO:0002274 | myeloid leukocyte activation | 4.8148E-06 |
| Biological Process | GO:0002446 | neutrophil mediated immunity | 8.1445E-06 |
| Biological Process | GO:0002366 | leukocyte activation involved in immune response | 8.4827E-06 |
| Biological Process | GO:0035556 | intracellular signal transduction | 8.6505E-06 |
| Biological Process | GO:0043299 | leukocyte degranulation | 8.8488E-06 |
| Biological Process | GO:0045321 | leukocyte activation | 9.2368E-06 |
| Biological Process | GO:0002263 | cell activation involved in immune response | 9.7805E-06 |
| Biological Process | GO:0002768 | immune response-regulating cell surface receptor signaling pathway | 1.059E-05 |

Fig. 21

| Category | GO ID | GO term | FDR | N of Genes |
|---|---|---|---|---|
| Biological Process | GO:0002682 | regulation of immune system process | 1.00E-10 | 67 |
| Biological Process | GO:0002376 | immune system process | 1.51E-07 | 84 |
| Biological Process | GO:0050776 | regulation of immune response | 5.90E-09 | 50 |
| Biological Process | GO:0050778 | positive regulation of immune response | 2.68E-07 | 38 |
| Biological Process | GO:0045321 | leukocyte activation | 3.61E-07 | 42 |
| Biological Process | GO:0002684 | positive regulation of immune system process | 5.45E-07 | 45 |
| Biological Process | GO:0048584 | positive regulation of response to stimulus | 5.99E-07 | 73 |
| Biological Process | GO:0001775 | cell activation | 6.94E-07 | 45 |
| Biological Process | GO:0036211 | protein modification process | 7.43E-07 | 86 |
| Biological Process | GO:0006464 | cellular protein modification process | 7.43E-07 | 86 |
| Biological Process | GO:0060255 | regulation of macromolecule metabolic process | 9.07E-07 | 139 |
| Biological Process | GO:0002757 | immune response-activating signal transduction | 1.06E-06 | 28 |
| Biological Process | GO:0002253 | activation of immune response | 1.07E-06 | 30 |
| Biological Process | GO:0002252 | immune effector process | 1.07E-06 | 43 |
| Biological Process | GO:0002764 | immune response-regulating signaling pathway | 1.12E-06 | 29 |
| Biological Process | GO:0043412 | macromolecule modification | 1.73E-06 | 88 |
| Biological Process | GO:0019222 | regulation of metabolic process | 4.11E-06 | 145 |
| Biological Process | GO:0002366 | leukocyte activation involved in immune response | 4.11E-06 | 31 |
| Biological Process | GO:0002263 | cell activation involved in immune response | 4.55E-06 | 31 |
| Biological Process | GO:0002274 | myeloid leukocyte activation | 5.48E-06 | 30 |

Fig. 22A

## Random Forest model1
### Adjusted by age, sex, treatments and addictions

| | Performance |
|---|---|
| AUC | 0.929 |
| CI 95% | [0.876 ; 0.981] |
| Se | 82.9% |
| Sp | 87.1% |
| PPV | 94.4% |
| NPV | 65.9% |
| Acc | 84.1% |
| Cutoff | 0.5 |

AUC=0.929

Sensitivity

Specificity

Random Forest model2
Adjusted by age, sex

AUC=0.911

| | Performance |
|---|---|
| AUC | 0.911 |
| CI 95% | [0.854 ; 0.968] |
| Se | 80.0% |
| Sp | 82.4% |
| PPV | 89.7% |
| NPV | 68.3% |
| Acc | 80.8% |
| Cutoff | 0.5 |

Fig. 22B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019185937 A1

**Non-patent literature cited in the description**

- Diagnostic and Statistics Manual for Mental Disorders
- DSM-5™ book. American Psychiatric Association, 2013, 155-188
- Diagnostic and Statistical Manual of Mental Disorders. American Psychiatric Association, 2013
- **DUNLOP K ; TALISHINSKY A ; LISTON C.** Intrinsic Brain Network Biomarkers of Antidepressant Response: a Review. *Current psychiatry reports.,* 13 August 2019, vol. 21 (9), 87
- **JENTSCH MC ; VAN BUEL EM ; BOSKER FJ et al.** Biomarker approaches in major depressive disorder evaluated in the context of current hypotheses. *Biomarkers in medicine.,* 2015, vol. 9 (3), 277-297
- **SMITH KM ; RENSHAW PF ; BILELLO J.** The diagnosis of depression: current and emerging methods. *Comprehensive psychiatry,* January 2013, vol. 54 (1), 1-6
- **CHIMIENTI F ; CAVAREC L ; VINCENT L et al.** Brain region-specific alterations of RNA editing in PDE8A mRNA in suicide decedents. *Translational psychiatry.,* 15 February 2019, vol. 9 (1), 91
- **DOBIN A ; GINGERAS TR.** Optimizing RNA-Seq Mapping with STAR. *Methods in molecular biology.,* 2016, vol. 1415, 245-262
- **DEPRISTO MA ; BANKS E ; POPLIN R et al.** A framework for variation discovery and genotyping using next-generation DNA sequencing data. *Nature genetics.,* May 2011, vol. 43 (5), 491-498
- **JOHN D ; WEIRICK T ; DIMMELER S ; UCHIDA S.** RNAEditor: easy detection of RNA editing events and the introduction of editing islands. *Briefings in bioinformatics.,* 01 November 2017, vol. 18 (6), 993-1001
- **YANG H ; WANG K.** Genomic variant annotation and prioritization with ANNOVAR and wANNOVAR. *Nature protocols.,* October 2015, vol. 10 (10), 1556-1566
- **TEMPEL S.** Using and understanding RepeatMasker. *Methods in molecular biology.,* 2012, vol. 859, 29-51

- **KASPRZYK A ; KEEFE D ; SMEDLEY D et al.** EnsMart: a generic system for fast and flexible access to biological data. *Genome research.,* January 2004, vol. 14 (1), 160-169
- **LANGMEAD B ; SALZBERG SL.** Fast gapped-read alignment with Bowtie 2. *Nature methods.,* 04 March 2012, vol. 9 (4), 357-359
- **LI H ; HANDSAKER B ; WYSOKER A et al.** The Sequence Alignment/Map format and SAMtools. *Bioinformatics.,* 15 August 2009, vol. 25 (16), 2078-2079
- **LI H.** A statistical framework for SNP calling, mutation discovery, association mapping and population genetical parameter estimation from sequencing data. *Bioinformatics.,* 01 November 2011, vol. 27 (21), 2987-2993
- **GENTLEMAN RC ; CAREY VJ ; BATES DM et al.** Bioconductor: open software development for computational biology and bioinformatics. *Genome biology.,* 2004, vol. 5 (10), R80
- **LINDEN A.** Measuring diagnostic and predictive accuracy in disease management: an introduction to receiver operating characteristic (ROC) analysis. *Journal of evaluation in clinical practice.,* April 2006, vol. 12 (2), 132-139
- **KRAMAR A ; FARAGGI D ; FORTUNE A ; REISER B.** mROC: a computer program for combining tumour markers in predicting disease states. *Computer methods and programs in biomedicine,* September 2001, vol. 66 (2-3), 199-207
- Random Forests. **BREIMAN L.** Machine Learning. Kluwer Academic Publishers, 2001, vol. 45, 5-32
- **CONSORTIUM GO.** Gene Ontology Consortium: going forward. *Nucleic acids research,* January 2015, vol. 43, D1049-1056
- **RAUDVERE U ; KOLBERG L ; KUZMIN I et al.** g:Profiler: a web server for functional enrichment analysis and conversions of gene lists (2019 update). *Nucleic acids research,* 02 July 2019, vol. 47 (W1), W191-W198
- **POMAZNOY M ; HA B ; PETERS B.** GOnet: a tool for interactive Gene Ontology analysis. *BMC bioinformatics.,* 07 December 2018, vol. 19 (1), 470

- **SALVETAT N ; VAN DER LAAN S ; VIRE B et al.** RNA editing blood biomarkers for predicting mood alterations in HCV patients. *Journal of neurovirology.,* 22 July 2019
- **SZKLARCZYK D ; GABLE AL ; LYON D et al.** STRING v11: protein-protein association networks with increased coverage, supporting functional discovery in genome-wide experimental datasets. *Nucleic acids research,* 08 January 2019, vol. 47 (D1), D607-D613
- **SHELTON RC.** The molecular neurobiology of depression. *The Psychiatric clinics of North America,* March 2007, vol. 30 (1), 1-11
- **COSTAS J ; GRATACOS M ; ESCARAMIS G et al.** Association study of 44 candidate genes with depressive and anxiety symptoms in post-partum women. *Journal of psychiatric research.,* August 2010, vol. 44 (11), 717-724
- **COLLEDGE M ; SNYDER EM ; CROZIER RA et al.** Ubiquitination regulates PSD-95 degradation and AMPA receptor surface expression. *Neuron.,* 30 October 2003, vol. 40 (3), 595-607
- **LIU B ; MINK S ; WONG KA et al.** PIAS1 selectively inhibits interferon-inducible genes and is important in innate immunity. *Nature immunology.,* September 2004, vol. 5 (9), 891-898
- **MURAKAMI Y ; ISHIBASHI T ; TOMITA E et al.** Depressive symptoms as a side effect of Interferon-alpha therapy induced by induction of indoleamine 2,3-dioxygenase 1. *Scientific reports.,* 20 July 2016, vol. 6, 29920
- **BRODIE EJ ; INFANTINO S ; LOW MSY ; TARLINTON DM.** Lyn, Lupus, and (B) Lymphocytes, a Lesson on the Critical Balance of Kinase Signaling in Immunity. *Frontiers in immunology,* 2018, vol. 9, 401
- **HAYASHI T ; UMEMORI H ; MISHINA M ; YAMAMOTO T.** The AMPA receptor interacts with and signals through the protein tyrosine kinase Lyn. *Nature,* 07 January 1999, vol. 397 (6714), 72-76
- **MOURA-ALVES P ; FAE K ; HOUTHUYS E et al.** AhR sensing of bacterial pigments regulates antibacterial defence. *Nature,* 28 August 2014, vol. 512 (7515), 387-392
- **SONG MS ; SONG SJ ; KIM SY ; OH HJ ; LIM DS.** The tumour suppressor RASSF1A promotes MDM2 self-ubiquitination by disrupting the MDM2-DAXX-HAUSP complex. *The EMBO journal,* 09 July 2008, vol. 27 (13), 1863-1874
- **WANG H ; NESTOR CE ; BENSON M ; ZHANG H.** GAB2 regulates type 2 T helper cell differentiation in humans. *Cytokine.,* August 2017, vol. 96, 234-237
- **VERPLOEGEN S ; LAMMERS JW ; KOENDERMAN L ; COFFER PJ.** Identification and characterization of CKLiK, a novel granulocyte Ca(++)/calmodulin-dependent kinase. *Blood.,* 01 November 2000, vol. 96 (9), 3215-3223
- **LIN E ; KUO PH ; LIU YL ; YU YW ; YANG AC ; TSAI SJ.** A Deep Learning Approach for Predicting Antidepressant Response in Major Depression Using Clinical and Genetic Biomarkers. *Frontiers in psychiatry.,* 2018, vol. 9, 290
- **ZHOU X ; CHEN Y ; MOK KY et al.** Identification of genetic risk factors in the Chinese population implicates a role of immune system in Alzheimer's disease pathogenesis. *Proceedings of the National Academy of Sciences of the United States of America,* 20 February 2018, vol. 115 (8), 1697-1706
- **BEUREL E ; LOWELL JA.** Th17 cells in depression. *Brain, behavior, and immunity.,* March 2018, vol. 69, 28-34
- **FISCHER EH ; CHARBONNEAU H ; TONKS NK.** Protein tyrosine phosphatases: a diverse family of intracellular and transmembrane enzymes. *Science,* 26 July 1991, vol. 253 (5018), 401-406
- **IRIE-SASAKI J ; SASAKI T ; MATSUMOTO W et al.** CD45 is a JAK phosphatase and negatively regulates cytokine receptor signalling. *Nature,* 18 January 2001, vol. 409 (6818), 349-354
- **BASTERZI AD ; YAZICI K ; BUTURAK V et al.** Effects of venlafaxine and fluoxetine on lymphocyte subsets in patients with major depressive disorder: a flow cytometric analysis. *Progress in neuro-psychopharmacology & biological psychiatry.,* 01 February 2010, vol. 34 (1), 70-75
- **ASTON C ; JIANG L ; SOKOLOV BP.** Transcriptional profiling reveals evidence for signaling and oligodendroglial abnormalities in the temporal cortex from patients with major depressive disorder. *Molecular psychiatry.,* March 2005, vol. 10 (3), 309-322
- **SENEY ML ; HUO Z ; CAHILL K et al.** Opposite Molecular Signatures of Depression in Men and Women. *Biological psychiatry.,* 01 July 2018, vol. 84 (1), 18-27
- **WOHLEB ES ; FRANKLIN T ; IWATA M ; DUMAN RS.** Integrating neuroimmune systems in the neurobiology of depression. *Nature reviews. Neuroscience.,* August 2016, vol. 17 (8), 497-511
- **WEISSMANN D ; VAN DER LAAN S ; UNDERWOOD MD et al.** Region-specific alterations of A-to-I RNA editing of serotonin 2c receptor in the cortex of suicides with major depression. *Translational psychiatry.,* 30 August 2016, vol. 6 (8), e878